# EUROPÄISCHE PATENTSCHRIFT

(11) **EP 2 399 996 B1**
(45) Veröffentlichungstag und Bekanntmachung des Hinweises auf die Patenterteilung: **06.09.2017**
(21) Anmeldenummer: 11007307.9
(22) Anmeldetag: 21.11.2003
(51) Int. Cl.: C12N 15/11, A61K 39/395, C12N 5/10, C07K 14/47, C07K 16/30, C07K 16/32, A61K 48/00, C07K 16/18, G01N 33/574

(54) **DIFFERENTIELL IN TUMOREN EXPRIMIERTE GENPRODUKTE UND DEREN VERWENDUNG**
IN TUMOURS DIFFERENTIALLY EXPRESSED GENE PRODUCTS AND USE OF THE SAME.
PRODUITS GÉNIQUES D'EXPRESSION DIFFÉRENTIELLE DANS LES TUMEURS ET LEUR UTILISATION

(30) Priorität: 22.11.2002 DE 10254601
(43) Veröffentlichungstag der Anmeldung: 28.12.2011
(62) Teilanmeldung aus: 03773719.4
(73) Patentinhaber: Ganymed Pharmaceuticals AG, 55131 Mainz (DE)
(72) Erfinder: Sahin, Ugur, 55116 Mainz (DE); Türeci, Özlem, 55116 Mainz (DE); Koslowski, Michael, 85764 Oberschleißheim (DE)
(74) Vertreter: Schnappauf, Georg

(56) Entgegenhaltungen:
- WO-A-01/27257
- WO-A-01/62920
- WO-A-01/68848
- WO-A-02/18576
- WO-A1-01/54708
- WO-A2-02/22684
- WO-A2-02/46224
- WO-A2-02/074237
- US-A1- 2002 119 130
- NIIMI T ET AL: "claudin-18, a novel downstream target gene for the T/EBP/NKX2.1 homeodomain transcription factor, encodes lung- and stomach-specific isoforms through alternative splicing.", MOLECULAR AND CELLULAR BIOLOGY NOV 2001, Bd. 21, Nr. 21, November 2001 (2001-11), Seiten 7380-7390, XP002433561, ISSN: 0270-7306
- REITER Y ET AL: "peptide-specific killing of antigen-presenting cells by a recombinant antibody-toxin fusion protein targeted to major histocompatibility complex/peptide class I complexes with T cell receptor-like specificity", PROCEEDINGS OF THE NATIONAL ACADEMY OF SCIENCES OF USA, NATIONAL ACADEMY OF SCIENCE. WASHINGTON, US, Bd. 94, April 1997 (1997-04), Seiten 4631-4636, XP002967291, ISSN: 0027-8424
- ENGBERG J ET AL: "RECOMBINANT ANTIBODIES WITH THE ANTIGEN-SPECIFIC, MHC RESTRICTED SPECIFICITY OF T CELLS: NOVEL REAGENTS FOR BASIC AND CLINICAL INVESTIGATIONS AND IMMUNOTHERAPY", IMMUNOTECHNOLOGY, XX, XX, Bd. 4, März 1999 (1999-03), Seiten 273-278, XP000991083,
- RUDOLPH M G ET AL: "The specificity of TCR/pMHC interaction", CURRENT OPINION IN IMMUNOLOGY, CURRENT BIOLOGY LTD, Bd. 14, Nr. 1, 1. Februar 2002 (2002-02-01), Seiten 52-65, XP004332735, ISSN: 0952-7915
- RIEMER A B ET AL: "Matching of trastuzumab (Herceptin<(>R)) epitope mimics onto the surface of Her-2/neu - a new method of epitope definition", MOLECULAR IMMUNOLOGY, PERGAMON, GB, vol. 42, no. 9, 1 May 2005 (2005-05-01), pages 1121-1124, XP027635008, ISSN: 0161-5890 [retrieved on 2005-05-01]
- P Büchler ET AL: "Therapy for pancreatic cancer with a recombinant humanized anti-HER2 antibody (herceptin)", Journal of Gastrointestinal Surgery, vol. 5, no. 2, 1 April 2001 (2001-04-01), pages 139-146, XP055188573, ISSN: 1091-255X, DOI: 10.1016/S1091-255X(01)80025-1

### SEQUENCE LISTING

<110> Ganymed Pharmaceuticals AG
   Sahin Dr., Ugur
   Tureci Dr., Özlem
   Koslowski Dr., Michael
<120> Differentiell in Tumoren exprimierte Genprodukte und deren Verwendung
<130> 342-4 EPT2
<150> DE 102 54 601.0 <151> 2002-11-22
<160> 23
<170> PatentIn version 3.1
<210> 7
   <211> 786
   <212> DNA
   <213> Homo sapiens
<400> 7
   atggccgtga ctgcctgtca gggcttgggg ttcgtggttt cactgattgg gattgcgggc     60
   atcattgctg ccacctgcat ggaccagtgg agcacccaag acttgtacaa caaccccgta    120
   acagctgttt tcaactacca ggggctgtgg cgctcctgtg tccgagagag ctctggcttc    180
   accgagtgcc ggggctactt caccctgctg gggctgccag ccatgctgca ggcagtgcga    240
   gccctgatga tcgtaggcat cgtcctgggt gccattggcc tcctggtatc catctttgcc    300
   ctgaaatgca tccgcattgg cagcatggag gactctgcca aagccaacat gacactgacc    360
   tccgggatca tgttcattgt ctcaggtctt tgtgcaattg ctggagtgtc tgtgtttgcc    420
   aacatgctgg tgactaactt ctggatgtcc acagctaaca tgtacaccgg catgggtggg    480
   atggtgcaga ctgttcagac caggtacaca tttggtgcgg ctctgttcgt gggctgggtc    540
   gctggaggcc tcacactaat tgggggtgtg atgatgtgca tcgcctgccg gggcctggca    600
   ccagaagaaa ccaactacaa agccgtttct tatcatgcct caggccacag tgttgcctac    660
   aagcctggag gcttcaaggc cagcactggc tttgggtcca acaccaaaaa caagaagata    720
   tacgatggag gtgcccgcac agaggacgag gtacaatctt atccttccaa gcacgactat    780
   gtgtaa                                                               786
<210> 8
   <211> 180
   <212> DNA
   <213> Homo sapiens
<400> 8
   tgcgccacca tggccgtgac tgcctgtcag ggcttggggt tcgtggtttc actgattggg     60
   attgcgggca tcattgctgc cacctgcatg gaccagtgga gcacccaaga cttgtacaac    120
   aaccccgtaa cagctgtttt caactaccag gggctgtggc gctcctgtgt ccgagagagc    180
<210> 16
   <211> 261
   <212> PRT
   <213> Homo sapiens
<400> 16
   Met Ala Val Thr Ala Cys Gln Gly Leu Gly Phe Val Val Ser Leu Ile
   1               5                   10                  15
   Gly Ile Ala Gly Ile Ile Ala Ala Thr Cys Met Asp Gln Trp Ser Thr
               20                  25                  30
   Gln Asp Leu Tyr Asn Asn Pro Val Thr Ala Val Phe Asn Tyr Gln Gly
           35                  40                  45
   Leu Trp Arg Ser Cys Val Arg Glu Ser Ser Gly Phe Thr Glu Cys Arg
       50                  55                  60
   Gly Tyr Phe Thr Leu Leu Gly Leu Pro Ala Met Leu Gln Ala Val Arg
   65                  70                  75                  80
   Ala Leu Met Ile Val Gly Ile Val Leu Gly Ala Ile Gly Leu Leu Val
                   85                  90                  95
   Ser Ile Phe Ala Leu Lys Cys Ile Arg Ile Gly Ser Met Glu Asp Ser
               100                 105                 110
   Ala Lys Ala Asn Met Thr Leu Thr Ser Gly Ile Met Phe Ile Val Ser
           115                 120                 125
   Gly Leu Cys Ala Ile Ala Gly Val Ser Val Phe Ala Asn Met Leu Val
       130                 135                 140
   Thr Asn Phe Trp Met Ser Thr Ala Asn Met Tyr Thr Gly Met Gly Gly
   145                 150                 155                 160
   Met Val Gln Thr Val Gln Thr Arg Tyr Thr Phe Gly Ala Ala Leu Phe
                   165                 170                 175
   Val Gly Trp Val Ala Gly Gly Leu Thr Leu Ile Gly Gly Val Met Met
               180                 185                 190
   Cys Ile Ala Cys Arg Gly Leu Ala Pro Glu Glu Thr Asn Tyr Lys Ala
           195                 200                 205
   Val Ser Tyr His Ala Ser Gly His Ser Val Ala Tyr Lys Pro Gly Gly
       210                 215                 220
   Phe Lys Ala Ser Thr Gly Phe Gly Ser Asn Thr Lys Asn Lys Lys Ile
   225                 230                 235                 240
   Tyr Asp Gly Gly Ala Arg Thr Glu Asp Glu Val Gln Ser Tyr Pro Ser
                   245                 250                 255
   Lys His Asp Tyr Val
               260
<210> 17
   <211> 10
   <212> PRT
   <213> Homo sapiens
<400> 17
   Asp Gln Trp Ser Thr Gln Asp Leu Tyr Asn
   1               5                   10
<210> 18
   <211> 11
   <212> PRT
   <213> Homo sapiens
<400> 18
   Asn Asn Pro Val Thr Ala Val Phe Asn Tyr Gln
   1 5 10
<210> 19
   <211> 47
   <212> PRT
   <213> Homo sapiens
<400> 19
   Met Ala Val Thr Ala Cys Gln Gly Leu Gly Phe Val Val Ser Leu Ile
   1 5 10 15
   Gly Ile Ala Gly Ile Ile Ala Ala Thr Cys Met Asp Gln Trp Ser Thr
   20 25 30
   Gln Asp Leu Tyr Asn Asn Pro Val Thr Ala Val Phe Asn Tyr Gln
   35 40 45
<210> 39
   <211> 28
   <212> DNA
   <213> Künstliche Sequenz
<220>
   <223> Beschreibung der künstlichen Sequenz: Oligonukleotid
<400> 39
   ggttcgtggt ttcactgatt gggattgc 28
<210> 40
   <211> 27
   <212> DNA
   <213> Künstliche Sequenz
<220>
   <223> Beschreibung der künstlichen Sequenz: Oligonukleotid
<400> 40
   cggctttgta gttggtttct tctggtg 27
<210> 107
   <211> 20
   <212> DNA
   <213> künstliche Sequenz
<220>
   <223> Beschreibung der künstlichen Sequenz: Oligonukleotid
<400> 107
   tgttttcaac taccaggggc 20
<210> 108
   <211> 20
   <212> DNA
   <213> künstliche Sequenz
<220>
   <223> Beschreibung der künstlichen Sequenz: Oligonukleotid
<400> 108
   tgttggcttt ggcagagtcc 20
<210> 109
   <211> 24
   <212> DNA
   <213> künstliche Sequenz
<220>
   <223> Beschreibung der künstlichen Sequenz: Oligonukleotid
<400> 109
   gaggcagagt tcaggcttca ccga 24
<210> 110
   <211> 20
   <212> DNA
   <213> künstliche Sequenz
<220>
   <223> Beschreibung der künstlichen Sequenz: Oligonukleotid
<400> 110
   tgttggcttt ggcagagtcc 20
<210> 111
   <211> 56
   <212> PRT
   <213> Homo sapiens
<400> 111
   Thr Gly Met Asp Met Trp Ser Thr Gln Asp Leu Tyr Asp Asn Pro Val
   1               5                   10                  15
   Thr Ser Val Phe Gln Tyr Glu Gly Leu Trp Arg Ser Cys Val Arg Gln
               20                  25                  30
   Ser Ser Gly Phe Thr Glu Cys Arg Pro Tyr Phe Thr Ile Leu Gly Leu
           35                  40                  45
   Pro Ala Met Leu Gln Ala Val Arg
       50                  55
<210> 112
   <211> 53
   <212> PRT
   <213> Homo sapiens
<400> 112
   Asp Gln Trp Ser Thr Gln Asp Leu Tyr Asn Asn Pro Val Thr Ala Val
   1               5                   10                  15
   Phe Asn Tyr Gln Gly Leu Trp Arg Ser Cys Val Arg Glu Ser Ser Gly
               20                  25                  30
   Phe Thr Glu Cys Arg Gly Tyr Phe Thr Leu Leu Gly Leu Pro Ala Met
           35                  40                  45
   Leu Gln Ala Val Arg
       50
<210> 113
   <211> 14
   <212> PRT
   <213> Homo sapiens
<400> 113
   Ser Thr Gln Asp Leu Tyr Asn Asn Pro Val Thr Ala Val Phe
   1               5                   10
<210> 114
   <211> 12
   <212> PRT
   <213> Homo sapiens
<400> 114
   Asp Met Trp Ser Thr Gln Asp Leu Tyr Asp Asn Pro
   1 5 10
<210> 115
   <211> 12
   <212> PRT
   <213> Homo sapiens
<400> 115
   Cys Arg Pro Tyr Phe Thr Ile Leu Gly Leu Pro Ala
   1 5 10
<210> 116
   <211> 13
   <212> PRT
   <213> Homo sapiens
<400> 116
   Thr Asn Phe Trp Met Ser Thr Ala Asn Met Tyr Thr Gly
   1 5 10
<210> 117
   <211> 816
   <212> DNA
   <213> Homo sapiens
<400> 117
   gccaggatca tgtccaccac cacatgccaa gtggtggcgt tcctcctgtc catcctgggg 60
   ctggccggct gcatcgcggc caccgggatg gacatgtgga gcacccagga cctgtacgac 120
   aaccccgtca cctccgtgtt ccagtacgaa gggctctgga ggagctgcgt gaggcagagt 180
   tcaggcttca ccgaatgcag gccctatttc accatcctgg gacttccagc catgctgcag 240
   gcagtgcgag ccctgatgat cgtaggcatc gtcctgggtg ccattggcct cctggtatcc    300
   atctttgccc tgaaatgcat ccgcattggc agcatggagg actctgccaa agccaacatg    360
   acactgacct ccgggatcat gttcattgtc tcaggtcttt gtgcaattgc tggagtgtct    420
   gtgtttgcca acatgctggt gactaacttc tggatgtcca cagctaacat gtacaccggc    480
   atgggtggga tggtgcagac tgttcagacc aggtacacat ttggtgcggc tctgttcgtg    540
   ggctgggtcg ctggaggcct cacactaatt gggggtgtga tgatgtgcat cgcctgccgg    600
   ggcctggcac cagaagaaac caactacaaa gccgtttctt atcatgcctc aggccacagt    660
   gttgcctaca agcctggagg cttcaaggcc agcactggct ttgggtccaa caccaaaaac    720
   aagaagatat acgatggagg tgcccgcaca gaggacgagg tacaatctta tccttccaag    780
   cacgactatg tgtaatgctc taagacctct cagcac                              816
<210> 118
   <211> 261
   <212> PRT
   <213> Homo sapiens
<400> 118
   Met Ser Thr Thr Thr Cys Gln Val Val Ala Phe Leu Leu Ser Ile Leu
   1               5                   10                  15
   Gly Leu Ala Gly Cys Ile Ala Ala Thr Gly Met Asp Met Trp Ser Thr
               20                  25                  30
   Gln Asp Leu Tyr Asp Asn Pro Val Thr Ser Val Phe Gln Tyr Glu Gly
           35                  40                  45
   Leu Trp Arg Ser Cys Val Arg Gln Ser Ser Gly Phe Thr Glu Cys Arg
       50                  55                  60
   Pro Tyr Phe Thr Ile Leu Gly Leu Pro Ala Met Leu Gln Ala Val Arg
   65                  70                  75                  80
   Ala Leu Met Ile Val Gly Ile Val Leu Gly Ala Ile Gly Leu Leu Val
                   85                  90                  95
   Ser Ile Phe Ala Leu Lys Cys Ile Arg Ile Gly Ser Met Glu Asp Ser
               100                 105                 110
   Ala Lys Ala Asn Met Thr Leu Thr Ser Gly Ile Met Phe Ile Val Ser
           115                 120                 125
   Gly Leu Cys Ala Ile Ala Gly Val Ser Val Phe Ala Asn Met Leu Val
       130                 135                 140
   Thr Asn Phe Trp Met Ser Thr Ala Asn Met Tyr Thr Gly Met Gly Gly
   145                 150                 155                 160
   Met Val Gln Thr Val Gln Thr Arg Tyr Thr Phe Gly Ala Ala Leu Phe
                   165                 170                 175
   Val Gly Trp Val Ala Gly Gly Leu Thr Leu Ile Gly Gly Val Met Met
               180                 185                 190
   Cys Ile Ala Cys Arg Gly Leu Ala Pro Glu Glu Thr Asn Tyr Lys Ala
           195                 200                 205
   Val Ser Tyr His Ala Ser Gly His Ser Val Ala Tyr Lys Pro Gly Gly
       210                 215                 220
   Phe Lys Ala Ser Thr Gly Phe Gly Ser Asn Thr Lys Asn Lys Lys Ile
   225                 230                 235                 240
   Tyr Asp Gly Gly Ala Arg Thr Glu Asp Glu Val Gln Ser Tyr Pro Ser
                   245                 250                 255
   Lys His Asp Tyr Val
               260
<210> 119
   <211> 227
   <212> DNA
   <213> Homo sapiens
<400> 119
   gccaggatca tgtccaccac cacatgccaa gtggtggcgt tcctcctgtc catcctgggg      60
   ctggccggct gcatcgcggc caccgggatg gacatgtgga gcacccagga cctgtacgac     120
   aaccccgtca cctccgtgtt ccagtacgaa gggctctgga ggagctgcgt gaggcagagt     180
   tcaggcttca ccgaatgcag gccctatttc accatcctgg gacttcc                   227
<210> 120
   <211> 69
   <212> PRT
   <213> Homo sapiens
<400> 120
   Met Ser Thr Thr Thr Cys Gln Val Val Ala Phe Leu Leu Ser Ile Leu
   1 5 10 15
   Gly Leu Ala Gly Cys Ile Ala Ala Thr Gly Met Asp Met Trp Ser Thr
   20 25 30
   Gln Asp Leu Tyr Asp Asn Pro Val Thr Ser Val Phe Gln Tyr Glu Gly
   35 40 45
   Leu Trp Arg Ser Cys Val Arg Gln Ser Ser Gly Phe Thr Glu Cys Arg
   50 55 60
   Pro Tyr Phe Thr Ile
   65
<210> 137
   <211> 32
   <212> PRT
   <213> Homo sapiens
<400> 137

## Beschreibung

Trotz interdisziplinärer Ansätze und Ausreizung klassischer Therapiemodalitäten gehören Krebserkrankungen weiterhin zu den führenden Todesursachen. Neuere therapeutische Konzepte zielen darauf ab, das patienteneigene Immunsystem durch Einsatz von rekombinanten Tumorvakzinen und anderen spezifischen Maßnahmen wie Antikörpertherapie in das therapeutische Gesamtkonzept mit einzubeziehen. Voraussetzung für den Erfolg einer solchen Strategie ist die Erkennung von Tumor-spezifischen oder Tumor-assoziierten Antigenen bzw. Epitopen durch das Immunsystem des Patienten, dessen Effektorfunktionen interventionell verstärkt werden sollen. Tumorzellen unterscheiden sich biologisch wesentlich von ihren nichtmalignen Ursprungszellen. Diese Differenzen sind durch während der Tumorentwicklung erworbene genetische Veränderungen bedingt und führen u.a. auch zur der Bildung qualitativ oder quantitativ veränderter molekularer Strukturen in den Krebszellen. Werden solche Tumor-assoziierten Strukturen vom spezifischen Immunsystem des tumortragenden Wirtes erkannt, spricht man von Tumor-assoziierten Antigenen. An der spezifischen Erkennung von Tumor-assoziierten Antigenen sind zelluläre und humorale Mechanismen beteiligt, die zwei miteinander funktionell vernetzte Einheiten darstellen: CD4⁺ und CD8⁺ T-Lymphozyten erkennen prozessierte Antigene, die auf den Molekülen der MHC- (Major Histocompatibility complex = Histokompatibilitätsantigene) Klassen II bzw. I präsentiert werden, während B-Lymphozyten zirkulierende Antikörpermoleküle produzieren, die direkt an unprozessierte Antigene binden. Die potentielle klinisch-therapeutische Bedeutung von Tumor-assoziierten Antigenen ergibt sich aus der Tatsache, dass die Erkennung von Antigenen auf neoplastischen Zellen durch das Immunsystem zur Initiierung von cytotoxischen Effektormechanismen führt und bei Vorhandensein von T-Helferzellen die Elimination der Krebszellen bewirken kann (Pardoll, Nat. Med. 4:525-31, 1998). Entsprechend ist es eine zentrale Zielsetzung der Tumorimmunologie, diese Strukturen molekular zu definieren. Die molekulare Natur dieser Antigene blieb lange enigmatisch. Erst als entsprechende Klonierungstechniken entwickelt wurden, gelang es, durch Analyse der Zielstrukturen von cytotoxischen T-Lymphozyten (CTL) (van der Bruggen et al., Science 254:1643-7, 1991) bzw. mit zirkulierenden Autoantikörpern (Sahin et al., Curr. Opin. Immunol. 9:709-16, 1997) als Sonden cDNA-Expressionsbanken von Tumoren systematisch auf Tumor-assoziierte Antigene zu screenen. Hierzu wurden cDNA-Expressionsbanken aus frischem Tumorgewebe hergestellt und in geeigneten Systemen als Proteine rekombinant exprimiert. Aus Patienten isolierte Immuneffektoren, nämlich CTL-Klone mit Tumor-spezifischem Lysemuster oder zirkulierende Autoantikörper, wurden genutzt, um die respektiven Antigene zu klonieren.

Durch diese Ansätze sind in den letzten Jahren eine Vielzahl von Antigenen in verschiedenen Neoplasien definiert worden. Allerdings nutzen die oben dargestellten klassischen Verfahren zur Antigenidentifizierung Immuneffektoren (zirkulierende Autoantikörper oder CTL-Klone) aus Patienten mit in der Regel bereits fortgeschrittenem Krebs als Sonden. Aus einer Reihe von Daten geht hervor, dass Tumoren z.B. zur Tolerisierung und Anergisierung von T-Zellen führen können und gerade im Verlauf der Erkrankung diejenigen Spezifitäten aus dem Immuneffektorenrepertoire verloren gehen, die eine effektive Immunerkennung bewirken könnten. Aus laufenden Patientenstudien hat sich noch kein gesicherter Beweis für eine tatsächliche Wirkung der bisher entdeckten und genutzten Tumor-assoziierten Antigene ergeben. Entsprechend kann nicht ausgeschlossen werden, dass spontane Immunantworten evozierende Proteine die falschen Zielstrukturen sind.

Es war die Aufgabe der vorliegenden Erfindung Zielstrukturen für eine Diagnose und Therapie von Krebserkrankungen bereitzustellen.

Diese Aufgabe wird erfindungsgemäß durch den Gegenstand der Patentansprüche gelöst.

Erfindungsgemäß wurde eine Strategie für eine Identifizierung und Bereitstellung Tumorassoziiert exprimierter Antigene und der dafür kodierenden Nukleinsäuren verfolgt. Diese Strategie beruht auf der Tatsache, dass bestimmte Gene, die Organ-spezifisch, z.B. ausschließlich im Kolon-, Lungen- oder Nieren-Gewebe, exprimiert werden, in den entsprechenden Organen auch von Tumorzellen und darüber hinaus in anderen Geweben in Tumorzellen ektop und unerlaubt reaktiviert werden. Durch Datamining wird zunächst eine möglichst komplette Liste aller bekannten Organ-spezifischen Gene aufgestellt und diese sodann durch Expressionsanalysen mittels spezifischer RT-PCR auf ihre aberrante Aktivierung in unterschiedlichen Tumoren evaluiert. Datamining ist ein bekanntes Verfahren zur Identifizierung von Tumor-assoziierten Genen. Bei den herkömmlichen Strategien werden allerdings in der Regel Transkriptome von Normalgewebebanken elektronisch von Tumorgewebsbanken subtrahiert unter der Annahme, dass die verbleibenden Gene Tumor-spezifisch sind (Schmitt et al., Nucleic Acids Res. 27:4251-60, 1999; Vasmatzis et al., Proc. Natl. Acad. Sci. U S A. 95:300-4, 1998; Scheurle et al., Cancer Res. 60:4037-43, 2000).
Das erfindungsgemäße Konzept, das sich als viel erfolgreicher erwiesen hat, beruht jedoch darauf, Datamining zur elektronischen Extraktion aller Organ-spezifischer Gene zu nutzen und diese sodann auf Expression in Tumoren zu evaluieren.

Somit betrifft die Erfindung in einem Aspekt eine Strategie zur Identifizierung von Gewebespezifischen und differentiell in Tumoren exprimierten Genen. Diese Strategie kombiniert Datamining von öffentlichen Sequenzbanken (*"in silico"*) mit darauf folgenden evaluierenden labor-experimentellen ("wet bench") Untersuchungen.

Eine kombinierte Strategie basierend auf zwei unterschiedlichen bioinformatischen Skripten ermöglichte erfindungsgemäß die Identifizierung neuer Tumor-Gene. Diese sind bisher als rein Organ-spezifisch eingestuft worden. Die Erkenntnis, dass diese Gene aberrant in Tumorzellen aktiviert werden, erlaubt, ihnen eine substantiell neue Qualität mit funktionellen Implikationen zuzuordnen. Die Identifizierung und Bereitstellung dieser Tumor-assoziierten Gene und der dadurch kodierten Genprodukte erfolgte erfindungsgemäß unabhängig von einer immunogenen Wirkung.

Die offenbarungsgemäß identifizierten Tumor-assoziierten Antigene weisen eine Aminosäuresequenz auf, die von einer Nukleinsäure kodiert wird, die aus der Gruppe ausgewählt ist, bestehend aus (a) einer Nukleinsäure, die eine Nukleinsäuresequenz umfasst, die aus der Gruppe bestehend aus SEQ ID NO: 7-8, 117 und 119, einem Teil oder Derivat davon ausgewählt ist, (b) einer Nukleinsäure, die unter stringenten Bedingungen mit der Nukleinsäure unter (a) hybridisiert, (c) einer Nukleinsäure, die in Bezug auf die Nukleinsäure unter (a) oder (b) degeneriert ist und (d) einer Nukleinsäure, die zu der Nukleinsäure unter (a), (b) oder (c) komplementär ist. In einer bevorzugten Ausführungsform weist ein erfindungsgemäß identifiziertes Tumor-assoziiertes Antigen eine Aminosäuresequenz auf, die von einer Nukleinsäure kodiert wird, die aus der Gruppe bestehend aus SEQ ID NO: 7-8, 117 und 119 ausgewählt ist. In einer weiteren bevorzugten Ausführungsform umfasst ein erfindungsgemäß identifiziertes Tumor-assoziiertes Antigen eine Aminosäuresequenz, die aus der Gruppe bestehend aus SEQ ID NO: 16-19, 111-116, 118, 120, und 137, einem Teil oder Derivat davon ausgewählt ist.

Die vorliegende Erfindung betrifft allgemein die Verwendung von offenbarungsgemäß identifizierten Tumor-assoziierten Antigenen bzw. von Antikörpern, die gegen diese Tumor-assoziierten Antigene gerichtet sind, für die Therapie und Diagnose. Diese Nutzung kann einzelne, aber auch Kombinationen von mehreren dieser Antigene, funktionalen Fragmente, Nukleinsäuren, Antikörper etc. betreffen, in einer Ausführungsform auch in Kombination mit anderen Tumor-assoziierten Genen und Antigenen für eine Diagnose, Therapie und Verlaufskontrolle.

Bevorzugte Erkrankungen für eine Therapie und/oder Diagnose sind solche, bei denen eine selektive Expression oder abnormale Expression von einem oder mehreren der erfindungsgemäß identifizierten Tumor-assoziierten Antigenen vorliegt.

Die Offenbarung betrifft auch Nukleinsäuren und Genprodukte, die tumorzellassoziiert exprimiert werden.

Desweiteren betrifft die Erfindung Genprodukte, d.h. Nukleinsäuren und Proteine bzw. Peptide, die durch verändertes Spleißen (Spleißvarianten) bekannter Gene bzw. durch veränderte Translation unter Nutzung alternativer offener Leserahmen entstehen. Die erfindungsgemäßen Spleißvarianten sind erfindungsgemäß als Targets für die Diagnostik und Therapie von Tumorerkrankungen verwendbar.

Für die Entstehung von Spleißvarianten können verschiedenste Mechanismen ursächlich sein, beispielsweise
- die Nutzung variabler Transkriptionsinitiationsstellen,
- die Nutzung zusätzlicher Exons,
- das vollständige oder unvollständige Ausspleißen von einzelnen oder mehreren Exons,
- durch Mutation veränderte Spleißregulatorsequenzen (Deletion bzw. Schaffung neuer Donor/Acceptorsequenzen),
- die unvollständige Elimination von Intronsequenzen.

Das veränderte Spleißen eines Gens führt zu einer veränderten Transkriptsequenz (Spleißvariante). Wird eine Spleißvariante im Bereich ihrer veränderten Sequenz translatiert, resultiert ein verändertes Protein, welches sich von dem ursprünglichen in Struktur und Funktion deutlich unterscheiden kann. Bei Tumor-assoziierten Spleißvarianten können Tumor-assoziierte Transkripte und Tumor-assoziierte Proteine/Antigene entstehen. Diese können als molekulare Marker sowohl zum Nachweis von Tumorzellen als auch zum therapeutischen Targeting von Tumoren genutzt werden. Die Detektion von Tumorzellen z.B. im Blut, Serum, Knochenmark, Sputum, Bronchial-Lavage, Körpersekreten und Gewebsbiopsien kann erfindungsgemäß z.B. nach Extraktion von Nukleinsäuren durch PCR-Amplifikation mit Spleißvarianten-spezifischen Oligonukleotiden erfolgen. Als Oligonukleotide eignen sich insbesondere Paare von Primern, von denen mindestens einer unter stringenten Bedingungen an die Region der Spleißvariante bindet, die Tumorassoziiert ist. Erfindungsgemäß geeignet sind die in den Beispielen für diesen Zweck beschriebenen Oligonukleotide, insbesondere Oligonukleotide, die eine Sequenz ausgewählt aus SEQ ID NO: 39, 40 und 107-110 des Sequenzprotokolls aufweisen bzw. umfassen. Zum Nachweis eignen sich erfindungsgemäß alle Sequenz-abhängigen Detektionssysteme. Neben der PCR sind diese z.B. Genchip-/Microarraysysteme, Northern-Blot, RNAse protection assays (RDA) und andere. Allen Detektionssystemen ist gemeinsam, dass die Detektion auf einer spezifischen Hybridisierung mit mindestens einer Spleißvarianten-spezifischen Nukleinsäuresequenz basiert. Die Detektion von Tumorzellen kann jedoch auch erfindungsgemäß durch Antikörper erfolgen, die ein durch die Spleißvariante kodiertes spezifisches Epitop erkennen. Für die Herstellung der Antikörper können Peptide zur Immunisierung verwendet werden, die für diese Spleißvariante spezifisch sind. In diesem Aspekt betrifft die Erfindung insbesondere Peptide, die eine Sequenz ausgewählt aus SEQ ID NO: 17-19, 111-115, 120 und 137 des Sequenzprotokolls aufweisen bzw. umfassen und dagegen gerichtete spezifische Antikörper. Für die Immunisierung eignen sich besonders die Aminosäuren, die deutliche Epitopunterschiede zu der (den) Variante(n) des Genprodukts aufweisen, welche(s) bevorzugt in gesunden Zellen gebildet wird (werden). Der Nachweis der Tumorzellen mit Antikörpern kann dabei an einer vom Patienten isolierten Probe oder als Imaging mit intravenös applizierten Antikörpern erfolgen. Neben der diagnostischen Nutzbarkeit stellen Spleißvarianten, die neue oder veränderte Epitope aufweisen, attraktive Targets für die Immuntherapie dar. Die erfindungsgemäßen Epitope können zum Targeting von therapeutisch wirksamen monoklonalen Antikörpern oder T-Lymphozyten genutzt werden. Bei der passiven Immuntherapie werden hierbei Antikörper oder T-Lymphozyten adoptiv transferriert, die Spleißvarianten-spezifische Epitope erkennen. Die Generierung von Antikörpern kann wie bei anderen Antigenen auch unter Nutzung von Standardtechnologien (Immunisierung von Tieren, Panningstrategien zur Isolation von rekombinanten Antikörpern) unter Nutzung von Polypeptiden, die diese Epitope beinhalten, erfolgen. Alternativ können zur Immunisierung Nukleinsäuren genutzt werden, die für Oligo- oder Polypeptide kodieren, die diese Epitope beinhalten. Verschiedene Techniken zur in vitro oder in vivo Generierung von epitopspezifischen T-Lymphozyten sind bekannt und ausführlich beschrieben (vgl. z.B. Kessler JH, et al. 2001, Sahin et al., 1997) und basieren ebenfalls auf der Nutzung von Oligo- oder Polypeptiden, die die Spleißvarianten-spezifischen Epitope beinhalten oder Nukleinsäuren, die für diese kodieren. Oligo- oder Polypeptiden, die die Spleißvarianten-spezifischen Epitope beinhalten, oder Nukleinsäuren, die für diese Polypeptide kodieren, sind auch als pharmazeutisch wirksame Substanzen bei der aktiven Immuntherapie (Vakzinierung, Vakzintherapie) verwendbar.

Erfindungsgemäß werden auch Proteine beschrieben, die sich durch Art und Menge ihrer sekundären Modifikationen in Normal- und Tumorgewebe unterscheiden (z.B. Durand & Seta, 2000; Clin. Chem. 46: 795-805; Hakomori, 1996; Cancer Res. 56: 5309-18).
Die Analyse von Proteinmodifikationen kann im Western-Blot erfolgen. Vor allem Glykosylierungen, die in der Regel eine Größe von mehreren kDa haben, führen zu einer größeren Gesamtmasse des Zielproteins, die sich in der SDS-PAGE auftrennen lässt. Zum Nachweis von spezifischen O- und N-glycosidischen Bindungen werden Proteinlysate vor der Denaturierung durch SDS mit O- oder N-Glykosylasen inkubiert (nach Angaben des jeweiligen Herstellers, z.B. PNgase, Endoglykosidase F, Endoglykosidase H, Roche Diagnostics). Anschließend erfolgt ein Western-Blot. Bei Verringerung der Größe eines Zielproteins kann so nach Inkubation mit einer Glykosidase eine spezifische Glykosylierung nachgewiesen und auf diesem Weg auch die Tumorspezifität einer Modifikation analysiert werden. Von besonderem Interesse sind Proteinbereiche, die in Tumorzellen und gesunden Zellen differenziell glykosyliert sind. Derartige Glykosylierungsunterschiede sind jedoch bisher für wenige Zelloberflächenproteine (z.B. Muc1) beschrieben.

Erfindungsgemäß konnte für Claudin-18 eine differentielle Glykosylierung in Tumoren nachgewiesen werden. Gastrointestinale Karzinome, Pankreaskarzinome, Ösophagustumoren, Prostatatumoren als auch Lungentumoren weisen eine weniger glykosylierte Form von Claudin-18 auf. Die Glykosylierung in gesunden Geweben maskiert Proteinepitope von Claudin-18, die auf Tumorzellen aufgrund fehlender Glykosylierung freigelegt sind. Entsprechend lassen sich erfindungsgemäß Liganden und Antikörper selektieren, die an diese Domänen binden. Derartige Liganden und Antikörper binden erfindungsgemäß nicht an das Claudin-18 auf gesunden Zellen, da hier die Epitope durch die Glykosylierung verdeckt sind.

Ähnlich wie oben für von Tumor-assoziierten Spleißvarianten abgeleitete Proteinepitope beschrieben kann somit die differenzielle Glycosylierung zur Unterscheidung von Normal- und Tumorzellen mit diagnostischer wie auch therapeutischer Intention genutzt werden.

In einem Aspekt betrifft die Offenbarung eine pharmazeutische Zusammensetzung umfassend ein Mittel, das das erfindungsgemäß identifizierte Tumor-assoziierte Antigen erkennt und vorzugsweise selektiv für Zellen ist, die eine Expression oder abnormale Expression eines erfindungsgemäß identifizierten Tumor-assoziierten Antigens aufweisen. Das Mittel kann in bestimmten Ausführungsformen die Induktion des Zelltods, die Reduktion des Zellwachstums, die Schädigung der Zellmembran oder die Sekretion von Zytokinen bewirken und weist vorzugsweise eine tumorhemmende Aktivität auf. In einer Ausführungsform ist das Mittel eine Antisense-Nukleinsäure, die selektiv mit der Nukleinsäure hybridisiert, die für das Tumor-assoziierte Antigen kodiert. In einer weiteren Ausführungsform ist das Mittel ein Antikörper, der selektiv an das Tumor-assoziierte Antigen bindet, insbesondere ein komplementaktivierter oder Toxin-konjugierter Antikörper, der selektiv an das Tumor-assoziierte Antigen bindet. In einer weiteren Ausführungsform umfasst das Mittel mehrere Mittel, die jeweils selektiv verschiedene Tumor-assoziierte Antigene erkennen, wobei mindestens eines der Tumor-assoziierten Antigene ein erfindungsgemäß identifiziertes Tumor-assoziiertes Antigen ist. Die Erkennung muss nicht direkt mit einer Hemmung von Aktivität oder Expression des Antigens einhergehen. In diesem Aspekt der Erfindung dient das selektiv auf Tumoren beschränkte Antigen vorzugsweise als Markierung zur Rekrutierung von Effektormechanismen an diesen spezifischen Ort. In einer bevorzugten Ausführungsform ist das Mittel ein cytotoxischer T-Lymphozyt, der das Antigen auf einem HLA-Molekül erkennt und die derartig markierte Zellen lysiert. In einer weiteren Ausführungsform ist das Mittel ein Antikörper, der selektiv an das Tumor-assoziierte Antigen bindet und somit natürliche oder artifizielle Effektormechanismen zu dieser Zelle rekrutiert. In einer weiteren Ausführungsform ist das Mittel ein T-Helfer-Lymphozyt, der Effektorfunktionen von anderen Zellen, die spezifisch dieses Antigen erkennen, stärkt.

In einem Aspekt betrifft die Offenbarung eine pharmazeutische Zusammensetzung umfassend ein Mittel, das die Expression oder Aktivität eines erfindungsgemäß identifizierten Tumor-assoziierten Antigens hemmt. In einer bevorzugten Ausführungsform ist das Mittel eine Antisense-Nukleinsäure, die selektiv mit der Nukleinsäure hybridisiert, die für das Tumor-assoziierte Antigen kodiert. In einer weiteren Ausführungsform ist das Mittel ein Antikörper, der selektiv an das Tumor-assoziierte Antigen bindet. In einer weiteren Ausführungsform umfasst das Mittel mehrere Mittel, die jeweils selektiv die Expression oder Aktivität verschiedener Tumor-assoziierter Antigene hemmen, wobei mindestens eines der Tumor-assoziierten Antigene ein erfindungsgemäß identifiziertes Tumor-assoziiertes Antigen ist.

Des weiteren betrifft die Offenbarung eine pharmazeutische Zusammensetzung, die ein Mittel umfasst, das bei einer Verabreichung selektiv die Menge an Komplexen zwischen einem HLA-Molekül und einem Peptidepitop aus dem erfindungsgemäß identifizierten Tumor-assoziierten Antigen erhöht. Das Mittel umfasst in einer Ausführungsform einen oder mehrere Bestandteile, die aus der Gruppe ausgewählt sind, bestehend aus (i) dem Tumor-assoziierten Antigen oder einem Teil davon, (ii) einer Nukleinsäure, die für das Tumor-assoziierte Antigen oder einen Teil davon kodiert, (iii) einer Wirtszelle, die das Tumor-assoziierte Antigen oder einen Teil davon exprimiert, und (iv) isolierten Komplexen zwischen Peptidepitopen aus dem Tumor-assoziierten Antigen und einem MHC-Molekül. In einer Ausführungsform umfasst das Mittel mehrere Mittel, die jeweils selektiv die Menge an Komplexen zwischen MHC-Molekülen und Peptidepitopen verschiedener Tumor-assoziierter Antigene erhöhen, wobei mindestens eines der Tumor-assoziierten Antigene ein erfindungsgemäß identifiziertes Tumor-assoziiertes Antigen ist.

Des weiteren betrifft die Offenbarung eine pharmazeutische Zusammensetzung, die einen oder mehrer Bestandteile umfasst, die aus der Gruppe ausgewählt sind, bestehend aus (i) einem erfindungsgemäß identifizierten Tumor-assoziierten Antigen oder einem Teil davon, (ii) einer Nukleinsäure, die für ein erfindungsgemäß identifiziertes Tumor-assoziiertes Antigen oder einen Teil davon kodiert, (iii) einem Antikörper, der an ein erfindungsgemäß identifiziertes Tumor-assoziiertes Antigen oder einen Teil davon bindet, (iv) einer Antisense-Nukleinsäure, die spezifisch mit einer Nukleinsäure, die für ein erfindungsgemäß identifiziertes Tumor-assoziiertes Antigen kodiert, hybridisiert, (v) einer Wirtszelle, die ein erfindungsgemäß identifiziertes Tumor-assoziiertes Antigen oder einen Teil davon exprimiert, und (vi) isolierten Komplexen zwischen einem erfindungsgemäß identifizierten Tumor-assoziierten Antigen oder einem Teil davon und einem HLA-Molekül.

Eine Nukleinsäure, die für ein erfindungsgemäß identifiziertes Tumor-assoziiertes Antigen oder einen Teil davon kodiert, kann in der pharmazeutische Zusammensetzung in einem Expressionsvektor vorliegen und funktionell mit einem Promotor verbunden sein.

Eine in einer offenbarungsgemäßen pharmazeutischen Zusammensetzung enthaltene Wirtszelle kann das Tumor-assoziierte Antigen oder den Teil davon sekretieren, auf der Oberfläche exprimieren oder kann zusätzlich ein HLA-Molekül exprimieren, das an das Tumor-assoziierte Antigen oder den Teil davon bindet. In einer Ausführungsform exprimiert die Wirtszelle das HLA-Molekül endogen. In einer weiteren Ausführungsform exprimiert die Wirtszelle das HLA-Molekül und/oder das Tumor-assoziierte Antigen oder den Teil davon rekombinant. Vorzugsweise ist die Wirtszelle nicht-proliferativ. In einer bevorzugten Ausführungsform ist die Wirtszelle eine Antigen-präsentierende Zelle, insbesondere eine dendritische Zelle, ein Monozyt oder ein Makrophage.

Ein in einer offenbarungsgemäßen pharmazeutischen Zusammensetzung enthaltener Antikörper kann ein monoklonaler Antikörper sein. In weiteren Ausführungsformen ist der Antikörper ein chimärer oder humanisierter Antikörper, ein Fragment eines natürlichen Antikörpers, oder ein synthetischer Antikörper, die alle durch kombinatorische Techniken hergestellt werden können. Der Antikörper kann mit einem therapeutisch oder diagnostisch nützlichen Mittel oder Stoff gekoppelt sein.

Eine in einer offenbarungsgemäßen pharmazeutischen Zusammensetzung enthaltene Antisense-Nukleinsäure kann eine Sequenz von 6-50, insbesondere 10-30, 15-30 oder 20-30 zusammenhängenden Nukleotiden aus der Nukleinsäure, die für das erfindungsgemäß identifizierte Tumor-assoziierte Antigen kodiert, umfassen.

In weiteren Ausführungsformen bindet ein durch eine offenbarungsgemäße pharmazeutische Zusammensetzung entweder direkt oder durch die Expression einer Nukleinsäure bereitgestelltes Tumor-assoziiertes Antigen oder ein Teil davon an MHC-Moleküle auf der Oberfläche von Zellen, wobei die Bindung vorzugsweise eine cytolytische Reaktion hervorruft und/oder eine Cytokinausschüttung induziert.

Eine offenbarungsgemäße pharmazeutische Zusammensetzung kann einen pharmazeutisch verträglichen Träger und/oder ein Adjuvans umfassen. Das Adjuvans kann aus Saponin, GM-CSF, CpG-Nukleotiden, RNA, einem Cytokin oder einem Chemokin ausgewählt sein. Eine offenbarungsgemäße pharmazeutische Zusammensetzung wird vorzugsweise zur Behandlung einer Erkrankung eingesetzt, die sich durch die selektive Expression oder abnormale Expression eines Tumor-assoziierten Antigens auszeichnet. In einer bevorzugten Ausführungsform ist die Erkrankung Krebs.

Des weiteren wird ein Verfahren zur Behandlung oder Diagnose einer Erkrankung offenbart, die sich durch die Expression oder abnormalen Expression eines oder mehrerer Tumor-assoziierter Antigene auszeichnet. In einer Ausführungsform umfasst die Behandlung die Verabreichung einer pharmazeutischen Zusammensetzung.

In einem Aspekt wird ein Verfahren zur Diagnose einer Erkrankung offenbart, die sich durch die Expression oder abnormale Expression eines erfindungsgemäß identifizierten Tumor-assoziierten Antigens auszeichnet. Das Verfahren umfasst den Nachweis (i) einer Nukleinsäure, die für das Tumor-assoziierte Antigen kodiert, oder eines Teils davon und/oder (ii) den Nachweis des Tumor-assoziierten Antigens oder eines Teils davon, und/oder (iii) den Nachweis eines Antikörpers gegen das Tumor-assoziierte Antigen oder einen Teil davon und/oder (iv) den Nachweis von cytotoxischen oder Helfer-T-Lymphozyten, die für das Tumor-assoziierte Antigen oder einen Teil davon spezifisch sind in einer aus einem Patienten isolierten biologischen Probe. In bestimmten Ausführungsformen umfasst der Nachweis (i) die Kontaktierung der biologischen Probe mit einem Mittel, das spezifisch an die Nukleinsäure, die für das Tumor-assoziierte Antigen kodiert, oder den Teil davon, an das Tumor-assoziierte Antigen oder den Teil davon, an den Antikörper oder an cytotoxische oder Helfer-T-Lymphozyten, die für das Tumor-assoziierte Antigen oder Teile davon spezifisch sind, bindet und (ii) den Nachweis der Komplexbildung zwischen dem Mittel und der Nukleinsäure oder dem Teil davon, dem Tumor-assoziierten Antigen oder dem Teil davon, dem Antikörper oder den cytotoxischen oder Helfer-T-Lymphozyten. In einer Ausführungsform zeichnet sich die Erkrankung durch die Expression oder abnormale Expression mehrerer verschiedener Tumor-assoziierter Antigene aus und der Nachweis umfasst einen Nachweis mehrerer Nukleinsäuren, die für die mehreren verschiedenen Tumor-assoziierten Antigene kodieren, oder von Teilen davon, den Nachweis der mehreren verschiedenen Tumor-assoziierten Antigene oder von Teilen davon, den Nachweis mehrerer Antikörper, die an die mehreren verschiedenen Tumor-assoziierten Antigene oder an Teile davon binden oder den Nachweis mehrerer cytotoxischer oder Helfer-T-Lymphozyten, die für die mehreren verschiedenen Tumor-assoziierten Antigene spezifisch sind. In einer weiteren Ausführungsform wird die isolierte biologische Probe aus dem Patienten mit einer vergleichbaren normalen biologischen Probe verglichen.

In einem weiteren Aspekt wird ein Verfahren zur Bestimmung der Regression, des Verlaufs oder des Ausbruchs einer Erkrankung offenbart, die sich durch die Expression oder abnormale Expression eines erfindungsgemäß identifizierten Tumor-assoziierten Antigens auszeichnet, umfassend die Überwachung einer Probe aus einem Patienten, der die Erkrankung aufweist oder in Verdacht steht, an der Erkrankung zu erkranken in Bezug auf einen oder mehrere Parameter, die aus der Gruppe ausgewählt sind, bestehend aus (i) der Menge der Nukleinsäure, die für das Tumor-assoziierte Antigen kodiert, oder eines Teil davon, (ii) der Menge des Tumor-assoziierten Antigens oder eines Teils davon, (iii) der Menge an Antikörpern, die an das Tumor-assoziierte Antigen oder einen Teil davon binden, und (iv) der Menge an cytolytischen T-Zellen oder Helfer-T-Zellen, die für einen Komplex zwischen dem Tumor-assoziierten Antigen oder einem Teil davon und einem MHC-Molekül spezifisch sind. Vorzugsweise umfasst das Verfahren die Bestimmung des oder der Parameter zu einem ersten Zeitpunkt in einer ersten Probe und zu einem zweiten Zeitpunkt in einer weiteren Probe, wobei durch einen Vergleich der beiden Proben der Verlauf der Erkrankung ermittelt wird. In bestimmten Ausführungsformen zeichnet sich die Erkrankung durch die Expression oder abnormale Expression mehrerer verschiedener Tumor-assoziierter Antigene aus und die Überwachung umfasst eine Überwachung (i) der Menge mehrerer Nukleinsäuren, die für die mehreren verschiedenen Tumor-assoziierten Antigene kodieren, oder von Teilen davon und/oder (ii) der Menge der mehreren verschiedenen Tumor-assoziierten Antigene oder von Teilen davon und/oder (iii) der Menge mehrerer Antikörper, die an die mehreren verschiedenen Tumor-assoziierten Antigene oder an Teile davon binden, und/oder (iv) der Menge mehrerer cytolytischer T-Zellen oder Helfer-T-Zellen, die für Komplexe zwischen den mehreren verschiedenen Tumor-assoziierten Antigenen oder von Teilen davon und MHC-Molekülen spezifisch sind.

Ein Nachweis einer Nukleinsäure oder eines Teils davon oder eine Überwachung der Menge einer Nukleinsäure oder eines Teils davon kann mit einer Polynukleotid-Sonde erfolgen, die spezifisch mit der Nukleinsäure oder dem Teil davon hybridisiert, oder kann durch selektive Amplifikation der Nukleinsäure oder des Teils davon erfolgen. In einer Ausführungsform umfasst die Polynukleotid-Sonde eine Sequenz von 6-50, insbesondere 10-30, 15-30 oder 20-30 zusammenhängenden Nukleotiden aus der Nukleinsäure.

In bestimmten Ausführungsformen liegt das nachzuweisende Tumor-assoziierte Antigen oder der Teil davon intrazellulär oder auf der Zelloberfläche vor. Ein Nachweis eines Tumor-assoziierten Antigens oder eines Teils davon oder eine Überwachung der Menge eines Tumor-assoziierten Antigens oder eines Teils davon kann erfindungsgemäß mit einem Antikörper erfolgen, der spezifisch an das Tumor-assoziierte Antigen oder den Teil davon bindet.

In weiteren Ausführungsformen liegt das nachzuweisende Tumor-assoziierte Antigen oder der Teil davon in einem Komplex mit einem MHC-Molekül, insbesondere einem HLA-Molekül vor.

Ein Nachweis eines Antikörpers oder die Überwachung der Menge an Antikörpern kann mit einem Protein oder Peptid erfolgen, das spezifisch an den Antikörper bindet.

Ein Nachweis von cytolytischen T-Zellen oder Helfer-T-Zellen oder die Überwachung der Menge an cytolytischen T-Zellen oder Helfer-T-Zellen, die für Komplexe zwischen einem Antigen oder einem Teil davon und MHC-Molekülen spezifisch sind, kann erfindungsgemäß mit einer Zelle erfolgen, die den Komplex zwischen dem Antigen oder dem Teil davon und einem MHC-Molekül präsentiert.

Die für einen Nachweis oder für eine Überwachung verwendete Polynukleotid-Sonde, der Antikörper, das Protein oder Peptid oder die Zelle sind vorzugsweise nachweisbar markiert. In bestimmten Ausführungsformen ist der nachweisbare Marker ein radioaktiver Marker oder ein Enzymmarker. Der Nachweis von T-Lymphozyten kann zusätzlich durch Nachweis ihrer Proliferation, ihrer Zytokinproduktion, sowie ihrer cytotoxischen Aktivität erfolgen, die durch die spezifische Stimulation mit dem Komplex aus MHC und Tumor-assoziiertem Antigen oder Teilen davon ausgelöst wird. Der Nachweis von T-Lymphozyten kann ferner durch ein rekombinantes MHC-Molekül oder auch einen Komplex aus mehreren MHC-Molekülen, die mit dem jeweiligen immunogenen Fragment aus einem oder mehreren der Tumor-assoziierten Antigene beladen sind, und durch Kontaktierung des spezifischen T-Zell-Rezeptors erfolgen, wodurch spezifische T-Lymphozyten identifiziert werden können.

In einem weiteren Aspekt wird ein Verfahren zur Behandlung, Diagnose oder Überwachung einer Erkrankung offenbart, die sich durch die Expression oder abnormale Expression eines erfindungsgemäß identifizierten Tumor-assoziierten Antigens auszeichnet, umfassend die Verabreichung eines Antikörpers, der an das Tumor-assoziierte Antigen oder einen Teil davon bindet und mit einem therapeutischen oder diagnostischen Mittel oder Stoff gekoppelt ist. Der Antikörper kann ein monoklonaler Antikörper sein. In weiteren Ausführungsformen ist der Antikörper ein chimärer oder humanisierter Antikörper oder ein Fragment eines natürlichen Antikörpers.

Die Offenbarung betrifft auch ein Verfahren zur Behandlung eines Patienten mit einer Erkrankung, die sich durch die Expression oder abnormale Expression eines erfindungsgemäß identifizierten Tumor-assoziierten Antigens auszeichnet, umfassend (i) die Entfernung einer Probe mit immunreaktiven Zellen aus dem Patienten, (ii) die Kontaktierung der Probe mit einer Wirtszelle, die das Tumor-assoziierte Antigen oder einen Teil davon exprimiert, unter Bedingungen, die eine Produktion cytolytischer T-Zellen gegen das Tumor-assoziierte Antigen oder einen Teil davon begünstigen, und (iii) das Einbringen der cytolytischen T-Zellen in den Patienten in einer Menge, die geeignet ist, Zellen zu lysieren, die das Tumor-assoziierte Antigen oder einen Teil davon exprimieren. Die Offenbarung betrifft ebenfalls die Klonierung des T-Zell-Rezeptors von cytolytischen T-Zellen gegen das Tumor-assoziierte Antigen. Dieser kann in andere T-Zellen transferiert werden, die damit die erwünschte Spezifität erhalten und wie unter (iii) in den Patienten eingebracht werden können.

In einer Ausführungsform exprimiert die Wirtszelle ein HLA-Molekül endogen. In einer weiteren Ausführungsform exprimiert die Wirtszelle ein HLA-Molekül und/oder das Tumor-assoziierte Antigen oder den Teil davon rekombinant. Vorzugsweise ist die Wirtszelle nicht-proliferativ. In einer bevorzugten Ausführungsform ist die Wirtszelle eine Antigen-präsentierende Zelle, insbesondere eine dendritische Zelle, ein Monozyt oder ein Makrophage.

In einem weiteren Aspekt wird ein Verfahren zur Behandlung eines Patienten mit einer Erkrankung offenbart, die sich durch die Expression oder abnormale Expression eines Tumor-assoziierten Antigens auszeichnet, umfassend (i) die Identifizierung einer für ein erfindungsgemäß identifiziertes Tumor-assoziiertes Antigen kodierenden Nukleinsäure, die von Zellen exprimiert wird, die mit der Erkrankung assoziiert sind, (ii) die Transfektion einer Wirtszelle mit der Nukleinsäure oder einem Teil davon, (iii) die Kultivierung der transfizierten Wirtszelle für eine Expression der Nukleinsäure (dies ist bei Erreichen einer hohen Transfektionsrate nicht obligat), und (iv) das Einbringen der Wirtszellen oder eines Extrakts davon in den Patienten in einer Menge, die geeignet ist, die Immunreaktion gegen die Zellen des Patienten, die mit der Erkrankung assoziiert sind, zu erhöhen. Das Verfahren kann ferner die Identifizierung eines MHC-Moleküls, das das Tumor-assoziierte Antigen oder einen Teil davon präsentiert, umfassen, wobei die Wirtszelle das identifizierte MHC-Molekül exprimiert und das Tumor-assoziierte Antigen oder einen Teil davon präsentiert. Die Immunreaktion kann eine B-Zellen-Reaktion oder eine T-Zellen-Reaktion umfassen. Des weiteren kann eine T-Zellen-Reaktion die Produktion von cytolytischen T-Zellen und/oder Helfer-T-Zellen umfassen, die spezifisch für die Wirtszellen sind, die das Tumor-assoziierte Antigen oder einen Teil davon präsentieren oder spezifisch für Zellen des Patienten sind, die das Tumor-assoziierte Antigen oder einen Teil davon exprimieren.

Die Offenbarung betrifft auch ein Verfahren zur Behandlung einer Erkrankung, die sich durch die Expression oder abnormale Expression eines erfindungsgemäß identifizierten Tumor-assoziierten Antigens auszeichnet, umfassend (i) die Identifikation von Zellen aus dem Patienten, die abnormale Mengen des Tumor-assoziierten Antigens exprimieren, (ii) die Isolierung einer Probe der Zellen, (iii) die Kultivierung der Zellen und (iv) das Einbringen der Zellen in den Patienten in einer Menge, die geeignet ist, eine Immunreaktion gegen die Zellen auszulösen.

Vorzugsweise sind die offenbarungsgemäß verwendeten Wirtszellen nicht-proliferativ oder werden nicht-proliferativ gemacht. Eine Erkrankung, die sich durch die Expression oder abnormale Expression eines Tumor-assoziierten Antigens auszeichnet, ist insbesondere Krebs.

In einem weiteren Aspekt betrifft die Offenbarung Promotorsequenzen von erfindungsgemäßen Nukleinsäuren. Diese können funktionell mit einem anderen Gen vorzugsweise in einem Expressionsvektor verbunden werden und somit die selektive Expression dieses Gens in entsprechenden Zellen gewährleisten.

In einem weiteren Aspekt wird ein rekombinantes Nukleinsäuremolekül offenbart, insbesondere DNA- oder RNA-Molekül, das eine offenbarungsgemäße Nukleinsäure umfasst.

Die Offenbarung betrifft auch Wirtszellen, die eine offenbarungsgemäße Nukleinsäure oder ein rekombinantes Nukleinsäuremolekül, das eine offenbarungsgemäße Nukleinsäure umfasst, enthalten.

Die Wirtszelle kann ferner eine Nukleinsäure umfassen, die für ein HLA-Molekül kodiert. In einer Ausführungsform exprimiert die Wirtszelle das HLA-Molekül endogen. In einer weiteren Ausführungsform exprimiert die Wirtszelle das HLA-Molekül und/oder die offenbarungsgemäße Nukleinsäure oder einen Teil davon rekombinant. Vorzugsweise ist die Wirtszelle nicht-proliferativ. In einer bevorzugten Ausführungsform ist die Wirtszelle eine Antigen-präsentierende Zelle, insbesondere eine dendritische Zelle, ein Monozyt oder ein Makrophage.

In einer weiteren Ausführungsform betrifft die Offenbarung Oligonukleotide, die mit einer offenbarungsgemäß identifizierten Nukleinsäure hybridisieren und als genetische Sonden oder als "Antisense"-Moleküle verwendet werden können. Nukleinsäuremoleküle in der Form von Oligonukleotid-Primern oder kompetenten Proben, die mit einer erfindungsgemäß identifizierten Nukleinsäure oder Teilen davon hybridisieren, können zum Auffinden von Nukleinsäuren verwendet werden, die zu der erfindungsgemäß identifizierten Nukleinsäure homolog sind. PCR-Amplifikation, Southern- und Northern-Hybridisierung können zum Auffinden homologer Nukleinsäuren eingesetzt werden. Die Hybridisierung kann unter niedrig-, besser unter mittel- und am besten unter hoch-stringenten Bedingungen erfolgen. Der Begriff "stringente Bedingungen" betrifft erfindungsgemäß Bedingungen, die eine spezifische Hybridisierung zwischen Polynukleotiden erlauben.

In einem weiteren Aspekt betrifft die Erfindung ein immunogenes Fragment eines identifizierten Tumor-assoziierten Antigens. Das Fragment bindet vorzugsweise an einen menschlichen HLA-Rezeptor oder menschlichen Antikörper. Vorzugsweise umfasst ein Fragment eine Sequenz von mindestens 6, insbesondere mindestens 8, mindestens 10, mindestens 12, mindestens 15, mindestens 20, mindestens 30 oder mindestens 50 Aminosäuren.

In diesem Aspekt betrifft die Offenbarung insbesondere ein Peptid, das eine Sequenz, ausgewählt aus der Gruppe bestehend aus SEQ ID NO: 17-19, 111-116, 120, und 137, einem Teil oder Derivat davon aufweist oder umfasst.

In einem weiteren Aspekt betrifft die Erfindung ein Mittel, das an ein erfindungsgemäß identifiziertes Tumor-assoziiertes Antigen oder an einen Teil davon bindet. In einer bevorzugten Ausführungsform ist das Mittel ein Antikörper. In weiteren Ausführungsformen ist der Antikörper ein chimärer, ein humanisierter oder mit kombinatorischen Techniken hergestellter Antikörper oder ein Fragment eines Antikörpers. Des weiteren wird ein Antikörper offenbart, der selektiv an einen Komplex aus (i) einem erfindungsgemäß identifizierten Tumor-assoziierten Antigen oder einem Teil davon und (ii) einem MHC-Molekül bindet, an das das erfindungsgemäß identifizierte Tumor-assoziierte Antigen oder der Teil davon bindet, wobei der Antikörper nicht alleine an (i) oder (ii) bindet. Ein erfindungsgemäßer Antikörper kann ein monoklonaler Antikörper sein. In weiteren Ausführungsformen ist der Antikörper ein chimärer oder humanisierter Antikörper oder ein Fragment eines natürlichen Antikörpers.

Insbesondere betrifft die Erfindung ein solches Mittel, insbesondere einen Antikörper, das/der spezifisch an ein Peptid bindet, das eine Sequenz ausgewählt aus der Gruppe bestehend aus SEQ ID NO: 17-19, 111-116, 120, und 137, einem Teil oder Derivat davon aufweist oder umfasst.

Des weiteren betrifft die Erfindung ein Konjugat zwischen einem erfindungsgemäßen Mittel, das an ein erfindungsgemäß identifiziertes Tumor-assoziiertes Antigen oder an einen Teil davon bindet, oder einem erfindungsgemäßen Antikörper und einem therapeutischen oder diagnostischen Mittel oder Stoff. In einer Ausführungsform ist das therapeutische oder diagnostische Mittel ein Toxin.

In einem weiteren Aspekt wird ein Kit zum Nachweis der Expression oder abnormalen Expression eines erfindungsgemäß identifizierten Tumor-assoziierten Antigens offenbart, umfassend Mittel zum Nachweis (i) der Nukleinsäure, die für das Tumor-assoziierte Antigen kodiert, oder eines Teils davon, (ii) des Tumor-assoziierten Antigens oder eines Teils davon, (iii) von Antikörpern, die an das Tumor-assoziierte Antigen oder einen Teil davon binden, und/oder (iv) von T-Zellen, die für einen Komplex zwischen dem Tumor-assoziierten Antigen oder einem Teil davon und einem MHC-Molekül spezifisch sind. In einer Ausführungsform sind die Mittel zum Nachweis der Nukleinsäure oder des Teils davon Nukleinsäuremoleküle für die selektive Amplifikation der Nukleinsäure, die insbesondere eine Sequenz von 6-50, insbesondere 10-30, 15-30 oder 20-30 zusammenhängenden Nukleotiden aus der Nukleinsäure umfassen.

### Detaillierte Beschreibung der Erfindung

Erfindungsgemäß werden Gene beschrieben, die in Tumorzellen selektiv exprimiert oder aberrant exprimiert werden und Tumor-assoziierte Antigene darstellen.

Erfindungsgemäß sind diese Gene und/oder deren Genprodukte und/oder ihre Derivate und/oder Teile bevorzugte Zielstrukturen für therapeutische Ansätze. Konzeptionell können die therapeutischen Ansätze auf eine Hemmung der Aktivität des selektiv exprimierten Tumor-assoziierten Genproduktes zielen. Dies ist dann sinnvoll, wenn die aberrante respektive selektive Expression funktionell von tumorpathogenetischer Bedeutung ist und ihre Unterbindung mit einer selektiven Schädigung der entsprechenden Zellen einhergeht. Andere therapeutische Konzepte betrachten Tumor-assoziierte Antigene als Markierungen, die Effektormechanismen mit zellschädigendem Potential selektiv zu Tumorzellen rekrutieren. Hierbei ist die Funktion des Zielmoleküls selbst und seine Rolle bei der Tumorentstehung vollkommen unerheblich.

Mit "Derivat" einer Nukleinsäure ist erfindungsgemäß gemeint, dass einzelne oder multiple Nukleotidsubstitutionen, -deletionen und/oder -additionen in der Nukleinsäure vorliegen. Weiterhin umfasst der Begriff "Derivat" auch eine chemische Derivatisierung einer Nukleinsäure an einer Nukleotidbase, am Zucker oder am Phosphat. Der Begriff "Derivat" umfasst auch Nukleinsäuren, die nicht in der Natur vorkommende Nukleotide und Nukleotidanaloga enthalten.

Eine Nukleinsäure ist erfindungsgemäß vorzugsweise Desoxyribonukleinsäure (DNA) oder Ribonukleinsäure (RNA). Nukleinsäuren umfassen erfindungsgemäß genomische DNA, cDNA, mRNA, rekombinant hergestellte und chemisch synthetisierte Moleküle. Eine Nukleinsäure kann erfindungsgemäß als einzelsträngiges oder doppelsträngiges und lineares oder kovalent kreisförmig geschlossenes Molekül vorliegen.

Die erfindungsgemäß beschriebenen Nukleinsäuren sind vorzugsweise isoliert. Der Begriff "isolierte Nukleinsäure" bedeutet erfindungsgemäß, dass die Nukleinsäure (i) *in vitro* amplifiziert wurde, zum Beispiel durch Polymerase-Kettenreaktion (PCR), (ii) rekombinant durch Klonierung produziert wurde, (iii) gereinigt wurde, zum Beispiel durch Spaltung und gelelektrophoretische Auftrennung, oder (iv) synthetisiert wurde, zum Beispiel durch chemische Synthese. Eine isolierte Nukleinsäure ist eine Nukleinsäure, die für eine Manipulierung durch rekombinante DNA-Techniken zur Verfügung steht.

Eine Nukleinsäure ist dann zu einer anderen Nukleinsäure "komplementär", wenn die beiden Sequenzen miteinander hybridisieren und ein stabiles Duplex eingehen können, wobei die Hybridisierung vorzugsweise unter Bedingungen erfolgt, die eine spezifische Hybridisierung zwischen Polynukleotiden erlauben (stringente Bedingungen). Stringente Bedingungen sind beispielsweise in Molecular Cloning: A Laboratory Manual, J. Sambrook et al., Hrsg., 2. Auflage, Cold Spring Harbor Laboratory Press, Cold Spring Harbor, New York, 1989 oder Current Protocols in Molecular Biology, F.M. Ausubel et al., Hrsg., John Wiley & Sons, Inc., New York beschrieben und betreffen beispielsweise die Hybridisierung bei 65°C in Hybridisierungspuffer (3,5 x SSC, 0,02% Ficoll, 0,02% Polyvinylpyrrolidon, 0,02% Rinderserumalbumin, 2,5mM NaH₂PO₄ (pH7), 0,5% SDS, 2mM EDTA). SSC ist 0,15 M Natriumchlorid/ 0,15 M Natriumcitrat, pH 7. Nach der Hybridisierung wird die Membran, auf die die DNA übertragen wurde beispielsweise in 2 x SSC bei Raumtemperatur und sodann in 0,1 - 0,5 x SSC/ 0,1 x SDS bei Temperaturen bis 68°C gewaschen.

Komplementäre Nukleinsäuren weisen erfindungsgemäß mindestens 40%, insbesondere mindestens 50%, mindestens 60%, mindestens 70%, mindestens 80%, mindestens 90% und vorzugsweise mindestens 95%, mindestens 98% oder mindestens 99% Identität der Nukleotide auf.

Nukleinsäuren, die für Tumor-assoziierte Antigene kodieren, können erfindungsgemäß alleine oder in Kombination mit anderen Nukleinsäuren, insbesondere heterologen Nukleinsäuren, vorliegen. In bevorzugten Ausführungsformen liegt eine Nukleinsäure funktionell in Verbindung mit Expressionskontrollsequenzen oder regulatorischen Sequenzen vor, die in Bezug zu der Nukleinsäure homolog oder heterolog sein können. Eine kodierende Sequenz und eine regulatorische Sequenz sind dann "funktionell" miteinander verbunden, falls sie derart kovalent miteinander verknüpft sind, dass die Expression oder Transkription der kodierenden Sequenz unter der Kontrolle oder unter dem Einfluss der regulatorischen Sequenz steht. Falls die kodierende Sequenz in ein funktionelles Protein translatiert werden soll, führt bei einer funktionellen Verbindung einer regulatorischen Sequenz mit der kodierenden Sequenz eine Induktion der regulatorischen Sequenz zu einer Transkription der kodierenden Sequenz, ohne dass es zu einer Leserasterverschiebung in der kodierenden Sequenz oder zu einem Unvermögen der kodierenden Sequenz kommt, in das gewünschte Protein oder Peptid translatiert zu werden.

Der Begriff "Expressionskontrollsequenz" oder "regulatorische Sequenz" umfasst erfindungsgemäß Promotoren, Enhancer und andere Kontrollelemente, die die Expression eines Gens steuern. In bestimmten erfindungsgemäßen Ausführungsformen sind die Expressionskontrollsequenzen regulierbar. Die genaue Struktur von regulatorischen Sequenzen kann speziesabhängig oder zelltypusabhängig variieren, umfasst jedoch im allgemeinen 5'-nicht-transkribierte und 5'-nicht-translatierte Sequenzen, die an der Initiation der Transkription bzw. Translation beteiligt sind wie TATA-Box, Capping-Sequenz, CAAT-Sequenz und ähnliches. Insbesondere umfassen 5'-nicht-transkribierte Regulationssequenzen eine Promotorregion, die eine Promotorsequenz für eine transkriptionelle Kontrolle des funktionell verbundenen Gens einschließt. Regulatorische Sequenzen können auch Enhancer-Sequenzen oder stromaufwärts gelegene Aktivatorsequenzen umfassen.

Zum einen können also die hier dargestellten Tumor-assoziierten Antigene mit beliebigen Expressionskontrollsequenzen und Promotoren kombiniert werden. Zum anderen aber können erfindungsgemäß die Promotoren der hier dargestellten Tumor-assoziierten Genprodukte mit beliebigen anderen Genen kombiniert werden. Dies erlaubt, die selektive Aktivität dieser Promotoren zu nutzen.

Des weiteren kann eine Nukleinsäure erfindungsgemäß in Verbindung mit einer anderen Nukleinsäure vorliegen, die für ein Polypeptid kodiert, das eine Sekretion des durch die Nukleinsäure kodierten Proteins oder Polypeptids aus einer Wirtszelle steuert. Auch kann eine Nukleinsäure erfindungsgemäß in Verbindung mit einer anderen Nukleinsäure vorliegen, die für ein Polypeptid kodiert, das eine Verankerung des kodierten Proteins oder Polypeptids auf der Zellmembran der Wirtszelle oder seine Kompartimentalisierung in bestimmte Organellen dieser Zelle herbeiführt. Gleichermaßen kann eine Verbindung mit einer Nukleinsäure erfolgen, die ein Reportergen oder einen beliebigen "Tag" darstellt.

In einer bevorzugten Ausführungsform ist ein rekombinantes DNA-Molekül erfindungsgemäß ein Vektor, gegebenenfalls mit einem Promotor, der die Expression einer Nukleinsäure, z.B. einer Nukleinsäure, die für eine erfindungsgemäßes Tumor-assoziiertes Antigen kodiert, steuert. Der Begriff "Vektor" wird dabei in seiner allgemeinsten Bedeutung verwendet und umfasst jegliche intermediären Vehikel für eine Nukleinsäure, die es z.B. ermöglichen, die Nukleinsäure in prokaryotische und/oder in eukaryotische Zellen einzubringen und gegebenenfalls in ein Genom zu integrieren. Solche Vektoren werden vorzugsweise in der Zelle repliziert und/oder exprimiert. Ein intermediäres Vehikel kann z.B. für den Gebrauch bei der Elektroporation, beim Mikroprojektilbeschuss, bei der liposomalen Verabreichung, beim Transfer mit Hilfe von Agrobakterien oder bei der Insertion über DNA- oder RNA-Viren angepasst sein. Vektoren umfassen Plasmide, Phagemide oder Virusgenome.

Die Nukleinsäuren, die für ein erfindungsgemäß identifiziertes Tumor-assoziiertes Antigen kodieren, können für eine Transfektion von Wirtszellen eingesetzt werden. Mit Nukleinsäuren ist dabei sowohl rekombinante DNA wie auch RNA gemeint. Rekombinante RNA kann durch in vitro-Transkription von einer DNA-Matritze hergestellt werden. Sie kann des weiteren vor Applikation durch stabilisierende Sequenzen, Capping und Poly-Adenylierung modifiziert werden.

Der Begriff "Wirtszelle" betrifft erfindungsgemäß jede Zelle, die mit einer exogenen Nukleinsäure transformierbar oder transfizierbar ist. Der Begriff "Wirtszellen" umfasst erfindungsgemäß prokaryontische (z.B. *E. coli*) oder eukaryontische (z.B. dendritische Zellen, B-Zellen, CHO-Zellen, COS-Zellen, K562-Zellen, Hefezellen und Insektenzellen). Besonders bevorzugt sind Säugerzellen wie Zellen aus Mensch, Maus, Hamster, Schwein, Ziege, Primaten. Die Zellen können aus einer Vielzahl von Gewebetypen abgeleitet sein und umfassen primäre Zellen und Zelllinien. Spezifische Beispiele umfassen Keratinozyten, periphere Blutleukozyten, Stammzellen des Knochenmarks und embryonale Stammzellen. In weiteren Ausführungsformen ist die Wirtszelle eine Antigen-präsentierende Zelle, insbesondere eine dendritische Zelle, ein Monozyt oder ein Makrophage. Eine Nukleinsäure kann in der Wirtszelle in einer einzigen oder in mehreren Kopien vorliegen und wird in einer Ausführungsform in der Wirtszelle exprimiert.

Der Begriff "Expression" wird erfindungsgemäß in seiner allgemeinsten Bedeutung verwendet und umfasst die Produktion von RNA oder von RNA und Protein. Er umfasst auch eine teilweise Expression von Nukleinsäuren. Des weiteren kann die Expression transient oder stabil erfolgen. Bevorzugte Expressionssysteme in Säugerzellen umfassen pcDNA3.1 und pRc/CMV (Invitrogen, Carlsbad, CA), die einen selektierbaren Marker enthalten wie ein Gen, das eine Resistenz gegenüber G418 verleiht (und somit eine Selektion stabil transfizierter Zelllinien ermöglicht) und die Enhancer-Promotor-Sequenzen von Cytomegalovirus (CMV).

In den Fällen der Erfindung, in denen ein HLA-Molekül ein Tumor-assoziiertes Antigen oder einen Teil davon präsentiert, kann ein Expressionsvektor auch eine Nukleinsäuresequenz umfassen, die für das HLA-Molekül kodiert. Die Nukleinsäuresequenz, die für das HLA-Molekül kodiert, kann auf demselben Expressionsvektor wie die Nukleinsäure, die für das Tumor-assoziierte Antigen oder den Teil davon kodiert, vorliegen oder beide Nukleinsäuren können auf verschiedenen Expressionsvektoren vorliegen. Im letzteren Fall können die beiden Expressionsvektoren in eine Zelle cotransfiziert werden. Falls eine Wirtszelle weder das Tumor-assoziierte Antigen oder den Teil davon noch das HLA-Molekül exprimiert, werden beide dafür kodierenden Nukleinsäuren entweder auf demselben Expressionsvektor oder auf verschiedenen Expressionsvektoren in die Zelle transfiziert. Falls die Zelle bereits das HLA-Molekül exprimiert, kann nur die Nukleinsäuresequenz, die für das Tumor-assoziierte Antigen oder den Teil davon kodiert, in die Zelle transfiziert werden.

Offenbart sind Kits zur Amplifikation einer Nukleinsäure, die für ein Tumor-assoziiertes Antigen kodiert. Solche Kits umfassen beispielsweise ein Paar von Amplifikationsprimern, die an die Nukleinsäure hybridisieren, die für das Tumor-assoziierte Antigen kodiert. Die Primer umfassen vorzugsweise eine Sequenz von 6-50, insbesondere 10-30, 15-30 oder 20-30 zusammenhängenden Nukleotiden aus der Nukleinsäure und sind nicht-überlappend, um die Bildung von Primer-Dimeren zu vermeiden. Einer der Primer wird an einen Strang der Nukleinsäure hybridisieren, die für das Tumor-assoziierte Antigen kodiert, und der andere Primer wird an den komplementären Strang in einer Anordnung hybridisieren, die eine Amplifikation der Nukleinsäure, die für das Tumor-assoziierte Antigen kodiert, erlaubt.

"Antisense"-Moleküle oder "Antisense"-Nukleinsäuren können zur Regulierung, insbesondere der Reduktion der Expression einer Nukleinsäure verwendet werden. Der Begriff "Antisense-Molekül" oder "Antisense-Nukleinsäure" betrifft offenbarungsgemäß ein Oligonukleotid, das ein Oligoribonukleotid, Oligodesoxyribonukleotid, modifiziertes Oligoribonukleotid oder modifiziertes Oligodesoxyribonukleotid ist und das unter physiologischen Bedingungen an DNA, die ein bestimmtes Gen umfasst, oder mRNA dieses Gens hybridisiert, wodurch die Transkription dieses Gens und/oder die Translation dieser mRNA gehemmt wird. Ein "Antisense-Molekül" umfasst offenbarungsgemäß auch ein Konstrukt, das eine Nukleinsäure oder einen Teil davon in reverser Orientierung in Bezug auf ihren natürlichen Promotor enthält. Ein Antisense-Transkript einer Nukleinsäure oder eines Teils davon kann eine Duplex mit der natürlich vorkommenden mRNA, die das Enzym spezifiziert, eingehen und so eine Akkumulation von oder die Translation der mRNA in das aktive Enzym verhindern. Eine weitere Möglichkeit ist die Verwendung von Ribozymen zur Inaktivierung einer Nukleinsäure. Bevorzugte Antisense-Oligonukleotide weisen eine Sequenz von 6-50, insbesondere 10-30, 15-30 oder 20-30 zusammenhängenden Nukleotiden aus der Ziel-Nukleinsäure auf und sind vorzugsweise vollständig zu der Ziel-Nukleinsäure oder einem Teil davon komplementär.

In bevorzugten Ausführungsformen hybridisiert das Antisense-Oligonukleotid mit einer N-terminalen oder 5'-stromaufwärts gelegenen Stelle wie einer Translationsinitiations-, Transkriptionsinitiations- oder Promotorstelle. In weiteren Ausführungsformen hybridisiert das Antisense-Oligonukleotid mit einer 3'-nicht-translatierten Region oder mRNA-Splicing-Stelle.

In einer Ausführungsform besteht ein Oligonukleotid aus Ribonukleotiden, Desoxyribonukleotiden oder einer Kombination davon. Dabei sind das 5'-Ende eines Nukleotids und das 3'-Ende eines anderen Nukleotids durch eine Phosphodiesterbindung miteinander verknüpft. Diese Oligonukleotide können in herkömmlicher Weise synthetisiert oder rekombinant produziert werden.

In bevorzugten Ausführungsformen ist ein Oligonukleotid ein "modifiziertes" Oligonukleotid. Dabei kann das Oligonukleotid, um beispielsweise seine Stabilität oder therapeutische Wirksamkeit zu erhöhen, auf verschiedenste Art und Weise modifiziert sein ohne dass seine Fähigkeit, an sein Ziel zu binden, beeinträchtigt wird. Der Begriff "modifiziertes Oligonukleotid" bedeutet ein Oligonukleotid, bei dem (i) mindestens zwei seiner Nukleotide durch eine synthetische Internukleosidbindung (d.h. eine Internukleosidbindung, die keine Phosphodiesterbindung ist) miteinander verknüpft sind und/oder (ii) eine chemische Gruppe kovalent mit dem Oligonukleotid verbunden ist, die normalerweise nicht bei Nukleinsäuren auftritt. Bevorzugte synthetische Internukleosidbindungen sind Phosphorothioate, Alkylphosphonate, Phosphorodithioate, Phosphatester, Alkylphosphonothioate, Phosphoramidate, Carbamate, Carbonate, Phosphattriester, Acetamidate, Carboxymethylester und Peptide.

Der Begriff "modifiziertes Oligonukleotid" umfasst auch Oligonukleotide mit einer kovalent modifizierten Base und/oder Zucker. "Modifizierte Oligonukleotide" umfassen beispielsweise Oligonukleotide mit Zuckerresten, die kovalent an organische Gruppen mit einem geringen Molekulargewicht gebunden sind, die keine Hydroxylgruppe an der 3'-Position und keine Phosphatgruppe an der 5'-Position sind. Modifizierte Oligonukleotide können beispielsweise einen 2'-O-alkylierten Riboserest oder einen anderen Zucker anstelle von Ribose wie Arabinose umfassen.

Die erfindungsgemäß beschriebenen Proteine und Polypeptide sind vorzugsweise isoliert. Die Begriffe "isoliertes Protein" oder "isoliertes Polypeptid" bedeuten, dass das Protein oder Polypeptid von seiner natürlichen Umgebung getrennt ist. Ein isoliertes Protein oder Polypeptid kann in einem im Wesentlichen aufgereinigten Zustand vorliegen. Der Begriff "im Wesentlichen aufgereinigt" bedeutet, dass das Protein oder Polypeptid im Wesentlichen frei von anderen Substanzen vorliegt, mit denen es in der Natur oder *in vivo* vorliegt.

Solche Proteine und Polypeptide dienen beispielsweise der Herstellung von Antikörpern und sind in einem immunologischen oder diagnostischen Assay oder als Therapeutika einsetzbar. Erfindungsgemäß beschriebene Proteine und Polypeptide können aus biologischen Proben wie Gewebe- oder Zellhomogenaten isoliert werden und können auch rekombinant in einer Vielzahl pro- oder eukaryontischer Expressionssysteme exprimiert werden.

"Derivate" eines Proteins oder Polypeptids oder einer Aminosäuresequenz im Sinne dieser Erfindung umfassen Aminosäure-Insertionsvarianten, Aminosäure-Deletionsvarianten und/oder Aminosäure-Substitutionsvarianten.

Aminosäure-Insertionsvarianten umfassen amino- und/oder carboxyterminale Fusionen, sowie Insertionen von einzelnen oder mehreren Aminosäuren in einer bestimmten Aminosäuresequenz. Bei Aminosäure-Sequenzvarianten mit einer Insertion werden ein oder mehrere Aminosäurereste in eine vorbestimmte Stelle in einer Aminosäuresequenz eingebracht, obwohl eine zufällige Insertion mit geeignetem Screening des resultierenden Produkts auch möglich ist. Aminosäure-Deletionsvarianten sind durch das Entfernen von einer oder mehreren Aminosäuren aus der Sequenz charakterisiert. Aminosäure-Substitutionsvarianten zeichnen sich dadurch aus, dass wenigstens ein Rest in der Sequenz entfernt und ein anderer Rest an dessen Stelle eingefügt wird. Vorzugsweise befinden sich die Modifikationen an Positionen in der Aminosäuresequenz, die zwischen homologen Proteinen oder Polypeptiden nicht konserviert sind. Vorzugsweise werden Aminosäuren durch andere mit ähnlichen Eigenschaften ersetzt, wie Hydrophobizität, Hydrophilizität, Elektronegativität, Volumen der Seitenkette und ähnliches (konservative Substitution). Konservative Substitutionen betreffen beispielsweise den Austausch einer Aminosäure durch eine andere, nachstehend in derselben Gruppe wie die substituierte Aminosäure aufgeführte Aminosäure:
1. kleine aliphatische, nicht-polare oder leicht-polare Reste: Ala, Ser, Thr (Pro, Gly)
2. negativ geladene Reste und ihre Amide: Asn, Asp, Glu, Gln
3. positiv geladene Reste: His, Arg, Lys
4. große aliphatische, nicht-polare Reste: Met, Leu, Ile, Val (Cys)
5. große aromatische Reste: Phe, Tyr, Trp.

Drei Reste sind aufgrund ihrer besonderen Rolle für die Proteinarchitektur in Klammern gesetzt. Gly ist der einzige Rest ohne eine Seitenkette und verleiht der Kette somit Flexibilität. Pro besitzt eine ungewöhnliche Geometrie, die die Kette stark einschränkt. Cys kann eine Disulfidbrücke bilden.

Die oben beschriebenen Aminosäure-Varianten können leicht mit Hilfe von bekannten Peptidsynthesetechniken wie z.B. durch "Solid Phase Synthesis" (Merrifield, 1964) und ähnliche Verfahren oder durch rekombinante DNA-Manipulation hergestellt werden. Techniken, um Substitutionsmutationen an vorbestimmten Stellen in DNA einzubringen, die eine bekannte oder teilweise bekannte Sequenz besitzt, sind gut bekannt und umfassen z.B. M13-Mutagenese. Die Manipulation von DNA-Sequenzen zur Herstellung von Proteinen mit Substitutionen, Insertionen oder Deletionen ist z.B. in Sambrook et. al. (1989) ausführlich beschrieben.

"Derivate" von Proteinen, Polypeptiden oder Peptiden umfassen auch einzelne oder multiple Substitutionen, Deletionen und/oder Additionen jeglicher Moleküle, die mit dem Enzym assoziiert sind, wie Kohlenhydrate, Lipide und/oder Proteine, Polypeptide oder Peptide. Ferner erstreckt sich der Begriff "Derivat" auch auf alle funktionellen chemischen Äquivalente der Proteine, Polypeptide oder Peptide.

Ein Teil oder Fragment eines Tumor-assoziierten Antigens weist offenbarungsgemäß eine funktionelle Eigenschaft des Polypeptids auf, aus dem es abgeleitet ist. Solche funktionellen Eigenschaften umfassen die Interaktion mit Antikörpern, die Interaktion mit anderen Polypeptiden oder Proteinen, die selektive Bindung von Nukleinsäuren und eine enzymatische Aktivität. Eine bedeutende Eigenschaft ist die Fähigkeit, einen Komplex mit HLA einzugehen und gegebenenfalls eine Immunreaktion zu erzeugen. Diese Immunreaktion kann auf Stimulation von cytotoxischen oder Helfer T-Zellen beruhen. Vorzugsweise umfasst ein erfindungsgemäßer Teil oder Fragment eines Tumor-assoziierten Antigens eine Sequenz von mindestens 6, insbesondere mindestens 8, mindestens 10, mindestens 12, mindestens 15, mindestens 20, mindestens 30 oder mindestens 50 aufeinanderfolgenden Aminosäuren aus dem Tumor-assoziierten Antigen.

Ein Teil oder ein Fragment einer Nukleinsäure, die für ein Tumor-assoziiertes Antigen kodiert, betrifft den Teil der Nukleinsäure, der zumindest für das Tumor-assoziierte Antigen kodiert und/oder für einen Teil oder ein Fragment des Tumor-assoziierten Antigens wie vorstehend definiert kodiert.

Die Isolierung und Identifizierung von Genen, die für Tumor-assoziierte Antigene kodieren, ermöglicht auch die Diagnose einer Erkrankung, die sich durch die Expression von einem oder mehreren Tumor-assoziierten Antigenen auszeichnet. Diese Verfahren umfassen die Bestimmung einer oder mehrerer Nukleinsäuren, die für ein Tumor-assoziiertes Antigen kodieren, und/oder die Bestimmung der kodierten Tumor-assoziierten Antigene und/oder von davon abgeleiteten Peptiden. Eine Bestimmung der Nukleinsäure kann in herkömmlicher Weise erfolgen, einschließlich durch Polymerase-Kettenreaktion oder Hybridisierung mit einer markierten Sonde. Eine Bestimmung von Tumor-assoziierten Antigenen oder davon abgeleiteten Peptiden kann durch ein Screening von Patienten-Antiseren in Bezug auf eine Erkennung des Antigens und/oder der Peptide erfolgen. Sie kann auch durch ein Screening von T-Zellen des Patienten auf Spezifität für das entsprechende Tumor-assoziierte Antigen erfolgen.

Die vorliegende Offenbarung ermöglicht auch die Isolierung von Proteinen, die an hier beschriebene Tumor-assoziierte Antigene binden, einschließlich Antikörper und zelluläre Bindepartner der Tumor-assoziierten Antigene.

Erfindungsgemäß werden auch in bestimmten Ausführungsformen "dominant negative" Polypeptide bereitgestellt, die von Tumor-assoziierten Antigenen abgeleitet sind. Ein dominant negatives Polypeptid ist eine inaktive Variante eines Proteins, die durch Interaktion mit der zellulären Maschinerie ein aktives Protein von seiner Interaktion mit der zellulären Maschinerie verdrängt oder mit dem aktiven Protein kompetitiert, wodurch die Wirkung des aktiven Proteins verringert wird. Zum Beispiel kann ein dominant negativer Rezeptor, der einen Liganden bindet, jedoch kein Signal in Reaktion auf die Bindung des Liganden erzeugt, die biologische Wirkung des Liganden verringern. In ähnlicher Weise kann eine dominant negative katalytisch-inaktive Kinase, die normalerweise mit Zielproteinen interagiert, jedoch die Zielproteine nicht phosphoryliert, die Phosphorylierung der Zielproteine in Reaktion auf ein zelluläres Signal verringern. In ähnlicher Weise kann ein dominant negativer Transkriptionsfaktor, der an eine Promotorstelle in der Kontrollregion eines Gens bindet, jedoch die Transkription des Gens nicht erhöht, die Wirkung eines normalen Transkriptionsfaktors durch die Besetzung von Promotorbindestellen ohne eine Erhöhung der Transkription verringern.

Das Ergebnis der Expression eines dominant negativen Polypeptids in einer Zelle ist eine Verringerung der Funktion aktiver Proteine. Der Fachmann kann dominant negative Varianten eines Proteins beispielsweise durch herkömmliche Mutageneseverfahren und Bewerten der dominant negativen Wirkung des Varianten-Polypeptids herstellen.

Erfindungsgemäß umfasst sind auch Stoffe wie Polypeptide, die an Tumor-assoziierte Antigene binden. Solche Bindestoffe können z.B. in Screening-Assays für einen Nachweis von Tumor-assoziierten Antigenen und Komplexen von Tumor-assoziierten Antigenen mit ihren Bindepartnern sowie bei einer Aufreinigung der Tumor-assoziierten Antigene und von Komplexen davon mit ihren Bindepartnern Verwendung finden. Solche Stoffe können auch für eine Hemmung der Aktivität Tumor-assoziierter Antigene beispielsweise durch Bindung an solche Antigene Verwendung finden.

Erfindungsgemäß umfasst sind daher Bindestoffe wie z.B. Antikörper oder Antikörperfragmente, die die Fähigkeit aufweisen, selektiv an Tumor-assoziierte Antigene zu binden. Antikörpern umfassen polyklonale und monoklonale Antikörper, die in herkömmlicher Weise hergestellt werden.

Solche Antikörper können Proteine in nativem und/oder denaturiertem Zustand erkennen (Anderson et al., J. Immunol. 143: 1899-1904, 1989; Gardsvoll, J. Immunol. Methods 234: 107-116, 2000; Kayyem et al., Eur. J. Biochem. 208: 1-8, 1992; Spiller et al., J. Immunol. Methods 224: 51-60, 1999).

Antiseren, die spezifische Antikörper enthalten, die an das Zielprotein spezifisch binden, können über verschiedene Standardverfahren hergestellt werden; vgl. beispielsweise "Monoclonal Antibodies: A Practical Approach" von Philip Shepherd, Christopher Dean ISBN 0-19-963722-9, "Antibodies: A Laboratory Manual" von Ed Harlow, David Lane ISBN: 0879693142 und "Using Antibodies: A Laboratory Manual: Portable Protocol NO" von Edward Harlow, David Lane, Ed Harlow ISBN: 0879695447. Dabei ist auch möglich, affine und spezifische Antikörper zu generieren, die komplexe Membranproteine in ihrer nativen Form erkennen (Azorsa et al., J. Immunol. Methods 229: 35-48, 1999; Anderson et al., J. Immunol. 143: 1899-1904, 1989; Gardsvoll, J. Immunol. Methods. 234: 107-116, 2000). Dies ist vor allem für die Herstellung von Antikörpern von Bedeutung, die therapeutisch eingesetzt werden sollen, aber auch für viele diagnostische Anwendungen. Dazu kann sowohl mit dem gesamten Protein, mit extrazellulären Teilsequenzen, wie auch mit Zellen, die das Zielmolekül in physiologisch gefalteter Form exprimieren, immunisiert werden.

Monoklonale Antikörper werden traditionell mit Hilfe der Hybridoma-Technologie hergestellt (Technische Details: siehe "Monoclonal Antibodies: A Practical Approach" von Philip Shepherd, Christopher Dean ISBN 0-19-963722-9; "Antibodies: A Laboratory Manual" von Ed Harlow, David Lane ISBN: 0879693142, "Using Antibodies: A Laboratory Manual: Portable Protocol NO" von Edward Harlow, David Lane, Ed Harlow ISBN: 0879695447).

Es ist bekannt, dass nur ein kleiner Teil eines Antikörpermoleküls, das Paratop, an der Bindung des Antikörpers an sein Epitop beteiligt ist (vgl. Clark, W.R. (1986), The Experimental Foundations of Modern Immunology, Wiley & Sons, Inc., New York; Roitt, I. (1991), Essential Immunology, 7. Auflage, Blackwell Scientific Publications, Oxford). Die pFc'- und Fc-Regionen sind z.B. Effektoren der Komplementkaskade, sind jedoch nicht an der Antigenbindung beteiligt. Ein Antikörper, von dem die pFc'-Region enzymatisch abgespalten wurde oder der ohne die pFc'-Region hergestellt wurde, bezeichnet als F(ab')₂-Fragment, trägt beide Antigenbindestellen eines vollständigen Antikörpers. In ähnlicher Weise trägt ein Antikörper, von dem die Fc-Region enzymatisch abgespalten wurde oder der ohne die Fc-Region hergestellt wurde, bezeichnet als Fab-Fragment, eine Antigenbindestelle eines intakten Antikörpermoleküls. Des weiteren bestehen Fab-Fragmente aus einer kovalent gebundenen leichten Kette eines Antikörpers und einem Teil der schweren Kette des Antikörpers, bezeichnet als Fd. Die Fd-Fragmente sind die Haupt-Determinanten der Antikörper-Spezifität (ein einzelnes Fd-Fragment kann mit bis zu zehn verschiedenen leichten Ketten assoziiert werden, ohne die Spezifität des Antikörpers zu verändern) und Fd-Fragmente behalten bei einer Isolierung die Fähigkeit, an ein Epitop zu binden.

Innerhalb des Antigen-bindenden Teils eines Antikörpers befinden sich komplementaritätsbestimmende Regionen (CDRs), die direkt mit dem Epitop des Antigens wechselwirken, und Gerüstregionen (FRs), die die Tertiärstruktur des Paratops aufrechterhalten. Sowohl in dem Fd-Fragment der schweren Kette als auch in der leichten Kette von IgG-Immunglobulinen befinden sich vier Gerüstregionen (FR1 bis FR4), die jeweils durch drei komplementaritätsbestimmende Regionen (CDR1 bis CDR3) getrennt sind. Die CDRs und insbesondere die CDR3-Regionen und noch mehr die CDR3-Region der schweren Kette sind größtenteils für die Antikörper-Spezifität verantwortlich.

Man weiß, dass die Nicht-CDR-Regionen eines Säuger-Antikörpers durch ähnliche Regionen von Antikörpern mit der gleichen oder einer anderen Spezifität ersetzt werden können, wobei die Spezifität für das Epitop des ursprünglichen Antikörpers erhalten bleibt. Dies ermöglichte die Entwicklung sogenannter "humanisierter" Antikörper, bei denen nicht-menschliche CDRs kovalent mit menschlichen FR- und/oder Fc/pFc'-Regionen für die Herstellung eines funktionellen Antikörpers verbunden sind.

Dies nutzt die sogenannte "SLAM"-Technologie. Hierbei werden B-Zellen aus Vollblut isoliert und die Zellen monoklonalisiert. Anschließend wird der Überstand der vereinzelten B-Zelle auf ihre Antikörperspezifität hin analysiert. Im Gegensatz zur Hybridomatechnologie wird anschließend die variable Region des Antikörpergens durch eine Einzelzell-PCR amplifiziert und in einen geeigneten Vektor kloniert. Auf diese Art und Weise wird die Gewinnung von monoklonalen Antikörpern beschleunigt (de Wildt et al. J. Immunol. Methods 207:61-67, 1997).

Als anderes Beispiel beschreibt die WO 92/04381 die Herstellung und Verwendung von humanisierten RSV-Antikörpern aus Maus, bei denen mindestens ein Teil der FR-Regionen aus Maus durch FR-Regionen eines menschlichen Ursprungs ersetzt wurden. Solche Antikörper, einschließlich Fragmente intakter Antikörper mit einer Antigen-Bindefähigkeit werden oft als "chimäre" Antikörpern bezeichnet.

Erfindungsgemäß werden auch F(ab')₂-, Fab-, Fv- und Fd-Fragmente von Antikörpern, chimäre Antikörper, bei denen die Fc- und/oder FR- und/oder CDR1- und/oder CDR2- und/oder leichte Kette-CDR3-Regionen durch homologe menschliche oder nicht-menschliche Sequenzen ersetzt wurden, chimäre F(ab')₂-Fragment-Antikörper, bei denen die FR- und/oder CDR1- und/oder CDR2- und/oder leichte Kette-CDR3-Regionen durch homologe menschliche oder nicht-menschliche Sequenzen ersetzt wurden, chimäre Fab-Fragment-Antikörper, bei denen die FR- und/oder CDR1- und/oder CDR2- und/oder leichte Kette-CDR3-Regionen durch homologe menschliche oder nicht-menschliche Sequenzen ersetzt wurden, und chimäre Fd-Fragment-Antikörper, bei denen die FR- und/oder CDR1- und/oder CDR2-Regionen durch homologe menschliche oder nicht-menschliche Sequenzen ersetzt wurden, bereitgestellt. Erfindungsgemäß umfasst sind auch sogenannte einzelkettige Antikörper.

Erfindungsgemäß umfasst sind auch Polypeptide, die spezifisch an Tumor-assoziierte Antigene binden. Beispielsweise können solche Polypeptid-Bindestoffe durch degenerierte Peptid-Bibliotheken bereitgestellt werden, die einfach in Lösung in einer immobilisierten Form oder als Phagen-Display-Bibliotheken hergestellt werden können. Kombinatorische Bibliotheken aus Peptiden mit einer oder mehreren Aminosäuren können ebenfalls hergestellt werden. Ferner können Bibliotheken aus Peptoiden und nicht-peptidischen synthetischen Resten hergestellt werden.

Phagen-Display kann besonders wirksam bei der Identifizierung erfindungsgemäßer Bindepeptide sein. Dabei wird beispielsweise eine Phagen-Bibliothek (durch Verwendung beispielsweise des m13-, fd- oder lambda-Phagen) hergestellt, die Inserts einer Länge von 4 bis etwa 80 Aminosäureresten präsentiert. Es werden sodann Phagen ausgewählt, die Inserts tragen, die an das Tumor-assoziierte Antigen binden. Dieser Prozess kann über mehrere Zyklen einer Rückselektion von Phagen wiederholt werden, die an das Tumor-assoziierte Antigen binden. Wiederholte Runden führen zu einer Anreicherung von Phagen, die bestimmte Sequenzen tragen. Es kann eine Analyse von DNA-Sequenzen erfolgen, um die Sequenzen der exprimierten Polypeptide zu identifizieren. Der kleinste lineare Anteil der Sequenz, der an das Tumor-assoziierte Antigen bindet, kann bestimmt werden. Das "twohybrid-System" aus Hefe kann auch für die Identifizierung von Polypeptiden eingesetzt werden, die an ein Tumor-assoziiertes Antigen binden. Erfindungsgemäß beschriebene Tumor-assoziierte Antigene oder Fragmente davon können für ein Screening von Peptid-Bibliotheken, einschließlich Phagen-Display-Bibliotheken, eingesetzt werden, um Peptid-Bindepartner der Tumor-assoziierten Antigene zu identifizieren und selektieren. Solche Moleküle können beispielsweise für Screening-Assays, Aufreinigungsprotokolle, für eine Interferenz mit der Funktion des Tumor-assoziierten Antigens und für andere Zwecke, die dem Fachmann bekannt sind, verwendet werden.

Die vorstehend beschriebenen Antikörper und andere Bindemoleküle können beispielsweise für die Identifizierung von Gewebe verwendet werden, das ein Tumor-assoziiertes Antigen exprimiert. Antikörper können auch an spezifische diagnostische Stoffe für eine Darstellung von Zellen und Geweben gekoppelt werden, die Tumor-assoziierte Antigene exprimieren. Sie können ferner an therapeutisch nützliche Stoffe gekoppelt werden. Diagnostische Stoffe umfassen in nicht begrenzender Weise Bariumsulfat, Iocetaminsäure, Iopansäure, Calcium-Ipodat, Natrium-Diatrizoat, Meglumin-Diatrizoat, Metrizamid, Natrium-Tyropanoat und Radiodiagnostika, einschließlich Positronen-Emitter wie Fluor-18 und Kohlenstoff-11, gamma-Emitter wie Iod-123, Technetium-99m, Iod-131 und Indium-111, Nuklide für magnetische Kernresonanz wie Fluorin und Gadolinium. Der Begriff "therapeutisch nützlicher Stoff" meint erfindungsgemäß jedes therapeutische Molekül, das wunschgemäß selektiv zu einer Zelle geführt wird, die ein oder mehrere Tumor-assoziierte Antigene exprimiert, einschließlich Antikrebsmittel, mit radioaktivem Iod versehene Verbindungen, Toxine, cytostatische oder cytolytische Arzneistoffe, usw. Antikrebsmittel umfassen beispielsweise Aminoglutethimid, Azathioprin, Bleomycinsulfat, Busulfan, Carmustin, Chlorambucil, Cisplatin, Cyclophosphamid, Cyclosporin, Cytarabidin, Dacarbazin, Dactinomycin, Daunorubin, Doxorubicin, Taxol, Etoposid, Fluoruracil, Interferon-α, Lomustin, Mercaptopurin, Methotrexat, Mitotan, Procarbazin-HCl, Thioguanin, Vinblastinsulfat und Vincristinsulfat. Weitere Antikrebsmittel sind beispielsweise in Goodman und Gilman, "The Pharmacological Basis of Therapeutics", 8. Auflage, 1990, McGraw-Hill, Inc., insbesondere Kapitel 52 (Antineoplastic Agents (Paul Calabresi und Bruce A. Chabner)) beschrieben. Toxine können Proteine wie Pokeweed-antivirales Protein, Choleratoxin, Pertussistoxin, Ricin, Gelonin, Abrin, Diphtherie-Exotoxin oder *Pseudomonas-*Exotoxin sein. Toxinreste können auch Hochenergie-emittierende Radionuklide wie Kobalt-60 sein.

Der Begriff "Patient" bedeutet erfindungsgemäß Mensch, nicht menschlicher Primat oder ein anderes Tier, insbesondere Säugetier wie Kuh, Pferd, Schwein, Schaf, Ziege, Hund, Katze oder Nagetier wie Maus und Ratte. In einer besonders bevorzugten Ausführungsform ist der Patient ein Mensch.

Der Begriff "Erkrankung" betrifft erfindungsgemäß jeden pathologischen Zustand, bei dem Tumor-assoziierte Antigene exprimiert oder abnormal exprimiert werden. "Abnormale Expression" bedeutet erfindungsgemäß, dass die Expression gegenüber dem Zustand bei einem gesunden Individuum verändert, vorzugsweise erhöht ist. Eine Erhöhung der Expression betrifft eine Erhöhung um mindestens 10%, insbesondere mindestens 20%, mindestens 50% oder mindestens 100%. In einer Ausführungsform wird das Tumor-assoziierte Antigen nur in Gewebe eines erkrankten Individuums exprimiert, während die Expression bei einem gesunden Individuum reprimiert ist. Ein Beispiel einer solchen Erkrankung ist Krebs, wobei der Begriff "Krebs" erfindungsgemäß Leukämien, Seminome, Melanome, Teratome, Gliome, Nieren-, Nebennieren-, Schilddrüsen-, Darm-, Leber-, Colon-, Magen-, Gastrointestinal-, Lymphknoten-, Speiseröhren-, Kolorektal-, Pankreas-, Hals, Nasen, Ohren (HNO)-, Brust-, Prostata-, Gebärmutter-, Ovarial-, und Lungenkrebs und deren Metastasen umfasst.

Eine biologische Probe kann erfindungsgemäß eine Gewebe- und/oder zelluläre Probe sein und kann für eine Verwendung in den verschiedenen, hier beschriebenen Verfahren in herkömmlicher Weise gewonnen werden, wie durch Gewebebiopsie, einschließlich Stanzbiopsie, und Entnahme von Blut, Bronchialaspirat, Sputum, Urin, Fäces oder anderen Körperflüssigkeiten.

Der Begriff "immunreaktive Zelle" bedeutet erfindungsgemäß eine Zelle, die in eine Immunzelle (wie B-Zelle, T-Helferzelle oder cytolytische T-Zelle) bei geeigneter Stimulierung reifen kann. Immunreaktive Zellen umfassen CD34⁺ hämatopoietische Stammzellen, unreife und reife T-Zellen sowie unreife und reife B-Zellen. Falls die Herstellung cytolytischer oder Helfer T-Zellen, die ein Tumor-assoziiertes Antigen erkennen, gewünscht ist, wird die immunreaktive Zelle mit einer Zelle, die ein Tumor-assoziiertes Antigen exprimiert, unter Bedingungen in Kontakt gebracht, die eine Produktion, Differenzierung und/oder Selektion von cytolytischen sowie Helfer T-Zellen begünstigen. Die Differenzierung von T-Zell-Vorläufern in eine cytolytische T-Zelle bei einer Exposition gegenüber einem Antigen ist ähnlich zur klonalen Selektion des Immunsystems.

Manche therapeutische Verfahren beruhen auf einer Reaktion des Immunsystems eines Patienten, die zu einer Lyse Antigen-präsentierender Zellen führt, wie Krebszellen, die ein oder mehrere Tumor-assoziierte Antigene präsentieren. Dabei werden beispielsweise autologe cytotoxische T-Lymphozyten, die für einen Komplex aus einem Tumor-assoziierten Antigen und einem MHC-Molekül spezifisch sind, an einen Patienten mit einer Zellabnormalie verabreicht. Die Produktion solcher cytotoxischer T-Lymphozyten *in vitro* ist bekannt. Ein Beispiel für ein Verfahren zur Differenzierung von T-Zellen findet sich in der WO-A-9633265. Im Allgemeinen wird eine Probe mit Zellen wie Blutzellen aus dem Patienten entnommen und die Zellen werden mit einer Zelle in Kontakt gebracht, die den Komplex präsentiert und eine Vermehrung von cytotoxischen T-Lymphozyten auslösen kann (z.B. dendritische Zellen). Die Zielzelle kann eine transfizierte Zelle wie eine COS-Zelle sein. Diese transfizierten Zellen präsentieren, den gewünschten Komplex auf ihrer Oberfläche und stimulieren bei einer Kontaktierung mit cytotoxischen T-Lymphozyten deren Vermehrung. Die klonal expandierten autologen cytotoxischen T-Lymphozyten werden sodann an den Patienten verabreicht.

Bei einem anderen Verfahren zur Selektion Antigen-spezifischer cytotoxischer T-Lymphozyten werden fluorogene Tetramere von MHC-Klasse I-Molekül/Peptid-Komplexen für einen Nachweis spezifischer Klone von cytotoxischen T-Lymphozyten verwendet (Altman et al., Science 274:94-96, 1996; Dunbar et al., Curr. Biol. 8:413-416, 1998). Lösliche MHC-Klasse I-Moleküle werden *in vitro* in Gegenwart von β₂-Mikroglobulin und eines Peptid-Antigens, das an das Klasse I-Molekül bindet, gefaltet. Nach Aufreinigung der MHC/Peptid-Komplexe werden diese mit Biotin markiert. Tetramere werden durch Mischen der biotinylierten Peptid-MHC-Komplexe mit markiertem Avidin (z.B. Phycoerythrin) bei einem molaren Verhältnis von 4:1 gebildet. Tetramere werden sodann mit cytotoxischen T-Lymphozyten wie peripherem Blut oder Lymphknoten in Kontakt gebracht. Die Tetramere binden an cytotoxische T-Lymphozyten, die den Peptid-Antigen/MHC-Klasse I-Komplex erkennen. Zellen, die an die Tetramere gebunden werden, können durch Fluoreszenz-gesteuerte Zellsortierung für eine Isolierung reaktiver cytotoxischer T-Lymphozyten sortiert werden. Die isolierten cytotoxischen T-Lymphozyten können sodann *in vitro* vermehrt werden.

Bei einem therapeutischen Verfahren, das als adoptiver Transfer bezeichnet wird (Greenberg, J. Immunol. 136(5):1917, 1986; Riddel et al., Science 257:238, 1992; Lynch et al., Eur. J. Immunol. 21:1403-1410, 1991; Kast et al., Cell 59:603-614, 1989), werden Zellen, die den gewünschten Komplex präsentieren (z.B. dendritische Zellen) mit cytotoxischen T-Lymphozyten des zu behandelnden Patienten kombiniert, was zu einer Vermehrung spezifischer cytotoxischer T-Lymphozyten führt. Die vermehrten cytotoxischen T-Lymphozyten werden sodann an einen Patienten mit einer zellulären Abnormalie verabreicht, die sich durch bestimmte abnormale Zellen auszeichnet, die den spezifischen Komplex präsentieren. Die cytotoxischen T-Lymphozyten lysieren sodann die abnormalen Zellen, wodurch eine gewünschte therapeutische Wirkung erreicht wird.

Oft lassen sich aus dem T-Zell-Repertoire eines Patienten lediglich niedrig-affine T-Zellen gegen einen solchen spezifischen Komplex vermehren, da die hochaffinen durch Toleranzentwicklung ausgelöscht worden sind. Eine Alternative kann hier ein Transfer des T-Zell-Rezeptors selbst sein. Hierfür werden ebenfalls Zellen, die den gewünschten Komplex präsentieren (z.B. dendritische Zellen) mit cytotoxischen T-Lymphozyten von gesunden Personen oder von einer anderen Spezies (z.B. Maus) kombiniert. Dies führt zu einer Vermehrung hochaffiner spezifischer cytotoxischer T-Lymphozyten, wenn die T-Lymphozyten aus einem Spenderorganismus kommen, der mit dem spezifischen Komplex bisher keinen Kontakt hatte. Der hochaffine T-Zell-Rezeptor aus diesen vermehrten spezifischen T-Lymphozyten wird kloniert. Wurden die hochaffinen T-Zellrezeptoren aus einer anderen Spezies kloniert, können diese in unterschiedlichem Ausmaß humanisiert werden. Durch Gentransfer z.B. mit retroviralen Vektoren werden solche T-ZellRezeptoren dann beliebig in T-Zellen von Patienten transduziert. Adoptiver Transfer erfolgt dann mit diesen genetisch veränderten T-Lymphozyten (Stanislawski et al., Nat Immunol. 2:962-70, 2001 ; Kessels et al., Nat Immunol. 2:957-61, 2001).

Die vorstehenden therapeutischen Aspekte gehen davon aus, dass zumindest manche der abnormalen Zellen des Patienten einen Komplex aus einem Tumor-assoziierten Antigen und einem HLA-Molekül präsentieren. Eine Identifizierung solcher Zellen kann in an sich bekannter Weise erfolgen. Sobald Zellen, die den Komplex präsentieren, identifiziert wurden, können sie mit einer Probe aus dem Patienten, die cytotoxische T-Lymphozyten enthält, kombiniert werden. Falls die Zellen, die den Komplex präsentieren, durch die cytotoxischen T-Lymphozyten lysiert werden, kann angenommen werden, dass ein Tumor-assoziiertes Antigen präsentiert wird.

Der adoptive Transfer ist nicht die einzige Therapieform, die erfindungsgemäß anwendbar ist. Cytotoxische T-Lymphozyten können auch *in vivo* in an sich bekannter Weise erzeugt werden. Bei einem Verfahren werden nicht-proliferative Zellen verwendet, die den Komplex exprimieren. Die Zellen, die dabei verwendet werden, werden diejenigen sein, die normalerweise den Komplex exprimieren, wie bestrahlte Tumorzellen oder Zellen, die mit einem oder beiden Genen transfizierte wurden, die für eine Präsentation des Komplexes notwendig sind (d.h. das antigene Peptid und das präsentierende HLA-Molekül). Verschiedene Zelltypen können eingesetzt werden. Des weiteren können Vektoren verwendet werden, die eines oder beide der interessierenden Gene tragen. Virale oder bakterielle Vektoren sind besonders bevorzugt. Zum Beispiel können Nukleinsäuren, die für ein Tumor-assoziiertes Antigen oder einen Teil davon kodieren, funktionell mit Promotor- und Enhancersequenzen verknüpft werden, die eine Expression des Tumor-assoziierten Antigens oder eines Fragments davon in bestimmten Geweben oder Zelltypen steuern. Die Nukleinsäure kann in einen Expressionsvektor eingebaut werden. Expressionsvektoren können nicht-modifizierte extrachromosomale Nukleinsäuren, Plasmide oder virale Genome sein, in die eine Insertion exogener Nukleinsäuren möglich ist. Nukleinsäuren, die für ein Tumor-assoziiertes Antigen kodieren, können auch in ein retrovirales Genom inseriert werden, wodurch die Integration der Nukleinsäure in das Genom des Zielgewebes oder der Zielzelle ermöglicht wird. Bei diesen Systemen trägt ein Mikroorganismus wie Vacciniavirus, Poxvirus, Herpes simplex-Virus, Retrovirus oder Adenovirus das interessierende Gen und "infiziert" de facto Wirtszellen. Eine weitere bevorzugte Form ist die Einbringung des Tumor-assoziierten Antigenes in Form von rekombinanter RNA. Diese kann z.B. durch liposomalen Transfer oder durch Elektroporation in Zellen eingebracht werden. Die resultierenden Zellen präsentieren den interessierenden Komplex und werden von autologen cytotoxischen T-Lymphozyten erkannt, die sich sodann vermehren.

Eine ähnliche Wirkung kann durch Kombination des Tumor-assoziierten Antigens oder eines Fragments davon mit einem Adjuvans erreicht werden, um einen Einbau in Antigen-präsentierende Zellen *in vivo* zu ermöglichen. Das Tumor-assoziierte Antigen oder ein Fragment davon können als Protein, als DNA (z.B. innerhalb eines Vektors) oder als RNA repräsentiert sein. Das Tumor-assoziierte Antigen wird prozessiert, um einen Peptidpartner für das HLA-Molekül zu ergeben, während ein Fragment davon präsentiert werden kann, ohne dass eine weitere Prozessierung erforderlich ist. Letzteres ist insbesondere der Fall, wenn diese an HLA-Moleküle binden können. Verabreichungsformen, bei denen das Gesamt-Antigen *in vivo* von einer dendritischen Zelle prozessiert wird, sind bevorzugt, da hier auch Helfer T-Zell-Antworten entstehen können. Eine effektive Immunantwort benötigt diese (Ossendorp et al., Immunol Lett. 74:75-9, 2000; Ossendorp et al., J. Exp. Med. 187:693-702, 1998). Im Allgemeinen kann eine wirksame Menge des Tumor-assoziierten Antigens an einen Patienten z.B. durch eine intradermale Injektion verabreicht werden. Die Injektion kann aber auch intranodal in einen Lymphknoten erfolgen (Maloy et al., Proc Natl Acad Sci USA 98:3299-303, 2001). Sie kann auch in Kombination mit Reagenzien erfolgen, die eine Aufnahme in dendritische Zellen erleichtern. Bevorzugte Tumor-assoziierte Antigene umfassen diejenigen, die mit allogenen Krebs-Antiseren oder mit T-Zellen vieler Krebs-Patienten reagieren. Von besonderem Interesse sind aber auch solche, gegen die keine spontanen Immunantworten vorbestehen. Gegen diese können nachweislich Immunantworten induziert werden, die Tumoren lysieren können (Keogh et al., J Immunol. 167:787-96, 2001; Appella et al., Biomed Pept Proteins Nucleic Acids 1:177-84, 1995; Wentworth et al., Mol Immunol. 32:603-12, 1995).

Die offenbarungsgemäß beschriebenen pharmazeutischen Zusammensetzungen können auch als Vakzine für die Immunisierung eingesetzt werden. Die Begriffe "Immunisierung" oder "Vakzinierung" bedeuten erfindungsgemäß eine Erhöhung oder Aktivierung einer Immunreaktion gegenüber einem Antigen. Tiermodelle können zum Testen einer immunisierenden Wirkung gegenüber Krebs durch Verwendung eines Tumor-assoziierten Antigens oder einer dafür kodierenden Nukleinsäure eingesetzt werden. Zum Beispiel können menschliche Krebszellen in eine Maus für die Schaffung eines Tumors eingebracht werden und eine oder mehrere Nukleinsäuren, die für Tumor-assoziierte Antigene kodieren, können verabreicht werden. Die Wirkung auf die Krebszellen (beispielsweise Verringerung der Tumorgröße) kann als Maß für die Wirksamkeit einer Immunisierung durch die Nukleinsäure gemessen werden.

Als Teil der Zusammensetzung für eine Immunisierung werden eines oder mehrere Tumor-assoziierte Antigene oder stimulierende Fragmente davon mit einem oder mehreren Adjuvanzien für eine Induktion einer Immunreaktion oder eine Erhöhung einer Immunreaktion verabreicht. Ein Adjuvans ist eine Substanz, die in das Antigen eingebaut oder gemeinsam mit diesem verabreicht wird und die Immunreaktion verstärkt. Adjuvanzien können die Immunreaktion durch Bereitstellen eines Antigen-Reservoirs (extrazellulär oder in Makrophagen), Aktivierung von Makrophagen und/oder Stimulierung bestimmter Lymphozyten verstärken. Adjuvanzien sind bekannt und umfassen in nicht begrenzender Weise Monophosphoryl-Lipid-A (MPL, SmithKline Beecham), Saponine wie QS21 (SmithKline Beecham), DQS21 (SmithKline Beecham; WO 96/33739), QS7, QS17, QS18 und QS-L1 (So et al., Mol. Cells 7:178-186, 1997), unvollständiges Freundsches Adjuvans, vollständiges Feundsches Adjuvans, Vitamin E, Montanid, Alaun, CpG-Oligonukleotide (vgl. Kreig et al., Nature 374:546-9, 1995) und verschiedene Wasser-in-Öl-Emulsionen, die aus biologisch abbaubaren Ölen wie Squalen und/oder Tocopherol hergestellt werden. Vorzugsweise werden die Peptide in einer Mischung mit DQS21/MPL verabreicht. Das Verhältnis von DQS21 zu MPL beträgt typischerweise etwa 1:10 bis 10:1, vorzugsweise etwa 1:5 bis 5:1 und insbesondere etwa 1:1. Für eine Verabreichung an den Menschen sind DQS21 und MPL typischerweise in einer Vakzine-Formulierung in einem Bereich von etwa 1 *µ*g bis etwa 100 *µ*g vorhanden.

Andere Stoffe, die eine Immunreaktion des Patienten stimulieren, können auch verabreicht werden. Zum Beispiel sind Cytokine bei einer Vakzinierung aufgrund ihrer regulatorischen Eigenschaften auf Lymphozyten verwendbar. Solche Cytokine umfassen z.B. Interleukin-12 (IL-12), von dem gezeigt wurde, dass es die schützenden Wirkungen von Vakzinen verstärkt (vgl. Science 268:1432-1434, 1995), GM-CSF und IL-18.

Es gibt eine Reihe von Verbindungen, die eine Immunreaktion verstärken und die daher bei einer Vakzinierung eingesetzt werden können. Diese umfassen co-stimulierende Moleküle, die in Form von Proteinen oder Nukleinsäuren bereitgestellt werden. Solche co-stimulierenden Moleküle sind beispielsweise B7-1 und B7-2 (CD80 bzw. CD86), die auf dendritischen Zellen (DC) exprimiert werden und mit dem auf den T-Zellen exprimierten CD28-Molekül interagieren. Diese Interaktion stellt eine Co-Stimulierung (Signal 2) für eine Antigen/MHC/TCR-stimulierte (Signal 1) T-Zelle bereit, wodurch die Vermehrung der T-Zelle und die Effektorfunktion verstärkt wird. B7 interagiert auch mit CTLA4 (CD 152) auf T-Zellen und Untersuchungen, die CTLA4- und B7-Liganden einbeziehen, zeigen, dass die B7-CTLA4-Interaktion eine Antitumor-Immunität und CTL-Vermehrung verstärken kann (Zheng, P. et al., Proc. Natl. Acad. Sci. USA 95(11):6284-6289 (1998)).

B7 wird typischerweise nicht auf Tumorzellen exprimiert, so dass diese keine wirksamen Antigen-präsentierenden Zellen (APCs) für T-Zellen sind. Eine Induktion der B7-Expression würde ermöglichen, dass Tumorzellen wirksamer eine Vermehrung von cytotoxischen T-Lymphozyten und eine Effektorfunktion stimulieren. Eine Co-Stimulierung durch eine Kombination von B7/IL-6/IL-12 zeigte eine Induktion des IFN-gamma- und Th1-Cytokin-Profils in einer T-Zell-Population, was zu einer weiter verstärkten T-Zell-Aktivität führt (Gajewski et al., J. Immunol. 154:5637-5648 (1995)).

Eine vollständige Aktivierung von cytotoxischen T-Lymphozyten und eine vollständige Effektorfunktion erfordert eine Mitwirkung von T-Helferzellen durch die Interaktion zwischen dem CD40-Liganden auf den T-Helferzellen und dem CD40-Molekül, das von dendritischen Zellen exprimiert wird (Ridge et al., Nature 393:474 (1998), Bennett et al., Nature 393:478 (1998), Schönberger et al., Nature 393:480 (1998)). Der Mechanismus dieses co-stimulierenden Signals betrifft wahrscheinlich die Steigerung der B7- und assoziierten IL-6/IL-12-Produktion durch die dendritischen Zellen (Antigen-präsentierenden Zellen). Die CD40-CD40L-Interaktion komplementiert so die Interaktionen des Signals 1 (Antigen/MHC-TCR) und des Signals 2 (B7-CD28).

Die Verwendung von anti-CD40-Antikörpern für eine Stimulierung von dendritischen Zellen würde erwartungsgemäß direkt eine Reaktion gegenüber Tumor-Antigenen verstärken, die normalerweise außerhalb des Bereichs einer entzündlichen Reaktion liegen oder von nicht-professionellen Antigen-präsentierenden Zellen (Tumorzellen) präsentiert werden. In diesen Situationen werden T-Helfer- und B7-co-stimulierende Signale nicht bereitgestellt. Dieser Mechanismus könnte im Zusammenhang mit Therapien verwendet werden, die auf Antigen-gepulsten dendritischen Zellen basieren.

Offenbarungsgemäß vorgesehen ist auch eine Verabreichung von Nukleinsäuren, Polypeptiden oder Peptiden. Eine Verabreichung von Polypeptiden und Peptiden kann in an sich bekannter Weise erfolgen. In einer Ausführungsform erfolgt die Verabreichung von Nukleinsäuren durch *ex vivo*-Verfahren, d.h. durch Entfernung von Zellen aus einem Patienten, genetische Veränderung der Zellen, um ein Tumor-assoziiertes Antigen einzubauen, und Wiedereinbringung der veränderten Zellen in den Patienten. Dies umfasst im Allgemeinen das Einbringen einer funktionellen Kopie eines Gens in die Zellen eines Patienten *in vitro* und die Rückführung der genetisch veränderten Zellen in den Patienten. Die funktionelle Kopie des Gens steht unter funktioneller Kontrolle von regulatorischen Elementen, die eine Expression des Gens in den genetisch veränderten Zellen erlauben. Transfektions- und Transduktionsverfahren sind dem Fachmann bekannt.

Offenbarungsgemäß vorgesehen ist auch eine Verabreichung von Nukleinsäuren *in vivo* durch die Verwendung von Vektoren wie Viren und zielgesteuerten Liposomen.

In einer bevorzugten Ausführungsform ist ein viraler Vektor für die Verabreichung einer Nukleinsäure, die für ein Tumor-assoziiertes Antigen kodiert, aus der Gruppe ausgewählt bestehend aus Adenoviren, Adeno-assoziierten Viren, Poxviren, einschließlich Vacciniavirus und attenuierten Poxviren, Semliki-Forest-Virus, Retroviren, Sindbis-Virus und Ty-Virus-ähnlichen Partikeln. Besonders bevorzugt sind Adenoviren und Retroviren. Die Retroviren sind üblicherweise replikationsdefizient (d.h. sie sind unfähig, infektiöse Partikel zu erzeugen).

Verschiedene Verfahren können eingesetzt werden, um Nukleinsäuren in Zellen *in vitro* oder *in vivo* einzubringen. Solche Verfahren umfassen die Transfektion von Nukleinsäure-CaPO₄-Präzipitaten, die Transfektion von Nukleinsäuren, die mit DEAE assoziiert sind, die Transfektion oder Infektion mit den vorstehenden Viren, die die interessierenden Nukleinsäuren tragen, die Liposomen-vermittelte Transfektion und ähnliches. In bestimmten Ausführungsformen ist eine Steuerung der Nukleinsäure an bestimmte Zellen bevorzugt. In solchen Ausführungsformen kann ein Träger, der für die Verabreichung einer Nukleinsäure an eine Zelle (z.B. ein Retrovirus oder ein Liposom) eingesetzt wird, ein gebundenes Zielsteuerungsmolekül aufweisen. Zum Beispiel kann ein Molekül wie ein Antikörper, der für ein Oberflächenmembran-Protein auf der Zielzelle spezifisch ist, oder ein Ligand für einen Rezeptor auf der Zielzelle in den Nukleinsäureträger eingebaut oder daran gebunden werden. Bevorzugte Antikörper umfassen Antikörper, die selektiv ein Tumor-assoziiertes Antigen binden. Falls eine Verabreichung einer Nukleinsäure durch Liposomen erwünscht ist, können Proteine, die an ein Oberflächenmembran-Protein binden, das mit der Endozytose assoziiert ist, in die Liposomenformulierung eingebaut werden, um eine Zielsteuerung und/oder Aufnahme zu ermöglichen. Solche Proteine umfassen Kapsid-Proteine oder Fragmente davon, die für einen bestimmten Zelltyp spezifisch sind, Antikörper gegen Proteine, die internalisiert werden, Proteine, die eine intrazelluläre Stelle ansteuern, und ähnliches.

Die erfindungsgemäßen therapeutischen Zusammensetzungen können in pharmazeutisch verträglichen Zubereitungen verabreicht werden. Solche Zubereitungen können gewöhnlich pharmazeutisch verträgliche Konzentrationen von Salzen, Pufferstoffen, Konservierungsstoffen, Trägen, ergänzenden immunitätssteigernden Stoffen wie Adjuvanzien, CpG und Cytokinen und gegebenenfalls andere therapeutische Wirkstoffe enthalten.

Die erfindungsgemäßen therapeutischen Wirkstoffe können auf jedem herkömmlichen Weg verabreicht werden, einschließlich durch Injektion oder durch Infusion. Die Verabreichung kann beispielsweise oral, intravenös, intraperitoneal, intramuskulär, subkutan oder transdermal erfolgen. Eine therapeutische Verabreichung von Antikörpern erfolgt vorzugsweise durch ein Lungenaerosol. Die Verabreichung von Antisense-Nukleinsäuren erfolgt vorzugsweise durch langsame intravenöse Verabreichung.

Die Zusammensetzungen werden in wirksamen Mengen verabreicht. Eine "wirksame Menge" betrifft die Menge, die alleine oder zusammen mit weiteren Dosen eine gewünschte Reaktion oder eine gewünschte Wirkung erzielt. Im Fall einer Behandlung einer bestimmten Erkrankung oder eines bestimmten Zustands, der sich durch die Expression eines oder mehrerer Tumor-assoziierter Antigene auszeichnet, betrifft die gewünschte Reaktion die Hemmung des Krankheitsverlaufs. Dies umfasst die Verlangsamung des Fortschreitens der Erkrankung und insbesondere eine Unterbrechung des Fortschreitens der Erkrankung. Die gewünschte Reaktion bei einer Behandlung einer Krankheit oder eines Zustands kann auch die Verzögerung des Ausbruchs oder eine Verhinderung des Ausbruchs der Krankheit oder des Zustands sein.

Eine wirksame Menge einer Zusammensetzung wird von dem zu behandelnden Zustand, der Schwere der Krankheit, den individuellen Parametern des Patienten, einschließlich Alter, physiologischer Zustand, Größe und Gewicht, der Dauer der Behandlung, der Art einer begleitenden Therapie (falls vorhanden), dem spezifischen Verabreichungsweg und ähnlichen Faktoren abhängen.

Die pharmazeutischen Zusammensetzungen sind vorzugsweise steril und enthalten eine wirksame Menge der therapeutisch wirksamen Substanz für die Erzeugung der gewünschten Reaktion oder der gewünschten Wirkung.

Die Dosen der Zusammensetzungen, die verabreicht werden, können von verschiedenen Parametern wie der Verabreichungsart, dem Zustand des Patienten, dem gewünschten Verabreichungszeitraum, usw. abhängen. Für den Fall, dass eine Reaktion bei einem Patienten bei einer anfänglichen Dosis unzureichend ist, können höhere Dosen (oder effektiv höhere Dosen, die durch einen anderen, stärker lokalisierten Verabreichungsweg erzielt werden) eingesetzt werden.

Im Allgemeinen werden für eine Behandlung oder für eine Erzeugung oder Erhöhung einer Immunreaktion Dosen des Tumor-assoziierten Antigens von 1 ng bis 1 mg, vorzugsweise von 10 ng bis 100 *µ*g formuliert und verabreicht. Falls die Verabreichung von Nukleinsäure (DNA sowie RNA), die für Tumor-assoziierte Antigene kodieren, erwünscht ist, werden Dosen von 1 ng bis 0,1 mg formuliert und verabreicht.

Die pharmazeutischen Zusammensetzungen werden im Allgemeinen in pharmazeutisch verträglichen Mengen und in pharmazeutisch verträglichen Zusammensetzungen verabreicht. Der Begriff "pharmazeutisch verträglich" betrifft ein nicht-toxisches Material, das nicht mit der Wirkung des aktiven Bestandteils der pharmazeutischen Zusammensetzung wechselwirkt. Solche Zubereitungen können gewöhnlich Salze, Pufferstoffe, Konservierungsstoffe, Träger und gegebenenfalls andere therapeutische Wirkstoffe enthalten. Bei einer Verwendung in der Medizin sollten die Salze pharmazeutisch verträglich sein. Nicht-pharmazeutisch verträgliche Salze können jedoch für die Herstellung pharmazeutisch verträglicher Salze davon verwendet werden und sind offenbarungsgemäß umfasst. Solche pharmakologisch und pharmazeutisch verträglichen Salze umfassen in nicht begrenzender Weise diejenigen, die aus den nachstehenden Säuren hergestellt werden: Chlorwasserstoff-, Bromwasserstoff-, Schwefel-, Salpeter-, Phosphor-, Malein-, Essig-, Salicyl-, Citronen-, Ameisen-, Malon-, Bernsteinsäure und ähnliches. Pharmazeutisch verträgliche Salze können auch als Alkalimetall- oder Erdalkalimetallsalze wie Natrium-, Kalium- oder Calciumsalze hergestellt werden.

Eine pharmazeutische Zusammensetzung kann einen pharmazeutisch verträglichen Träger umfassen. Der Begriff "pharmazeutisch verträglicher Träger" betrifft einen oder mehrere kompatible feste oder flüssige Füllstoffe, Verdünnungsmittel oder Kapselsubstanzen, die für eine Verabreichung an einen Menschen geeignet sind. Der Begriff "Träger" betrifft einen organischen oder anorganischen Bestandteil, natürlicher oder synthetischer Natur, in dem der aktive Bestandteil kombiniert wird, um eine Anwendung zu erleichtern. Die Bestandteile der pharmazeutischen Zusammensetzung sind gewöhnlich derart, dass keine Interaktion auftritt, die die gewünschte pharmazeutische Wirksamkeit wesentlich beeinträchtigt.

Die pharmazeutischen Zusammensetzungen können geeignete Pufferstoffe wie Essigsäure in einem Salz, Citronensäure in einem Salz, Borsäure in einem Salz und Phosphorsäure in einem Salz enthalten.

Die pharmazeutischen Zusammensetzungen können auch gegebenenfalls geeignete Konservierungsstoffe wie Benzalkoniumchlorid, Chlorbutanol, Parabene und Thimerosal enthalten.

Die pharmazeutischen Zusammensetzungen werden gewöhnlich in einer einheitlichen Dosisform dargeboten und können in an sich bekannter Weise hergestellt werden. Pharmazeutische Zusammensetzungen können beispielsweise in Form von Kapseln, Tabletten, Lutschpastillen, Suspensionen, Sirupen, Elixieren oder als Emulsion vorliegen.

Zusammensetzungen, die für eine parenterale Verabreichung geeignet sind, umfassen gewöhnlich eine sterile wässrige oder nicht-wässrige Zubereitung des Wirkstoffs, die vorzugsweise mit dem Blut des Empfängers isotonisch ist. Verträgliche Träger und Lösungsmittel sind beispielsweise Ringer-Lösung und isotonische Natriumchloridlösung. Zusätzlich werden gewöhnlich sterile, fixierte Öle als Lösungs- oder Suspensionsmedium eingesetzt.

Die vorliegende Erfindung wird durch die nachstehenden Abbildungen und Beispiele ausführlich beschrieben, die der Erläuterung dienen. Dem Fachmann sind aufgrund der Beschreibung und der Beispiele weitere Ausführungsformen zugänglich.

### Abbildungen:

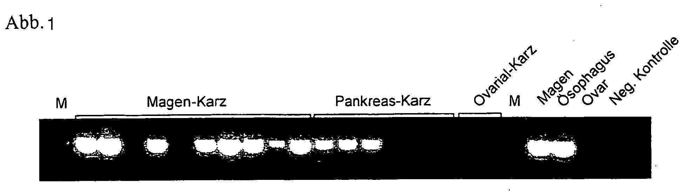

### Abb. 1. Claudin-18A2.1-Expression im Magen, Ösophagus, Magen- und Pankreaskarzinom

RT-PCR-Analyse mit Claudin-18A2.1-spezifischen Primern (SEQ ID NO: 39, 40) zeigte erfindungsgemäß in 8/10 Magenkarzinom-Biopsien, sowie in 3/6 Pankreaskarzinom-Biopsien eine ausgeprägte Claudin-18A2.1-Expression. In Magen und Ösophagus-Normalgewebe wurde ebenfalls eine deutliche Expression nachgewiesen. Im Gegensatz dazu wurde im Ovar und im Ovarkarzinom keine Expression detektiert.

### Abb. 2. Schematische Darstellung von Claudin-18-Spleissvarianten

Die beiden Claudin-18 Spleißvarianten A1 und A2 unterscheiden sich im N-Terminus und zeigen unterschiedliche potentielle Glykosylierungsstellen.

### Abb. 3. Quantitative Expression von Claudin-18, Variante A1

Claudin-A1 ist in einer Vielzahl von Tumorgeweben stark aktiviert. Eine besonders starke Expression findet sich in Magentumoren, Lungentumoren, Pankreaskarzinomen und Speiseröhrenkarzinomen.

### Abb. 4. Quantitative Expression von Claudin-18, Variante A2

Wie die Variante A1 ist die Variante A2 in vielen Tumoren aktiviert.

### Abb. 5. Verwendung Claudin-18A2-spezifischer Antikörper (extrazelluläre Domäne)

(oben) Färbung von Claudin-18A2-positiven Magenkarzinomzellen (SNU-16) mit einem Antikörper, der durch Immunisierung mit einem Peptid (SEQ ID NO: 17) hergestellt wurde. Membranfärbung tritt besonders stark in den Zell/Zell-Interaktionsbereichen auf. A-Präimmun., MeOH; B-Immunserum MeOH, 5 µg/ml (unten) Nachweis der Spezifität des Antikörpes durch Kolokalisationsanalyse in Claudin-18A2-GFP-transfizierten 293T-Zellen. A-Claudin-18A2 GFP; B-anti-Claudin-A2; C-Überlagerung.

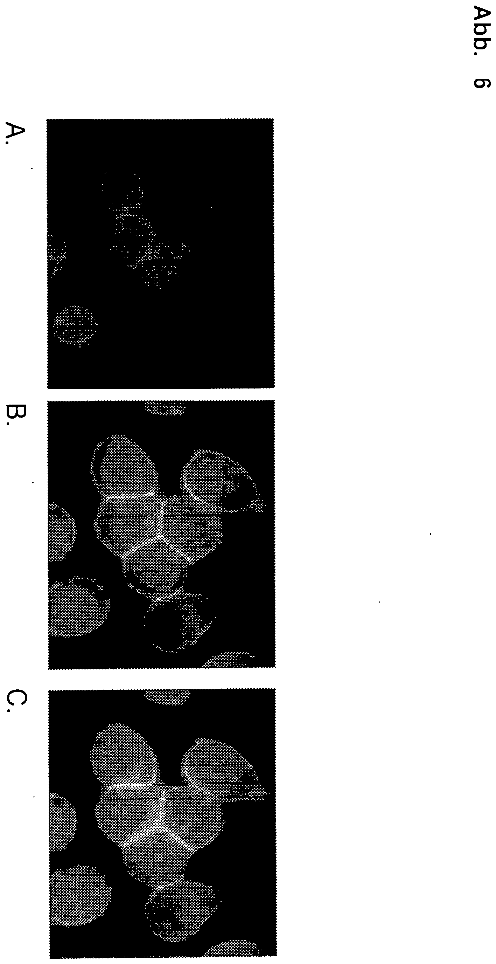

### Abb. 6. Verwendung Claudin-18A2-spezifischer Antikörper (extrazelluläre Domäne)

Membranfärbung von Claudin-18A2-positiven Magenkarzinomzellen (SNU-16) mit einem Antikörper, der durch Immunisierung mit einem Peptid (SEQ ID NO:113, N-terminal-gelegene extrazelluläre Domäne) hergestellt wurde. Zur Gegenfärbung wurde ein monoklonaler Antikörper verwendet, der gegen E-Cadherin gerichtet ist. A-Antikörper; B-Gegenfärbung; C-Überlagerung.

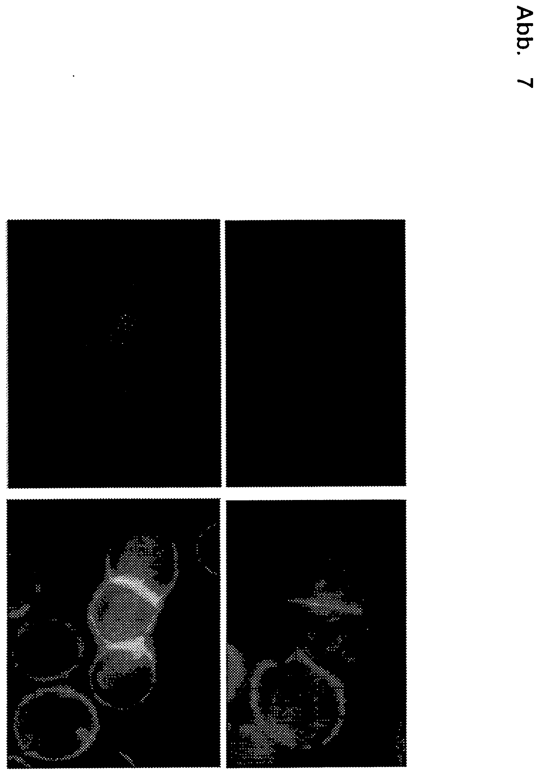

### Abb. 7. Verwendung von Antikörpern gegen die C-terminal extrazelluläre Domäne von Claudin-18

(links, oben und unten) Membranfärbung von Claudin-18A2-positiven Magenkarzinomzellen (SNU-16) mit einem Antikörper, der durch Immunisierung mit einem Peptid (SEQ ID NO:116, C-terminal-gelegene extrazelluläre Domäne) hergestellt wurde. Zur Gegenfärbung wurde ein monoklonaler Antikörper verwendet, der gegen E-Cadherin gerichtet ist (rechts oben, unten).

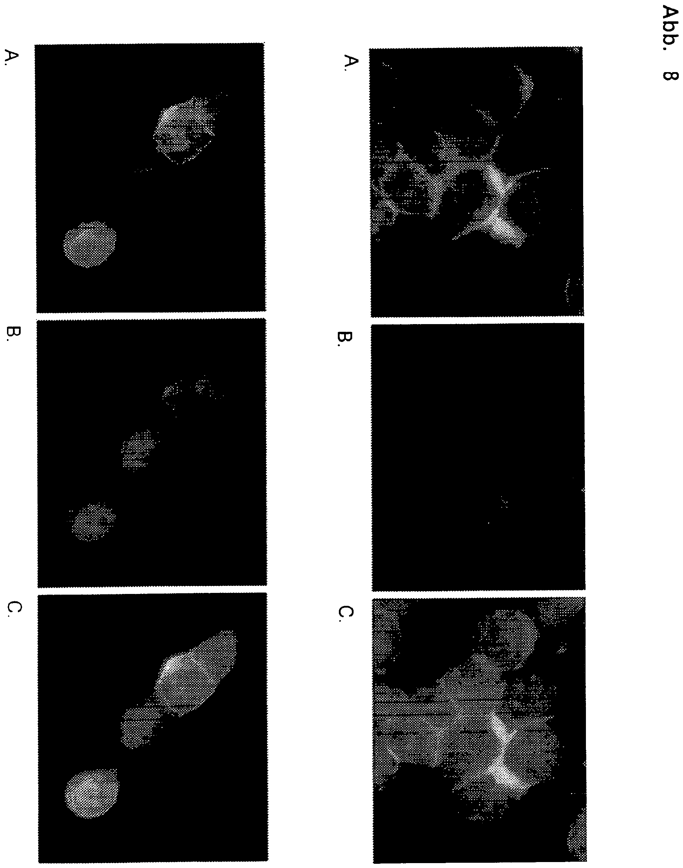

### Abb. 8. Verwendung Claudin-18A1-spezifischer Antikörper

(oben) schwache bis fehlende Färbung von Magenkarzinomezellen (SNU-16; Claudin18A2 positiv) mit einem Antikörper, der durch Immunisierung mit einem Claudin-18A1-spezifischen Peptid (SEQ ID NO: 115) hergestellt wurde. A-anti-E-Cadherin; B-anti-Claudin-18A1; C-Überlagerung.

(unten) Nachweis der Spezifität des Antikörpes durch Kolokalisationsanalyse in Claudin-18A1-GFP-transfizierten 293T-Zellen. A- GFP-Claudin-18A1; B-anti-Claudin-18A1; C-Überlagerung.

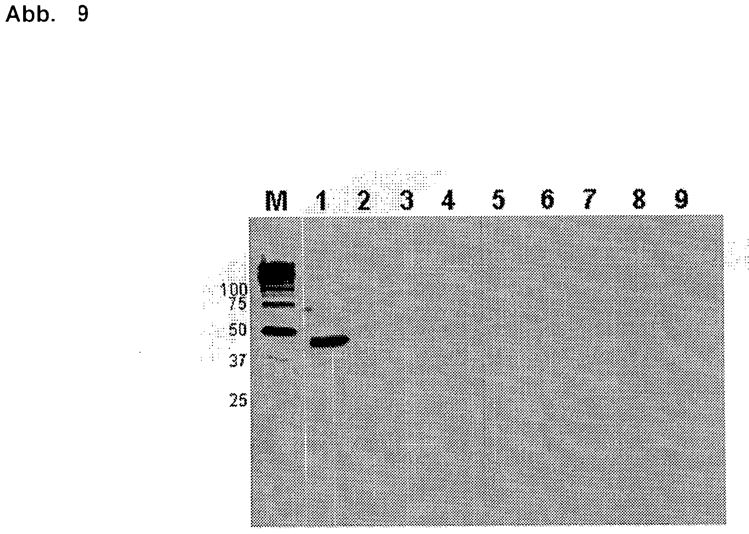

### Abb. 9. Nachweis von Claudin-18A2 im Western-Blot.

Western-Blot mit Lysaten aus verschiedenen gesunden Geweben mit einem Claudin-18A2 spezifischen Antikörper, gerichtet gegen das Epitop mit SEQ ID NO:17. 1-Magen; 2-Testis; 3-Haut; 4-Brust; 5-Leber; 6-Dickdarm; 7-Lunge; 8-Niere; 9-Lymphknoten.

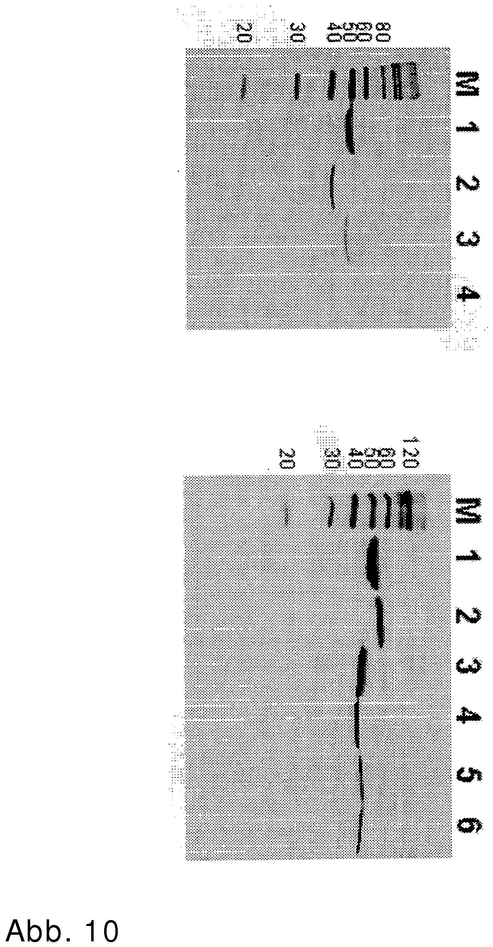

### Abb. 10. Claudin-18A2 Western-Blot mit Proben aus Magen und Magentumoren

Lysate aus Magen und Magentumoren wurden geblottet und mit einem Claudin-18A2-spezifischen Antikörper gegen das Epitop mit SEQ ID NO:17 getestet. Magentumoren weisen eine geringer glykosylierte Form von Claudin-18A2 auf. PNGase F-Behandlung von Magenlysaten führt zur Bildung der niedrigglykosylierten Form.

links: 1-Magen No #A; 2-Magen Tu #A; 3-Magen No #B; 4-Magen Tu #Brechts: 1-Magen No #A; 2-Magen No #B; 3-Magen No #B + PNGase F; 4-Magen Tu #C; 5-Magen Tu #D; 6-Magen Tu #D+ PNGase F

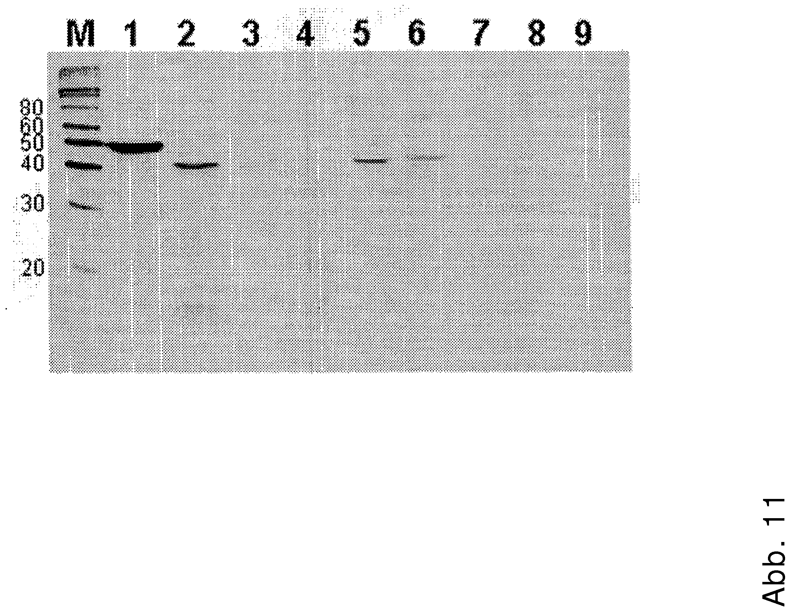

### Abb. 11. Expression von Claudin-18 in Lungentumoren

Entsprechend zu Abb. 10 erfolgte ein Nachweis von niedrigglykosylierten Claudin-18A2-Varianten in Lungentumoren. 1-Magen No; 2-Magen Tu; 3-9-Lunge Tu.

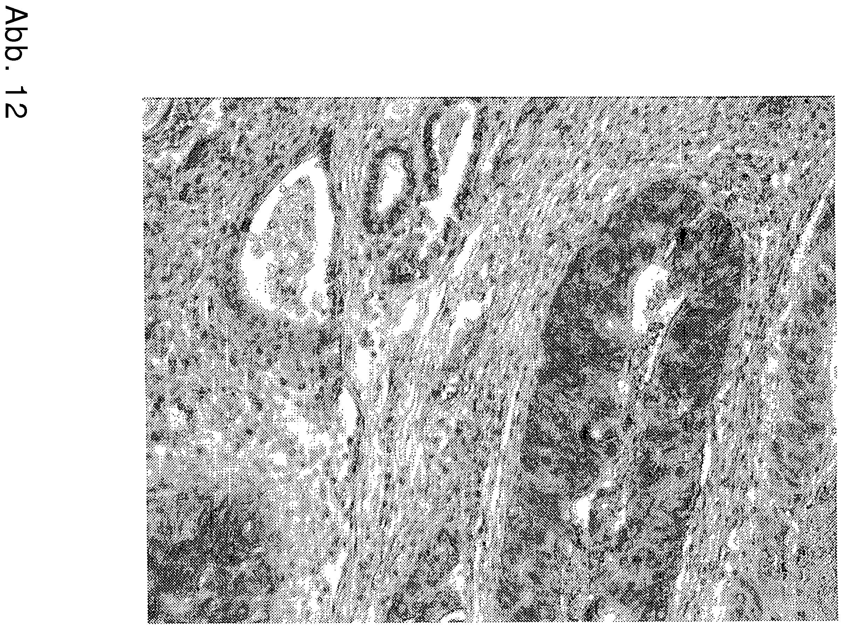

### Abb. 12. Immunhistochemische Analyse von Claudin-18 mit Claudin-18A2-spezifischen Antikörpern in Magentumorgewebe

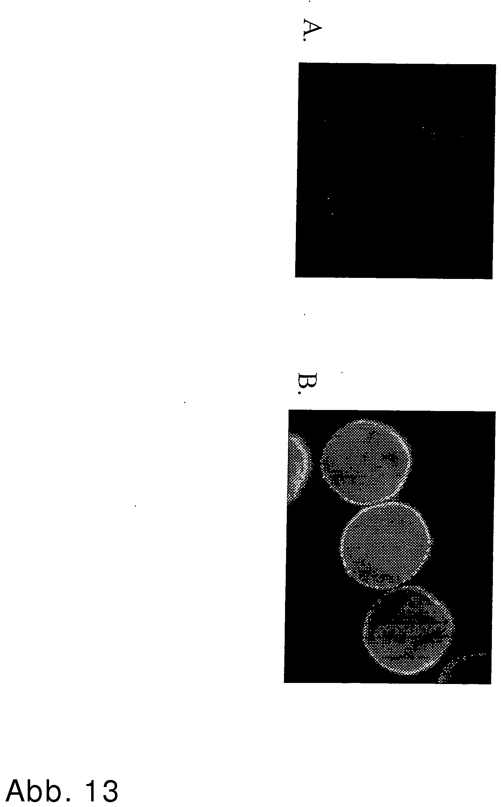

### Abb. 13. Indirekte Immunfluoreszenz von Magen-spezifischen Snu16-Zellen mit einem Claudin-18-spezifischen polyklonalen Antiserum

A. Färbung mit einem Präimmunserum, generiert vor der Immunisierung; B Färbung mit dem Claudin-18-spezifischen Serum.

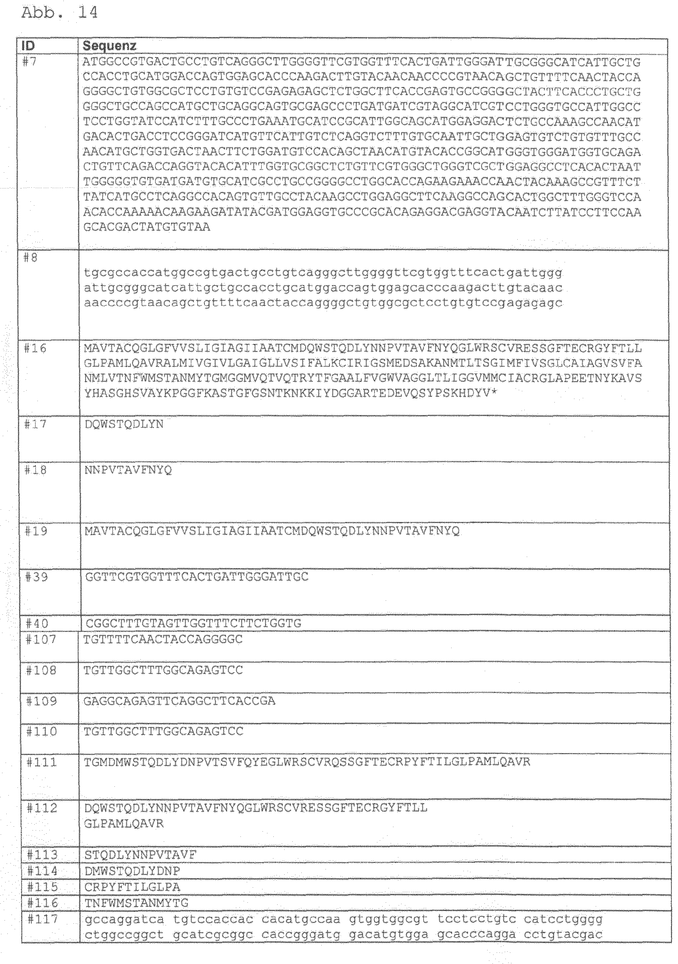

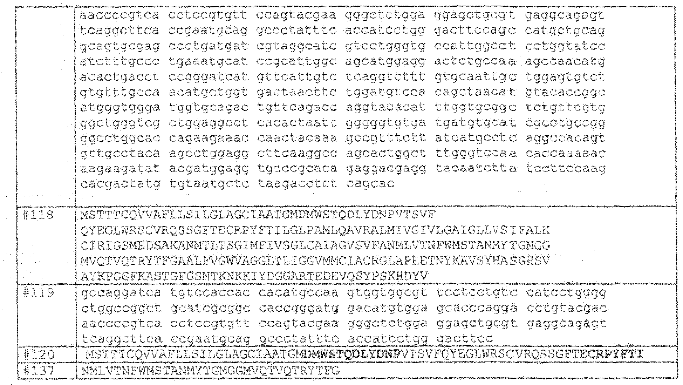

### Abb. 14. Sequenzen

Gezeigt sind die Sequenzen, auf die hierin verwiesen wird.

### Beispiele:

### Material und Methoden

Die Begriffe *"in silico",* "elektronisch" und "virtuell klonieren" beziehen sich rein auf die Nutzung von auf Datenbanken beruhenden Verfahren, mit denen auch Labor-experimentelle Vorgänge simuliert werden können.

Alle anderen Begriffe und Termini werden, falls nicht explizit anders definiert, so verwendet, wie sie der Fachmann versteht. Die genannten Techniken und Methoden erfolgen in an sich bekannter Weise und sind z.B. in Sambrook et al., Molecular Cloning: A Laboratory Manual, 2. Auflage (1989) Cold Spring Harbor Laboratory Press, Cold Spring Harbor, N. Y beschrieben. Alle Verfahren, die die Verwendung von Kits und Reagenzien einschließen, sind entsprechend den Angaben der Hersteller durchgeführt.

### Datamining-basierte Strategie zur Ermittlung von neuen Tumor-assoziierten Genen

Zwei *in silico* Strategien nämlich GenBank-Schlagwort-Suche und der cDNAxProfiler wurden kombiniert. Es wurde unter Nutzung des ENTREZ Search and Retrieval Systems des NCBI (http://www.ncbi.nlm.nih.gov/Entrez) eine Suche nach Kandidaten-Genen in der GenBank durchgeführt, die annotiert sind als spezifisch exprimiert in bestimmten Geweben (Wheeler et al., Nucleic Acids Research 28:10-14, 2000).
Durch Suchabfragen mit Schlagworten wie beispielsweise "colon-specific gene", " stomachspecific gene", oder "kidney-specific gene" wurden Kandidatengene (GOI, genes of interest) aus den Datenbanken herausextrahiert. Die Suche wurde auf einen Teil der Gesamtinformation dieser Datenbanken eingeschränkt, indem als Limits "homo sapiens" für den Organismus und "mRNA" für die Molekülart eingesetzt wurden.
Die Liste der gefundenen GOI wurde kuratiert, indem unterschiedliche Bezeichnungen für dieselbe Sequenz ermittelt und solche Redundanzen behoben wurden.
Alle Kandidatengene, die sich durch die Schlagwort-Suche ergaben, wurden wiederum durch das Verfahren des "electronic Northern" (eNorthern) bezüglich ihrer Gewebeverteilung untersucht. Der eNorthern basiert darauf, dass die Sequenz eines GOI gegenüber einer EST- (expressed sequence tag) Datenbank (Adams et al., Science 252:1651, 1991) abgeglichen wird (http://www.ncbi.nlm.nih.gov/BLAST). Zu jedem EST, das sich als homolog zum eingegebenen GOI ergibt, lässt sich die Gewebeherkunft ermitteln und durch die Summe aller ESTs auf diese Weise eine vorläufige Einschätzung der Gewebeverteilung des GOI erreichen. Nur diejenigen GOI wurden weiteren Untersuchungen zugeführt, die keine Homologien zu EST aus nicht Organ-spezifischen Normalgeweben hatten. Für dies Beurteilung wurde auch berücksichtigt, dass es falschannotierte cDNA-Banken in der öffentlichen Domäne gibt (Scheurle et al., Cancer Res. 60: 4037-4043, 2000) (www.fau.edu/cmbb/publications/cancergenes6.htm). Als zweites Datamining-Verfahren wurde der DNA xProfiler des Cancer Genome Anatomy Projekts des NCBI (http://cgap.nci.nih.gov/Tissues/xProfiler) genutzt (Hillier et al., Genome Research 6:807-828, 1996; Pennisi, Science 276:1023-1024, 1997). Dieser erlaubt, Pools von in Datenbanken abgelegten Transkriptomen durch logische Operatoren in Beziehung zueinander zu setzen. Wir haben einen Pool A definiert, dem beispielsweise alle aus Colon hergestellten Expressionsbibliotheken, unter Ausschluss von gemischten Bibliotheken zugeordnet wurden. Dem Pool B wurden alle cDNA-Bibliotheken zugeordnet, die von Normalgeweben mit Ausnahme von Colon hergestellt waren. Generell wurden alle cDNA-Banken unabhängig vom zugrundeliegenden Herstellungsverfahren genutzt, allerdings lediglich solche mit einer Mächtigkeit > 1000 zugelassen. Mittels des BUT NOT Operators wurde Pool B digital von Pool A subtrahiert. Auch das Set der auf diese Weise gefundenen GOI wurde eNorthern-Studien unterzogen, sowie durch eine Literaturrecherche abgesichert. Dieses kombinierte Datamining schließt alle etwa 13 000 Volllänge-Gene in der öffentlichen Domäne ein und prädiziert aus diesen Gene mit potentieller Organ-spezifischer Expression.

Alle anderen Gene wurden zunächst durch spezifische RT-PCR in Normalgeweben evaluiert. Alle GOI, die sich als in nicht Organ-spezifischen Normalgeweben exprimiert erwiesen, hatten als Falsch-Positive zu gelten und wurden aus weiteren Untersuchungen ausgeschlossen. Die verbliebenen wurden in einem großen Panel an verschiedensten Tumorgeweben untersucht. Die unten dargestellten Antigene erwiesen sich dabei als in Tumorzellen aktiviert.

### RNA-Extraktion, Herstellung von poly-d(T) geprimter cDNA und konventionelle RT-PCR Analyse

Gesamt-RNA aus nativem Gewebematerial wurde unter Verwendung von Guanidiumisothiocyanat als chaotrophem Agens extrahiert (Chomczynski & Sacchi, Anal. Biochem. 162:156-9, 1987). Nach Extraktion mit saurem Phenol und Fällung mit Isopropanol wurde die RNA in DEPC-behandeltem Wasser gelöst.

Aus 2-4 *µ*g Gesamt-RNA wurde in einem 20*µ*l Reaktionsansatz mittels Superscript II (Invitrogen) entsprechend den Angaben des Herstellers eine Erststrang-cDNA-Synthese durchgeführt. Als Primer wurde ein dT(18) Oligonukleotid verwendet. Integrität und Qualität der cDNA wurden durch Amplifikation von p53 in einer 30 Zyklen-PCR (sense CGTGAGCGCTTCGAGATGTTCCG, antisense CCTAACCAGCTGCCCAACTGTAG, Hybridisierungstemperatur 67°C) überprüft.
Es wurde ein Archiv aus Erststrang-cDNAs aus einer Reihe von Normalgeweben und Tumorentitäten hergestellt. Für Expressionsstudien wurden 0,5 *µ*l dieser cDNAs in einem 30*µ*l Reaktionsansatz mit GOI-spezifischen Primern (siehe unten) und 1 U HotStarTaq DNA Polymerase (Qiagen) amplifiziert. Der Reaktionsansatz enthielt jeweils 0,3 mM dNTPs, je 0,3 *µ*M jeden Primers und 3 *µ*l 10 x Reaktionspuffer.
Die Primer wurden so ausgewählt, dass sie in 2 verschiedenen Exons liegen, und die Beseitigung der Interferenz durch kontaminierende genomische DNA als Grund für falsch positive Resultate wurde durch Testen von nicht revers transkribierter DNA als Matrize bestätigt. Nach 15 Minuten bei 95°C zur Aktivierung der HotStarTaq DNA Polymerase wurden 35 Zyklen PCR durchgeführt (1 min 94°C, 1 min jeweilige Hybridisierungstemperatur, 2 min 72°C und abschließende Elongation bei 72°C für 6 min). 20*µ*l dieser Reaktion wurden auf einem mit Ethidiumbromid gefärbten Agarosegel aufgetrennt und analysiert.

Folgende Primer wurden für die Expressionsanalyse der entsprechenden Antigene bei der angegebenen Hybridisierungstemperatur verwendet.

### Claudin-18A2 (68 ° C)

Sense1: 5' -GGTTCGTGGTTTCACTGATTGGGATTGC-3' (SEQ ID NO: 39)
Antisense1: 5' -CGGCTTTGTAGTTGGTTTCTTCTGGTG-3' (SEQ ID NO: 40)
Sense2: 5' - TGTTTTCAACTACCAGGGGC-3' (SEQ ID NO: 107)
Antisense2: 5' - TGTTGGCTTTGGCAGAGTCC-3' (SEQ ID NO: 108)

### Claudin-18A1 (64°C)

Sense: 5' -GAGGCAGAGTTCAGGCTTCACCGA-3' (SEQ ID NO: 109)
Antisense: 5' - TGTTGGCTTTGGCAGAGTCC-3' (SEQ ID NO: 110)

### Herstellung von Random-Hexamer-geprimter cDNA und quantitative Real-Time-PCR

Die Expression mehrere Gene wurde mittels Real-Time-PCR quantifiziert.

Dabei wurden die PCR-Produkte mit SYBR-Green als interkalierendem Reporterfarbstoff detektiert. Die Reporterfluoreszenz von SYBR-Green ist in Lösung supprimiert und erst nach Bindung an dopplesträngige DNA-Fragmente ist der Farbstoff aktiv. Das Ansteigen der SYBR-Green-Fluoreszenz als Folge der spezifischen Amplifikation mittels GOI-spezifischen Primern nach jedem PCR-Zyklus wird zur Quantifizierung genutzt. Die Expressionsquantifizierung des Zielgens erfolgt absolut oder relativ zur Expression eines Kontrollgens mit konstanter Expression in den zu untersuchenden Geweben. Die Expression wurde nach Normalisierung der Proben gegen 18s RNA als sog. Housekeeping-Gen mittels der ΔΔ-Cₜ Methode (PE Biosystems, USA) ermittelt. Die Reaktionen wurden in Duplex-Ansätzen durchgeführt und in Triplikaten bestimmt. Verwendet wurde der QuantiTect SYBR-Green PCR Kit (Qiagen, Hilden) nach Angaben des Herstellers. Die Synthese der cDNA erfolgte mit dem High Capacity cDNA Archive Kit (PE Biosystems, USA) unter Verwendung von Hexamer-Primern nach Angaben des Herstellers. Jeweils 5 *µ*l der verdünnten cDNA wurden in 25 *µ*l Gesamtvolumen für die PCR eingesetzt: sense-Primer 300nM, antisense-Primer 300nM; initiale Denaturierung 95°C 15 min; 95°C 30 sec; Annealing 30 sec; 72°C 30 sec; 40 Zyklen. Die Sequenzen der verwendeten Primer sind in den jeweiligen Beispielen aufgeführt.

### Klonierung und Sequenzanalyse

Klonierung von Vollängen bzw. Genfragmenten erfolgte nach gängigen Methoden. Zur Ermittlung der Sequenz wurden entsprechende Antigene mittels der Proofreading-Polymerase pfu (Stratagene) amplifiziert. Nach Beendigung der PCR wurde Adenosin mittels HotStarTaq DNA Polymerase an die Enden des Amplikons ligiert, um die Fragmente entsprechend den Angaben des Herstellers in den TOPO-TA-Vektor zu klonieren. Die Sequenzierung wurde durch einen kommerziellen Service durchgeführt. Die Sequenzen wurden mittels gängiger Prädiktionsprogramme und Algorithmen analysiert.

### Western-Blot

Zellen aus Zellkultur (endogene Expression des Zielgens oder Synthese des Zielproteins nach Transfektion eines Expressionsvektors, der das Zielprotein kodiert) oder Gewebeproben, die das Zielprotein enthalten könnten, werden in einer 1%igen SDS Lösung lysiert. Das SDS denaturiert dabei die im Lysat enthaltenen Proteine. Die Lysate eines experimentellen Ansatzes werden abhängig von der erwarteten Proteingröße auf 8-15 % igen denaturierenden Polyacrylamidgelen (enthalten 1% SDS) der Größe nach elektrophoretisch aufgetrennt (SDS-Polyacrylamid-Gelelektrophorese, SDS-PAGE). Anschließend werden die Proteine durch das semi-dry Elektroblot Verfahren (Biorad) auf Nitrozellulose-Membran (Schleicher & Schüll) transferiert, auf der das gewünschte Protein nachgewiesen werden kann. Dazu wird die Membran zunächst blockiert (z.B. mit Milchpulver) und anschließend mit dem spezifischen Antikörper in einer Verdünnung von 1:20-1:200 (je nach Spezifität des Antikörpers) für 60 Minuten inkubiert. Nach einem Waschschritt wird die Membran mit einem zweiten, mit einem Marker (z.B. Enzyme wie Peroxidase oder alkalische Phosphatase) gekoppelten Antikörper inkubiert, der den ersten Antikörper erkennt. Nach einem weiteren Waschschritt wird anschließend das Zielprotein in einer Farb- oder Chemilumineszenz-Reaktion auf der Membran mittels einer Enzymreaktion sichtbar gemacht (z.B. ECL, Amersham Bioscience). Das Ergebnis wird durch Aufnahme mit einer geeigneten Kamera dokumentiert.

Die Analyse von Proteinmodifikationen erfolgt in der Regel im Western-Blot. Glykosylierungen, die in der Regel eine Größe von mehreren kDa haben, führen zu einer größeren Gesamtmasse des Zielproteins, die sich in der SDS-PAGE auftrennen lässt. Zum Nachweis von spezifischen O- und N-glykosidischen Bindungen werden Proteinlysate aus Geweben oder Zellen vor der Denaturierung durch SDS mit O- oder N-Glykosidasen inkubiert (nach Angaben des jeweiligen Herstellers, z.B. PNgase, Endoglykosidase F, Endoglykosidase H, Roche Diagnostics). Anschließend erfolgt ein Western-Blot wie vorstehend beschrieben. Bei Verringerung der Größe eines Zielproteins kann so nach Inkubation mit einer Glykosidase eine spezifische Glykosylierung nachgewiesen und auf diesem Weg auch die Tumorspezifität einer Modifikation analysiert werden. Mit Algorithmen und Prädiktionsprogrammen kann die genaue Position der glykosylierten Aminosäure prädiziert werden.

### Immunfluoreszenz

Es werden Zellen etablierter Zelllinien benutzt, die entweder das Zielprotein endogen synthetisieren (Nachweis der RNA in der RT-PCR oder des Proteins im Western-Blot) oder aber vor der IF mit Plasmid-DNA transfiziert worden sind. Zur Transfektion von Zelllinien mit DNA sind die verschiedensten Methoden (z.B. Elektroporation, Liposomen-basierte Transfektion, Calciumphosphatpräzipitation) gut etabliert (z.B. Lemoine et al. Methods Mol. Biol. 1997; 75: 441-7). Das transfizierte Plasmid kann bei der Immunfluoreszenz das unmodifizierte Protein kodieren oder aber auch unterschiedliche Aminosäuremarker an das Zielprotein koppeln. Die wichtigsten Marker sind z.B. das fluoreszierende "green fluorescent protein" (GFP) in seinen verschiedenen differentiell fluoreszierenden Formen und kurze Peptidsequenzen von 6-12 Aminosäuren, für die hoch affine und spezifische Antikörper zur Verfügung stehen. Zellen, die das Zielprotein synthetisieren, werden mit Paraformaldehyd, Saponin oder Methanol fixiert. Anschließend können die Zellen bei Bedarf durch Inkubation mit Detergenzien (z.B. 0,2% Triton X-100) permeabilisiert werden. Nach der Fixierung/Permeabilisierung werden die Zellen mit einem primären Antikörper inkubiert, der gegen das Zielprotein oder gegen einen der gekoppelten Marker gerichtet ist. Nach einem Waschschritt wird der Ansatz mit einem zweiten, mit einem fluoreszierenden Marker (z.B. Fluorescin, Texas Red, Dako) gekoppelten Antikörper inkubiert, der an den ersten Antikörper bindet. Anschließend werden die so markierten Zellen mit Glycerin überschichtet und mit Hilfe eines Fluoreszenzmikroskops nach den Angaben des Herstellers analysiert. Spezifische Fluoreszenzemissionen werden dabei, abhängig von den eingesetzten Substanzen, durch spezifische Anregung erreicht. Die Analyse erlaubt in der Regel die sichere Lokalisation des Zielproteins, wobei zur Bestätigung der Antikörperqualität und des Zielproteins in Doppelfärbungen zusätzlich zum Zielprotein auch die gekoppelten Aminosäuremarker oder andere Markerproteine angefärbt werden, deren Lokalisation bereits in der Literatur beschrieben ist. Ein Sonderfall stellt das GFP und seine Derivate dar, dass direkt angeregt werden kann und selbst fluoresziert, so dass zum Nachweis keine Antikörper benötigt werden.

### Immunhistochemie

Die IHC dient im einzelnen dazu, um (1) die Menge an Zielprotein in Tumor- und Normalgeweben abschätzen zu können, (2) zu analysieren, wie viele Zellen in Tumor- und gesundem Gewebe das Zielgen synthetisieren, und/oder (3) den Zelltyp in einem Gewebe (Tumor, gesunde Zellen) zu definieren, in dem das Zielprotein nachweisbar ist.
Je nach dem individuellen Antikörper müssen unterschiedliche Protokolle verwendet werden (z.B. "Diagnostic Immunohistochemistry by David J., MD Dabbs ISBN: 0443065667" oder in "Microscopy, Immunohistochemistry, and Antigen Retrieval Methods: For Light and Electron Microscopy ISBN: 0306467704").

Die Immunhistochemie (IHC) an spezifischen Gewebeproben dient dem Proteinnachweis im entsprechenden Gewebe. Ziel dieses Verfahrens ist es, die Lokalisation eines Proteins in einem funktionell intakten Gewebeverband zu identifizieren. Die IHC dient im einzelnen dazu, um (1) die Menge an Zielprotein in Tumor- und Normalgeweben abschätzen zu können, (2) zu analysieren, wie viele Zellen in Tumor- und gesundem Gewebe das Zielgen synthetisieren, und (3) den Zelltyp in einem Gewebe (Tumor, gesunde Zellen) zu definieren, in dem das Zielprotein nachweisbar ist. Alternativ können die Proteinmengen eines Zielgens durch Gewebsimmunfluoreszenz mittels Digitalkamera und geeigneter Software (z.B. Tillvision, Till-photonics, Deutschland) quantifiziert werden. Die Technologie ist häufig publiziert worden, Details für Färbung und Mikroskopie sind daher z.B. "Diagnostic Immunohistochemistry" von David J., MD Dabbs ISBN: 0443065667 oder "Microscopy, Immunohistochemistry, and Antigen Retrieval Methods: For Light and Electron Microscopy" ISBN: 0306467704 zu entnehmen. Zu beachten ist, dass aufgrund der Eigenschaften von Antikörpern unterschiedliche Protokolle verwendet werden müssen (nachstehend ist ein Beispiel beschrieben), um zu einem aussagekräftigen Ergebnis zu kommen.

In der Regel werden histologisch definierte Tumorgewebe und als Referenz vergleichbare gesunde Gewebe in der IHC eingesetzt. Als Positiv- und Negativkontrollen können dabei auch Zelllinien dienen, bei denen die Präsenz des Zielgens durch RT-PCR-Analysen bekannt ist. Eine Hintergrundkontrolle ist immer mitzuführen.

Fixierte Gewebe (z.B. Fixation mit aldehydhaltigen Substanzen, Formaldehyd, Paraformaldehyd oder in alkoholischen Lösungen) oder schockgefrorene Gewebestücke mit einer Dicke von 1-10*µ*m werden auf einem Glasträger aufgebracht. Paraffineingebettete Proben werden z.B. mit Xylol deparaffiniert. Die Proben werden mit TBS-T gewaschen und in Serum blockiert. Anschließend erfolgt die Inkubation mit dem ersten Antikörper (Verdünnung: 1:2 bis 1:2000) für 1-18 Stunden, wobei in der Regel affinitätsgereinigete Antikörper verwendet werden. Nach einem Waschschritt erfolgt eine ca. 30-60 minütige Inkubation mit einem zweiten Antikörper, der mit einer Alkalischen Phosphatase (alternativ: z.B. Peroxidase) gekoppelt und gegen den ersten Antikörper gerichtet ist. Anschließend erfolgt eine Farbreaktion unter Verwendung der von Farbsubstraten, die von dem gebundenen Enyzmen umgesetzt werden (vgl. beispielsweise Shi et al., J. Histochem. Cytochem. 39: 741-748, 1991; Shin et al., Lab. Invest. 64: 693-702, 1991). Zum Nachweis der Antikörper-Spezifität kann die Reaktion durch vorherige Zugabe des Immunogens kompetitiert werden.

### Immunisierung

(Siehe auch Monoclonal Antibodies: A Practical Approach by Philip Shepherd, Christopher Dean isbn 0-19-963722-9; Antibodies: A Laboratory Manual by Ed Harlow, David Lane ISBN: 0879693142; Using Antibodies: A Laboratory Manual : Portable Protocol NO. by Edward Harlow, David Lane, Ed Harlow ISBN: 0879695447).

Im Folgenden wird der Herstellungsprozess von Antikörpern kurz beschrieben, Details sind den zitierten Publikationen zu entnehmen. Zunächst werden Tiere (z.B. Kaninchen) durch eine erste Injektion des gewünschten Zielproteins immunisiert. Durch eine zweite oder dritte Immunisierung innerhalb eines definierten Zeitraums (ca. 2-4 Wochen nach der vorangegangenen Immunisierung) lässt sich die Immunantwort des Tieres gegen das Immunogen verstärken. Wiederum nach verschiedenen definierten Zeitabständen (1. Blutung nach 4 Wochen, anschließend ca. alle 2 Wochen mit insgesamt bis zu 5 Entnahmen) wird den Tieren Blut entnommen und daraus ein Immunserum gewonnen.

Die Immunisierung der Tiere erfolgt in der Regel über eines von vier gut etablierten Verfahren, wobei auch andere Verfahren verfügbar sind. Immunisiert werden kann dabei mit Peptiden, die für das Zielprotein spezifisch sind, dem gesamten Protein oder mit extrazellulären Teilsequenzen eines Proteins, das experimentell oder über Prediktionsprogramme identifiziert werden kann.
(1) Im ersten Fall werden an KLH (keyhole limpet hemocyanin) konjugierte-Peptide (Länge: 8-12 Aminosäuren) über ein standardisiertes in vitro-Verfahren synthetisiert und diese Peptide zur Immunisierung verwendet. In der Regel erfolgen 3 Immunisierungen mit einer Konzentration von 5-1000 *µ*g/Immunisierung. Die Durchführung der Immunisierung kann auch als Service von Dienstleistern erfolgen.
(2) Alternativ kann die Immunisierung durch rekombinante Proteine erfolgen. Dazu wird die klonierte DNA des Zielgens in einen Expressionsvektor kloniert und das Zielprotein analog den Bedingungen des jeweiligen Herstellers (z.B. Roche Diagnostics, Invitrogen, Clontech, Qiagen) z.B. zellfrei in vitro, in Bakterien (z.B. E. coli), in Hefe (z.B. S. pombe), in Insektenzellen oder in mammalen Zellen synthetisiert. Nach Synthese in einem der Systeme wird das Zielprotein aufgereinigt, wobei die Aufreinigung dabei in der Regel über standardisierte chromatografische Methoden erfolgt. Dabei können auch Proteine für die Immunisierung verwendet werden, die über einen molekularen Anker als Hilfsmittel zur Reinigung verfügen (z.B. His-Tag, Qiagen; FLAG-Tag, Roche Diagnostics; Gst-Fusionsproteine). Eine Vielzahl von Protokollen finden sich z.B. in den "Current Protocols in Molecular Biology", John Wiley & Sons Ltd., Wiley InterScience.
(3) Falls eine Zelllinie zur Verfügung steht, die das gewünschte Protein endogen synthetisiert, kann auch diese Zelllinie zur Herstellung des spezifischen Antiserums verwendet werden. Die Immunisierung erfolgt dabei in 1-3 Injektionen mit jeweils ca. 1-5 x 10⁷ Zellen.
(4) Die Immunisierung kann auch durch Injektion von DNA (DNA Immunisierung) erfolgen. Dazu wird das Zielgen zunächst in einen Expressionsvektor kloniert, so dass die Zielsequenz unter der Kontrolle eines starken eukaryontischen Promotors steht (z.B. CMV-Promotor). Anschließend werden 5-100 *µ*g DNA als Immunogen mit einer "gene gun" in stark durchblutete, kapillare Bereiche eines Organismus transferiert (z.B. Maus, Kaninchen). Die transferierte DNA wird von Zellen des Tieres aufgenommen, das Zielgen wird exprimiert und das Tier entwickelt schließlich eine Immunantwort gegen das Zielgen (Jung et al., Mol Cells 12: 41-49, 2001; Kasinrerk et al., Hybrid Hybridomics 21: 287-293, 2002).

### Qualitätskontrolle des polyklonalen Serums bzw. Antikörpers

Zum Spezifitätsnachweis eignen sich am besten auf Zellkultur-basierende Tests mit anschließendem Western-Blot (verschiedene Variationen sind z.B. in "Current Protocols in Proteinchemistry", John Wiley & Sons Ltd., Wiley InterScience, beschrieben). Für den Nachweis werden Zellen mit einer cDNA für das Zielprotein transfiziert, die unter der Kontrolle eines starken eukaryontischen Promotors steht (z.B. Cytomegalovirus-Promotor). Zur Transfektion von Zelllinien mit DNA sind die verschiedensten Verfahren (z.B. Elektroporation, auf Liposomen basierende Transfektion, Kalziumphosphatpräzipitation) gut etabliert (z.B. Lemoine et al., Methods Mol. Biol. 75: 441-7, 1997). Alternativ können auch Zelllinien verwendet werden, die das Zielgen endogen exprimieren (Nachweis über Zielgenspezifische RT-PCR). Zur Kontrolle werden im Experiment im Idealfall homologe Gene mit transfiziert, um im folgenden Western-Blot die Spezifität des analysierten Antikörpers nachweisen zu können.

Im anschließenden Western-Blot werden Zellen aus Zellkultur oder Gewebeproben, die das Zielprotein enthalten könnten, in einer 1%igen SDS Lösung lysiert und die Proteine dabei denaturiert. Die Lysate werden auf 8-15%igen denaturierenden Polyacrylamidgelen (enthalten 1% SDS) der Größe nach elekrophoretisch aufgetrennt (SDS-Polyacrylamid-Gelelektrophorese, SDS-PAGE). Anschließend werden die Proteine durch eines von mehreren Blotting-Verfahren (z.B. semi-dry Elektroblot; Biorad) auf eine spezifische Membran transferiert (z.B. Nitrozellulose, Schleicher & Schüll). Auf dieser Membran kann das gewünschte Protein sichtbar gemacht werden. Dazu wird die Membran zunächst mit dem Antikörper, der das Zielprotein erkennt (Verdünnung ca. 1:20-1:200, je nach Spezifität des Antikörpers), für 60 Minuten inkubiert. Nach einem Waschschritt wird die Membran mit einem zweiten, mit einem Marker (z.B. Enzyme wie Peroxidase oder alkalische Phosphatase) gekoppelten Antikörper inkubiert, der den ersten Antikörper erkennt. In einer Farb- oder chemilumineszenten Reaktion kann anschließend das Zielprotein auf der Membran sichtbar gemacht werden (z.B. ECL, Amersham Bioscience). Ein Antikörper mit einer hohen Spezifität für das Zielprotein sollte im Idealfall nur das gewünschte Protein selbst erkennen.

Zur Bestätigung der im in silico-Ansatz identifizierten Membranlokalisation des Zielproteins werden verschiedene Verfahren verwendet. Ein wichtiges und gut etabliertes Verfahren unter Verwendung der vorstehend beschriebenen Antikörper ist die Immunfluoreszenz (IF). Dazu werden Zellen etablierter Zelllinien benutzt, die entweder das Zielprotein synthetisieren (Nachweis der RNA in der RT-PCR oder des Proteins im Western-Blot) oder aber mit Plasmid-DNA transfiziert worden sind. Zur Transfektion von Zelllinien mit DNA sind die verschiedensten Vefahren (z.B. Elektroporation, auf Liposomen basierende Transfektion, Kalziumphosphatpräzipitation) gut etabliert (z.B. Lemoine et al., Methods Mol. Biol. 75: 441-7, 1997). Das in die Zellen transfizierte Plasmid kann bei der Immunfluoreszenz das unmodifizierte Protein kodieren oder aber auch unterschiedliche Aminosäuremarker an das Zielprotein koppeln. Die wichtigsten Marker sind z.B. das fluoreszierende "green fluorescent protein" (GFP) in seinen verschiedenen differentiell fluoreszierenden Formen, kurze Peptidsequenzen von 6-12 Aminosäuren, für die hoch affine und spezifische Antikörper zur Verfügung stehen, oder die kurze Aminosäuresequenz Cys-Cys-X-X-Cys-Cys, die über ihre Cysteine spezifische fluoreszierende Substanzen binden kann (Invitrogen). Zellen, die das Zielprotein synthetisieren, werden z.B. mit Paraformaldehyd oder Methanol fixiert. Anschließend können die Zellen bei Bedarf durch Inkubation mit Detergenzien (z.B. 0,2% Triton X-100) permeabilisiert werden. Anschließend werden die Zellen mit einem primären Antikörper inkubiert, der gegen das Zielprotein oder gegen einen der gekoppelten Marker gerichtet ist. Nach einem Waschschritt wird der Ansatz mit einem zweiten, mit einem fluoreszierenden Marker (z.B. Fluorescin, Texas Red, Dako) gekoppelten Antikörper inkubiert, der an den ersten Antikörper bindet. Anschließend werden die so markierten Zellen mit Glycerin überschichtet und mit Hilfe eines Fluoreszenzmikroskops nach den Angaben des Herstellers analysiert. Spezifische Fluoreszenzemissionen werden dabei, abhängig von den eingesetzten Substanzen, durch spezifische Anregung erreicht. Die Analyse erlaubt in der Regel die sichere Lokalisation des Zielproteins, wobei zur Bestätigung der Antikörperqualität und des Zielproteins in Doppelfärbungen zusätzlich zum Zielprotein auch die gekoppelten Aminosäuremarker oder andere Markerproteine angefärbt werden, deren Lokalisation bereits in der Literatur beschrieben ist. Ein Sonderfall stellt das GFP und seine Derivate dar, die direkt angeregt werden können und selbst fluoreszieren. Die Membranpermeabilität, die durch den Einsatz von Detergenzien gesteuert werden kann, erlaubt in der Immunfluoreszenz den Nachweis, ob ein immunogenes Epitop innerhalb oder außerhalb der Zelle lokalisiert ist. Die Prädiktion der ausgewählten Proteine kann so experimentell untermauert werden. Alternativ kann der Nachweis von extrazellulären Domänen mittels Durchflusszytometrie erfolgen. Dazu werden Zellen unter nicht permeabilisierenden Bedingungen (z.B. mit PBS/Na-Azid/2% FCS/ 5 mM EDTA) fixiert und im Durchflusszytometer nach Angaben des Herstellers analysiert. Nur extrazelluläre Epitope können bei diesem Verfahren von dem zu analysierenden Antikörper erkannt werden. Im Unterschied zur Immunfluoreszenz kann durch Verwendung von z.B. Propidiumiodid oder Trypanblau zwischen toten und lebenden Zellen unterschieden werden und damit falsch positive Ergebnisse vermieden werden.

### Affinitätsreinigung

Die Reinigung der polyklonalen Seren erfolgte im Fall der Peptidantikörper gänzlich oder im Fall der Antikörper gegen rekombinante Proteine teilweise als Service durch die beauftragten Firmen. Hierzu wurde in beiden Fällen das entsprechende Peptid bzw. rekombinante Protein kovalent an eine Matrix gebunden, diese nach der Kopplung mit einem nativen Puffer (PBS: phosphate buffered saline) äquilibriert und im Anschluß mit dem Rohserum inkubiert. Nach einem weiteren Waschschritt mit PBS wurde der Antikörper mit 100 mM Glycin, pH 2,7 eluiert und das Eluat sogleich in 2 M TRIS, pH 8 neutralisiert. Die so gereinigten Antikörper konnten dann zur spezifischen Detektion der Zielproteine sowohl durch Westernblotting als auch durch Immunfluoreszenz eingesetzt werden.

### Herstellung von EGFP-Transfektanten

Für die Immunfluoreszenz-Mikroskopie von heterolog exprimierten Tumor-assoziierten Antigenen wurde der komplette ORF der Antigene in pEGFP-C1- und pEGFP-N3-Vektoren (Clontech) kloniert. Auf Objekträgern kultivierte CHO- und NIH3T3-Zellen wurden mit den entsprechenden Plasmidkonstrukten unter Verwendung von Fugene-Transfektionsreagenz (Roche) nach Herstellerangaben transfiziert und nach 12-24h mittels Immunfluoreszenz-Mikroskopie analysiert.

### Beispiel 1: Identifizierung von Claudin-18A1- und Claudin-18A2-Spleißvarianten als diagnostische und therapeutische Krebs-Targets

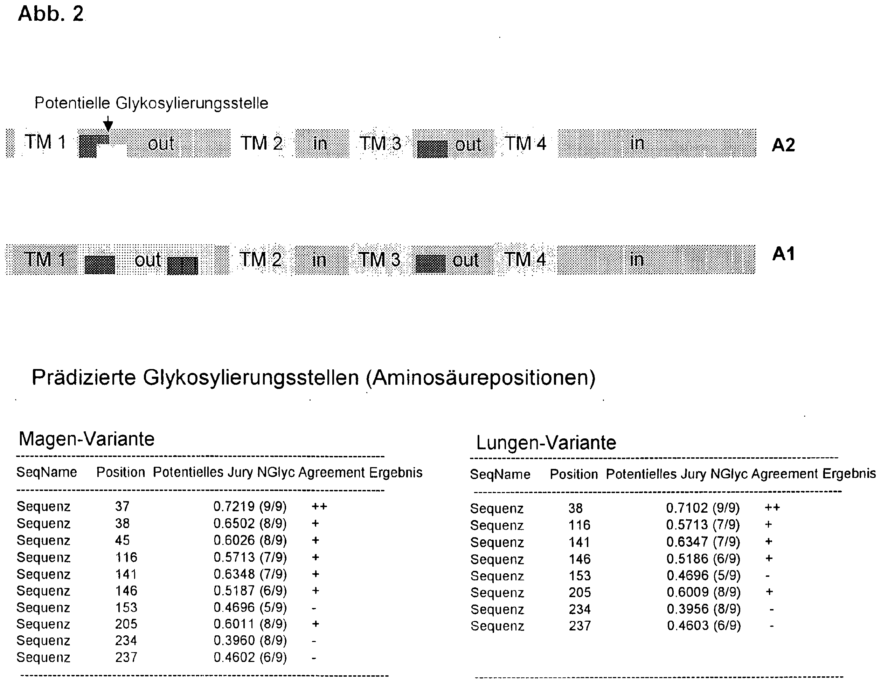

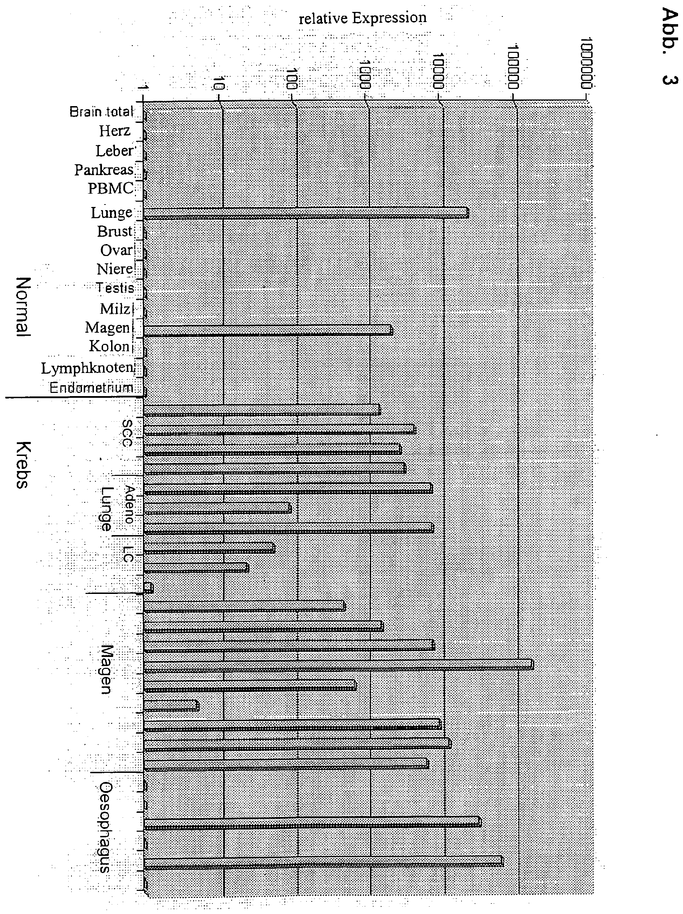

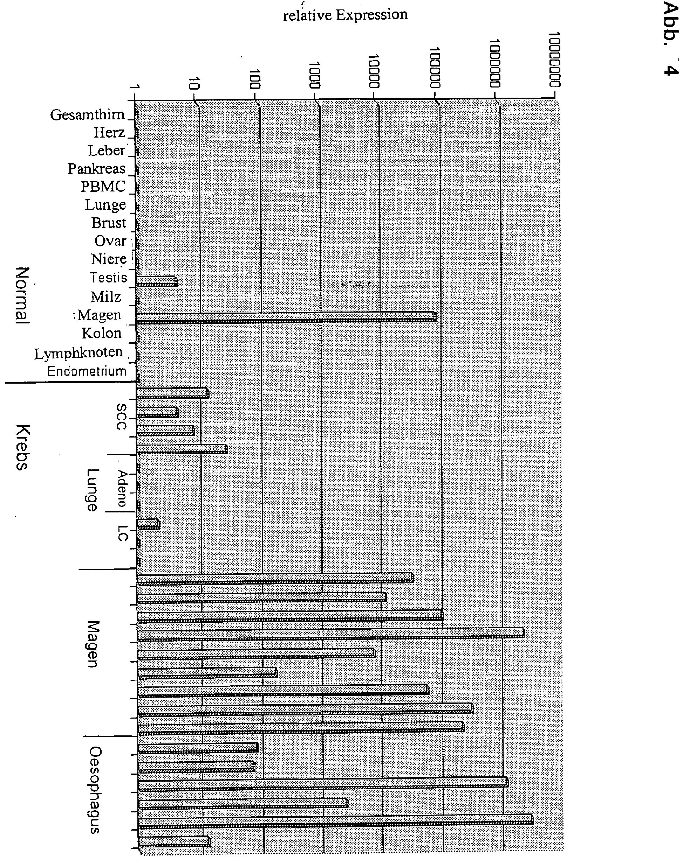

Das Claudin-18-Gen kodiert für ein Oberflächenmembranmolekül mit 4 Transmembrandomänen und intrazellulärem N- wie auch C-Terminus. Niimi und Kollegen (Mol. Cell. Biol. 21:7380-90, 2001) beschrieben zwei Spleißvarianten des Maus- und humanen Claudin-18, die als selektiv in Lungengewebe (Claudin-18A1) bzw. in Magengewebe (Claudin-18A2) exprimiert beschrieben wurden. Diese Varianten unterscheiden sich im N-Terminus (Abb. 2). Die internationalen Anmeldungen WO02/18576 und WO01/68848 beschreiben verschiedene Nukleinsäuren und hiervon abgeleitete Translationsprodukte und schlagen u.a. vor, das Translationsprodukt mit der Aminosäuresequenz von Claudin-18A1 als Zielstruktur für diagnostische und therapeutische Mittel in Zusammenhang mit Lungenkrebs zu verwenden. Erfindungsgemäß wurde untersucht, inwieweit die Spleißvarianten Claudin-18A2 (SEQ ID NO:7) und Claudin-18A1 (SEQ ID NO: 117) sowie ihre jeweiligen Translationsprodukte (SEQ ID NO: 16 und 118) als Marker bzw. therapeutische Zielstrukturen für Tumoren genutzt werden können. Es wurde eine quantitative PCR, die zwischen beiden Varianten unterscheiden kann, etabliert, indem A1-spezifische (SEQ ID NO 109 & 110) bzw. A2-spezifische (SEQ ID NO 107 & 108) Primerpaare ausgewählt wurden. Die Spleißvariante A2 wurde zusätzlich mit einem zweiten Primerpaar in einer konventionellen PCR getestet (SEQ ID NO: 39 & 40). Für die Variante A1 ist beschrieben, dass sie nur in der normalen Lunge aktiv ist. Jedoch wurde erfindungsgemäß überraschenderweise festgestellt, dass die Variante A1 auch in der Magenschleimhaut aktiv ist. Magen und Lunge sind die einzigen Normalgewebe, die eine signifikante Aktivierung aufweisen. Alle anderen Normalgewebe sind negativ für Claudin-A1. Bei der Untersuchung von Tumoren wurde überraschend festgestellt, dass Claudin-A1 in einer Vielzahl von Tumorgeweben stark aktiviert ist. Ein besonders starke Expression findet sich in Magentumoren, Lungentumoren, Pankreaskarzinomen, Speiseröhrenkarzinomen (Abb. 3), Tumoren des HNO-Bereichs und Prostatakarzinomen. Die Expressionslevel von Claudin-A1 in HNO-, Prostata-, Pankreas- und Speiseröhrentumoren sind 100-10000 höher als die Level in dem korrospondierenden Normalgeweben. Für die Untersuchung der Claudin-A2-Spleißvariante wurden Oligonukleotide verwendet, die spezifisch die Amplifikation dieses Transkripts ermöglichen (SEQ ID NO: 39 & 40 bzw. 107 & 108). Die Untersuchung ergab, dass die Spleißvariante A2 in keinem der mehr als 20 untersuchten Normalgewebe außer in Magenschleimhaut und in geringem Ausmaß auch Testisgewebe exprimiert wird. Wir stellten fest, dass wie die Variante A1 auch die Variante A2 in vielen Tumoren aktiviert ist (beispielhaft dargestellt in Abb. 4). Hierzu zählen Magentumore (8/10), Bauchspeicheldrüsentumoren (6/6), Speiseröhrenkarzinome (5/10) und Leberkarzinome. Obwohl sich in gesunder Lunge keine Aktivierung von Claudin-18A2 nachweisen lässt, wurde überraschend festgestellt, dass ein Teil der Lungentumoren die Spleißvariante A2.1 exprimieren.

**Tabelle 1A. Expression von Claudin-18A2 in Normal- und Tumor-Geweben**

| **Normalgewebe** | **Expression** |
|---|---|
| Gehirn | - |
| Cerebellum | - |
| Myokard | - |
| Skelettmuskel | - |
| Endometrium | - |
| Magen | +++ |
| Colon (Dickdarm) | - |
| Pankreas | - |
| Niere | - |
| Leber | - |
| Testis (Hoden) | + |
| Thymus | - |
| Mamma (Brust) | - |
| Ovar | - |
| Uterus | - |
| Haut | - |
| Lunge | - |
| Thyroid | - |
| Lymphknoten | - |
| Milz | - |
| PBMC | - |
| Ösophagus | - |
| | |

| **Tumortyp** | **Expression** |
|---|---|
| Colonkarzinom | - |
| Pankreaskarzinom | ++ |
| Ösophaguskarzinom | ++ |
| Magenkarzinom | +++ |
| Bronchialkarzinom | ++ |
| Mammakarzinom | - |
| Ovarialkarzinom | - |
| Endometriumkarzino | n.u. |
| HNO-Tumoren | ++ |
| Nierenzellkarzinom | - |
| Prostatakarzinom | - |
| | |
| | |

**Tabelle 1B. Expression von Claudin-18A1 in Normal- und Tumor-Geweben**

| **Normalgewebe** | **Expression** |
|---|---|
| Gehirn | - |
| Cerebellum | - |
| Myokard | - |
| Skelettmuskel | - |
| Endometrium | - |
| Magen | +++ |
| Colon (Dickdarm) | - |
| Pankreas | - |
| Niere | - |
| Leber | - |
| Testis (Hoden) | + |
| Thymus | - |
| Mamma (Brust) | - |
| Ovar | - |
| Uterus | - |
| Haut | - |
| Lunge | +++ |
| Thyroid | - |
| Lymphknoten | - |
| Milz | - |
| PBMC | - |
| Ösophagus | - |
| | |

| **Tumortyp** | **Expression** |
|---|---|
| Colonkarzinom | - |
| Pankreaskarzinom | ++ |
| Ösophaguskarzinom | ++ |
| Magenkarzinom | +++ |
| Bronchialkarzinom | ++ |
| Mammakarzinom | + |
| Ovarialkarzinom | n.u |
| Endometriumkarzino | n.u. |
| HNO-Tumoren | ++ |
| Nierenzellkarzinom | - |
| Prostatakarzinom | ++ |
| | |
| | |

Auch die konventionelle PCR als unabhängige Kontrolluntersuchung bestätigte die Resultate der quantitativen PCR. Hierzu wurden Oligonukleotide (SEQ ID NO: 39, 40) verwendet, die eine spezifische Amplifikation der Spleißvariante A2 erlauben. Erfindungsgemäß wurde gezeigt, dass 8/10 Magenkarzinome und die Hälfte der getesteten Pankreaskarzinome eine starke Expression dieser Spleißvariante aufweisen (Abb. 1). Dagegen ist eine Expression mit konventioneller PCR in anderen Geweben nicht nachweisbar. Insbesondere findet sich in Lunge, Leber, Blut, Lymphknoten, Brust- und Nierengewebe keine Expression (Tab. 1). Hiermit stellen die Spleißvarianten erfindungsgemäß hochspezifische molekulare Marker für Tumoren des oberen Magendarmtraktes wie auch Lungentumoren, HNO-Tumoren, Prostatakarzinome und ihre Metastasen dar. Diese molekulare Marker können erfindungsgemäß zum Nachweis von Tumorzellen genutzt werden. Die Detektion der Tumoren kann erfindungsgemäß mit den genannten Oligonukleotiden (SEQ ID NO. 39, 40, 107-110) erfolgen. Als Oligonukleotide eignen sich insbesondere Primerpaare, von denen mindestens einer unter stringenten Bedingungen an einen 180 Basenpaare langen Abschnitt des Transkripts bindet, der spezifisch für die eine (SEQ ID NO: 8) oder andere Spleißvariante (SEQ ID NO: 119) ist.

Um diese Daten auf Proteinebene zu bestätigen, wurden Claudin-spezifische Antikörper bzw. Immunseren durch Immunisierung von Tieren generiert. Über Analyse der Transmembrandomänen mit bioinformatischen Werkzeugen (TMHMM, TMPRED) und Immunfluoreszensuntersuchungen von Zellen, die mit enhanced GFP getagte Claudin-18-Fusionsproteine exprimieren, wurde die Plasmamembranlokalisation von Claudin-18 und die Proteintopologie bestätigt. Claudin 18 besitzt zwei extrazelluläre Domänen. Die N-terminale extrazelluläre Domäne unterscheidet sich in der Sequenz bei den beiden Spleißvarianten (SEQ ID NO: 111 für A1 und SEQ ID NO: 112 für A2). Die C-terminale extrazelluläre Domäne ist für beide Varianten identisch (SEQ ID NO: 137). Bisher sind noch keine Antikörper beschrieben, die an die extrazelluläre Domänen von Claudin-18 binden. Erfindungsgemäß wurden für die Immunisierung extrazellulär gelegene Peptidepitope ausgewählt, die spezifisch für die Variante A1 oder A2 sind bzw. in beiden Varianten vorkommen. Beide Varianten von Claudin-18 haben keine klassischen Glykosylierungsmotive und eine Glykosylierung des Proteins war daher nicht zu erwarten. Trotzdem wurde bei der Auswahl der Epitope berücksichtigt, dass Epitope, die Asparagin, Serin, Threonin enthalten in seltenen Fällen auch ohne klassische Glykosylierungsstellen potentiell glykosyliert sind. Die Glykosylierung eines Epitops kann die Bindung eines für dieses Epitop-spezifischen Antikörpers verhindern. Erfindungsgemäß wurden u.a. Epitope so ausgewählt, dass die damit generierten Antikörper eine Unterscheidung des Glykosylierungsstatus des Antigens erlauben.

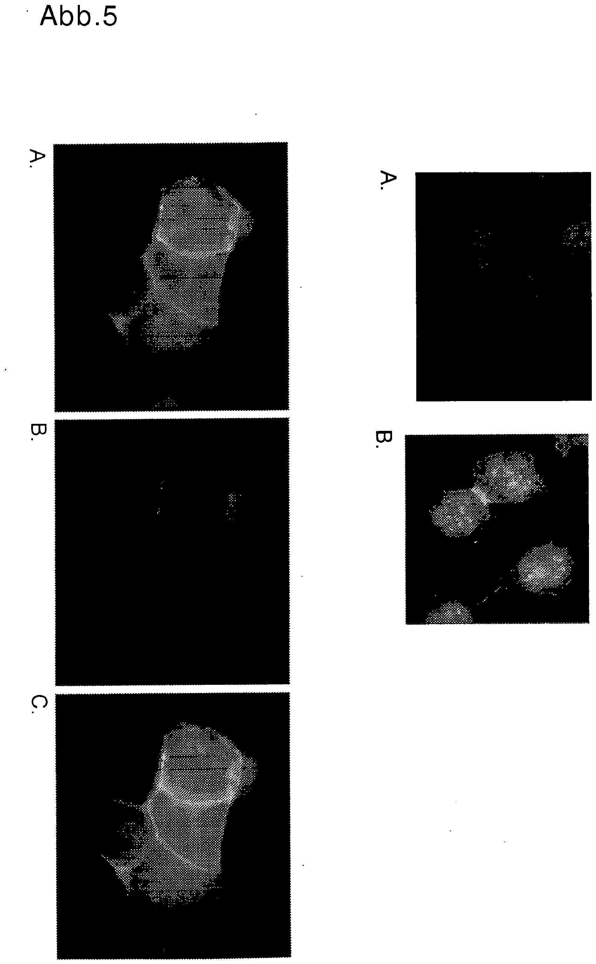

Unter anderem wurden zur Immunisierung folgende Peptide für die Herstellung von Antikörpern ausgewählt:
SEQ ID NO: 17: DQWSTQDLYN (N-terminal-extrazelluläre Domäne, A2-spezifisch, Bindung unabhängig von Glykosylierung)
SEQ ID NO: 18: NNPVTAVFNYQ (N-terminal-extrazelluläre Domäne, A2-spezifisch, Bindung hauptsächlich an unglykosylierte Form, N37)
SEQ ID NO: 113: STQDLYNNPVTAVF (N-terminal-extrazelluläre Domäne, A2-spezifisch, Bindung nur an nicht glykosylierte Form, N37)
SEQ ID NO: 114: DMWSTQDLYDNP (N-terminal-extrazelluläre Domäne, A1-spezifisch)
SEQ ID NO: 115: CRPYFTILGLPA (N-terminal-extrazelluläre Domäne, hauptsächlich spezifisch für A1)
SEQ ID NO: 116: TNFWMSTANMYTG (C-terminal-extrazelluläre Domäne, erkennt sowohl A1 als auch A2).
Beispielhaft werden die Daten für den A2-spezifischen Antikörper, der durch Immunisierung mit SEQ ID NO: 17 hergestellt wurde, dargestellt. Der spezifische Antikörper lässt sich unter verschiedenen Fixationsbedigungen für Immunfluoreszenz-Untersuchungen nutzen. Bei vergleichenden Färbungen von RT-PCR-positiven wie auch negativen Zelllinien ist das entsprechende Protein in gut nachweisbarer Menge spezifisch in den als positiv typisierten Magenkarzinom-Zelllinien detektierbar (Abb. 5). Das endogene Protein ist membranlokalisiert und bildet größere fokale Aggregate an der Membran. Dieser Antikörper wurde des weiteren für einen Proteinnachweis im Western-Blot eingesetzt. Erwartungsgemäß wird Protein lediglich in Magen und keinem anderen Normalgewebe, auch nicht Lunge detektiert (Abb. 9). Bei der vergleichenden Färbung von Magentumoren und adjazentem normalem Magengewebe aus Patienten fiel überraschend auf, dass in allen Magentumoren, in denen Claudin-18 A2 detektiert wird, dieses Protein ein kleineres Massengewicht hat (Abb. 10, links). In einer Serie von Experimenten wurde erfindungsgemäß festgestellt, dass eine Bande auf dieser Höhe sich auch ergibt, wenn man Lysat normalen Magengewebes mit dem deglykosylierenden Agens PNGase F behandelt (Abb. 10, rechts). Während in allen normalen Magengeweben ausschließlich die glykosylierte Form der Variante A2 nachweisbar ist, ist A2 als solches in über 60% der untersuchten Magenkarzinome und zwar ausschließlich in der deglykosylierten Form nachweisbar. Obwohl die A2-Variante von Claudin-18 in normaler Lunge auch auf Proteinebene nicht detektiert wird, ist sie wie auch schon in der quantitativen RT-PCR in Bronchialkarzinomen zu finden. Auch hier liegt lediglich die deglykosylierte Variante vor (Abb. 11). Erfindungsgemäß sind Antikörper hergestellt worden, die die extrazelluläre Domäne der Spleißsvariante Claudin-18-A2 erkennen. Des weiteren wurden Antikörper hergestellt, die selektiv die N-terminale Domäne der Spleißvariante Claudin-18-A1 erkennen (Abb. 8) bzw. Antikörper, die an beide Varianten im Bereich der C-terminalextrazellulären Domäne binden (Abb. 7). Erfindungsgemäß können derartige Antikörper für diagnostische Zwecke z.B. Immunhistologie (Abb. 12) aber auch für therapeutische Zwecke wie oben erläutert verwendet werden. Ein weiterer wichtiger Aspekt betrifft differentiell glykosylierte Domänen von Claudin-18. Erfindungsgemäß wurden Antikörper hergestellt, die exklusiv an nicht-glykosylierte Epitope binden. Claudin-18 selbst ist ein hochselektives Differenzierungsantigen von Magengewebe (A2) bzw. von Lunge und Magen (A1). Da es offensichtlich von Veränderungen der Glykosylierungmaschinerie in Tumoren betroffen ist, entsteht in Tumoren eine besondere Variante von A2, die deglykosyliert ist. Dies kann diagnostisch wie auch therapeutisch genutzt werden. Immunseren, wie das hier beschriebene (gegen Peptid SEQ ID NO: 17) können z.B. im Western-Blot diagnostisch genutzt werden. Antikörper, die an das glykosylierte Epitop gar nicht binden können, wie z.B. durch Immunisierung mit Peptid SEQ ID NO: 113 (Abbildung 6) erhalten, können in der Bindung Tumor- von Normalgewebe unterscheiden. Insbesondere können solche Antikörper therapeutisch eingesetzt werden, da sie hochselektiv sind. Die hergestellten Antikörper können direkt auch zur Herstellung von chimären oder humanisierten rekombinanten Antikörpern verwendet werden. Dies kann auch direkt mit Antikörpern erfolgen, die aus Kaninchen gewonnen wurden (s. dazu J Biol Chem. 2000 May 5;275(18):13668-76 von Rader C, Ritter G, Nathan S, Elia M, Gout I, Jungbluth AA, Cohen LS, Welt S, Old LJ, Barbas CF 3rd. "The rabbit antibody repertoire as a novel source for the generation of therapeutic human antibodies"). Hierzu wurden von den immunisierten Tieren Lymphozyten asserviert. Auch für immuntherapeutische Verfahren wie Vakzinen bzw. den adoptiven Transfer von Antigen-spezifischen T-Lymphozyten stellen die Aminosäuren 1-47 (SEQ ID NO: 19 und 120) besonders gute Epitope dar.

## Patentansprüche

1. Pharmazeutische Zusammensetzung zur Verwendung in einem Verfahren zur Behandlung einer Krebserkrankung, die sich durch die Expression eines Tumor-assoziierten Antigens auszeichnet, umfassend einen oder mehrere Bestandteile, die aus der Gruppe ausgewählt sind, bestehend aus:
(i) einem Tumor-assoziierten Antigen oder einem Teil davon,
(ii) einer Nukleinsäure, die für ein Tumor-assoziiertes Antigen oder einen Teil davon kodiert,
(iii) einem Antikörper, der an ein Tumor-assoziiertes Antigen bindet, und
(iv) einer Wirtszelle, die ein Tumor-assoziiertes Antigen oder einen Teil davon exprimiert,
wobei der Teil mindestens 6 aufeinanderfolgende Aminosäuren des Tumor-assoziierten Antigens umfasst und wobei das Tumor-assoziierte Antigen von einer Nukleinsäuresequenz gemäß SEQ ID NO: 117 kodiert wird und wobei die Krebserkrankung ein Pankreaskarzinom oder ein Ösophaguskarzinom ist.

2. Pharmazeutische Zusammensetzung zur Verwendung nach Anspruch 1, wobei die Wirtszelle das Tumor-assoziierte Antigen oder den Teil davon sekretiert, auf der Oberfläche exprimiert oder ein HLA-Molekül exprimiert, das an das Tumor-assoziierte Antigen oder den Teil davon bindet.

3. Verfahren zur Diagnose einer Krebserkrankung, die sich durch die Expression eines Tumor-assoziierten Antigens auszeichnet, umfassend
(i) den Nachweis einer Nukleinsäure, die für das Tumor-assoziierte Antigen kodiert, und/oder
(ii) den Nachweis des Tumor-assoziierten Antigens,
in einer aus einem Patienten isolierten biologischen Probe, wobei das Tumor-assoziierte Antigen von einer Nukleinsäuresequenz gemäß SEQ ID NO: 117 kodiert wird und wobei die Krebserkrankung ein Pankreaskarzinom oder ein Ösophaguskarzinom ist.

4. Antikörper, der spezifisch an ein Tumor-assoziiertes Antigen bindet, zur Verwendung in einem Verfahren zur Behandlung, Diagnose oder Überwachung einer Krebserkrankung, die sich durch die Expression des Tumor-assoziierten Antigens auszeichnet, wobei das Verfahren die Verabreichung des Antikörpers umfasst, wobei das Tumor-assoziierte Antigen von einer Nukleinsäuresequenz gemäß SEQ ID NO: 117 kodiert wird und wobei die Krebserkrankung ein Pankreaskarzinom oder ein Ösophaguskarzinom ist.

5. Pharmazeutische Zusammensetzung zur Verwendung nach Anspruch 1 oder Antikörper zur Verwendung nach Anspruch 4, wobei der Antikörper mit einem therapeutischen oder diagnostischen Mittel gekoppelt ist.

6. Pharmazeutische Zusammensetzung zur Verwendung nach Anspruch 1 oder 5 oder Antikörper zur Verwendung nach einem der Ansprüche 4 oder 5 wobei der Antikörper ein monoklonaler, chimärer oder humanisierter Antikörper oder ein Antigen-bindendes Fragment eines Antikörpers ist.

7. Pharmazeutische Zusammensetzung zur Verwendung nach einem der Ansprüche 1 oder 2, 5 oder 6 Verfahren nach Anspruch 3 oder Antikörper zur Verwendung nach einem der Ansprüche 4 bis 6 wobei das Tumor-assoziierte Antigen eine Aminosäuresequenz gemäß SEQ ID NO: 118 aufweist.

8. Verwendung eines Kits zur Diagnose einer Krebserkrankung, die sich durch die Expression eines Tumor-assoziierten Antigens auszeichnet, umfassend Mittel zum Nachweis
(i) der Nukleinsäure, die für das Tumor-assoziierte Antigen kodiert, oder
(ii) des Tumor-assoziierten Antigens,
wobei das Tumor-assoziierte Antigen von einer Nukleinsäuresequenz gemäß SEQ ID NO: 117 kodiert wird und wobei die Krebserkrankung ein Pankreaskarzinom oder ein Ösophaguskarzinom ist.

## Claims

1. A pharmaceutical composition for use in a method of treatment of a cancer disease, which is **characterized by** expression of a tumor-associated antigen, comprising one or more components selected from the group consisting of:
(i) a tumor-associated antigen or a part thereof,
(ii) a nucleic acid which codes for a tumor-associated antigen or a part thereof,
(iii) an antibody which binds to a tumor-associated antigen, and
(iv) a host cell which expresses a tumor-associated antigen or a part thereof,
wherein the part comprises at least 6 consecutive amino acids of the tumor-associated antigen and wherein the tumor-associated antigen is encoded by a nucleic acid sequence according to SEQ ID NO: 117 and wherein the cancer disease is a pancreas carcinoma or an esophagus carcinoma.

2. The pharmaceutical composition for use of claim 1, wherein the host cell secretes the tumor-associated antigen or the part thereof, expresses it on the surface or expresses and HLA-molecule which binds to the tumor-associated antigen or to the part thereof.

3. A method for diagnosing a cancer disease, which is **characterized by** expression of a tumor-associated antigen, comprising
(i) detection of a nucleic acid which codes for the tumor-associated antigen, and/or
(ii) detection of the tumor-associated antigen,
in a biological sample isolated from a patient, wherein the tumor-associated antigen is encoded by a nucleic acid sequence according to SEQ ID NO: 117 and wherein the cancer disease is a pancreas carcinoma or an esophagus carcinoma.

4. An antibody which binds specifically to a tumor-associated antigen for use in a method of treatment, diagnosis or monitoring a cancer disease, which is **characterized by** expression of the tumor-associated antigen, wherein the method comprises the administration of the antibody, wherein the tumor-associated antigen is encoded by a nucleic acid sequence according to SEQ ID NO: 117 and wherein the cancer disease is a pancreas carcinoma or an esophagus carcinoma.

5. Pharmaceutical composition for use of claim 1 or antibody for use of claim 4, wherein the antibody is coupled to a therapeutic or diagnostic agent.

6. Pharmaceutical composition for use of claim 1 or 5 or antibody for use of claim 4 or 5, wherein the antibody is a monoclonal, chimeric or humanized antibody or an antigen-binding fragment of an antibody.

7. Pharmaceutical composition for use of any one of claims 1 or 2, 5 or 6, method of claim 3 or antibody for use of any one of claims 4 to 6, wherein the tumor-associated antigen has an amino acid sequence according to SEQ ID NO: 118.

8. Use of a kit for diagnosing a cancer disease, which is **characterized by** expression of a tumor-associated antigen, comprising agents for detection of
(i) the nucleic acid which codes for the tumor-associated antigen, or
(ii) the tumor-associated antigen,
wherein the tumor-associated antigen is encoded by a nucleic acid sequence according to SEQ ID NO: 117 and wherein the cancer disease is a pancreas carcinoma or an esophagus carcinoma.

## Revendications

1. Composition pharmaceutique pour utilisation dans un procédé pour le traitement d'une affection cancéreuse, qui se manifeste par l'expression d'un antigène associé à une tumeur, comprenant un ou plusieurs composants qui sont choisis dans le groupe consistant en:
(i) un antigène associé à une tumeur ou une partie de celui-ci,
(ii) un acide nucléique qui code pour un antigène associé à une tumeur ou une partie de celui-ci,
(iii) un anticorps qui se lie à un antigène associé à une tumeur, et
(iv) une cellule hôte qui exprime un antigène associé à une tumeur ou une partie de celui-ci,
tandis que la partie englobe au moins 6 acides aminés consécutifs de l'antigène associé à une tumeur, et tandis que l'antigène associé à une tumeur est codé par une séquence d'acide nucléique selon SEQ ID NO: 117, et tandis que l'affection cancéreuse est un carcinome du pancréas ou un carcinome de l'oesophage.

2. Composition pharmaceutique pour utilisation selon la revendication 1, dans laquelle la cellule hôte sécrète l'antigène associé à une tumeur ou une partie de celui-ci, exprime à la surface ou exprime une molécule de HLA qui se lie à l'antigène associé à une tumeur ou à une partie de celui-ci.

3. Procédé pour le diagnostic d'une affection cancéreuse, qui se manifeste par l'expression d'un antigène associé à une tumeur, comprenant
(i) l'identification d'un acide nucléique qui code pour l'antigène associé à une tumeur, et/ou
(ii) l'identification de l'antigène associé à une tumeur,
dans un échantillon biologique isolé à partir d'un patient, tandis que l'antigène associé à une tumeur est codé par une séquence d'acide nucléique selon SEQ ID NO: 117 et tandis que l'affection cancéreuse est un carcinome du pancréas ou un carcinome de l'oesophage.

4. Anticorps qui se lie spécifiquement à un antigène associé à une tumeur, pour utilisation dans un procédé pour le traitement, le diagnostic ou le suivi d'une affection cancéreuse qui se manifeste par l'expression de l'antigène associé à une tumeur, tandis que le procédé englobe l'administration de l'anticorps, l'antigène associé à une tumeur étant codé par une séquence d'acide nucléique selon SEQ ID NO: 117 et l'affection cancéreuse étant un carcinome du pancréas ou un carcinome de l'oesophage.

5. Composition pharmaceutique pour utilisation selon la revendication 1 ou anticorps pour utilisation selon la revendication 4, dans lequel l'anticorps est couplé avec un agent thérapeutique ou de diagnostic.

6. Composition pharmaceutique pour utilisation selon la revendication 1 ou 5 ou anticorps pour utilisation selon l'une des revendications 4 ou 5, dans lesquels l'anticorps est un anticorps chimérique ou humanisé monoclonal ou un fragment d'un anticorps lié à un antigène.

7. Composition pharmaceutique pour utilisation selon l'une des revendications 1 ou 2, 5 ou 6, procédé selon la revendication 3, ou anticorps pour utilisation selon l'une des revendications 4 à 6, tandis que l'antigène associé à une tumeur présente une séquence d'acides aminés selon SEQ ID NO: 118.

8. Utilisation d'un kit pour le diagnostic d'une affection cancéreuse, qui se **caractérise par** l'expression d'un antigène associé à une tumeur, comprenant un moyen pour l'identification
(i) de l'acide nucléique qui code pour l'antigène associé à une tumeur, ou
(ii) de l'antigène associé à une tumeur,
tandis que l'antigène associé à une tumeur est codé par une séquence d'acide nucléique selon SEQ ID NO: 117 et tandis que l'affection cancéreuse est un carcinome du pancréas ou un carcinome de l'oesophage.
